(19) Europäisches Patentamt
European Patent Office
Office européen des brevets

(11) Veröffentlichungsnummer: **0 288 048 B1**

# (12) EUROPÄISCHE PATENTSCHRIFT

(45) Veröffentlichungstag der Patentschrift: **29.06.94**

(21) Anmeldenummer: **88106370.5**

(22) Anmeldetag: **21.04.88**

(51) Int. Cl.[5]: **C07D 217/14**, C07D 217/16, C07D 495/04, A61K 31/44, //(C07D495/04,333:00,221:00)

## (54) Benzo- und Thieno-3,4-dihydro-pyridinderivate, Verfahren zu ihrer Herstellung und diese Verbindungen enthaltende Arzneimittel.

(30) Priorität: **24.04.87 DE 3713743**
**03.06.87 DE 3718570**

(43) Veröffentlichungstag der Anmeldung:
**26.10.88 Patentblatt 88/43**

(45) Bekanntmachung des Hinweises auf die Patenterteilung:
**29.06.94 Patentblatt 94/26**

(84) Benannte Vertragsstaaten:
**AT BE CH DE ES FR GB GR IT LI LU NL SE**

(56) Entgegenhaltungen:
**EP-A- 0 037 934**
**EP-A- 0 198 227**
**EP-A- 0 251 194**

**JOURNAL OF ORGANIC CHEMISTRY, Band 48, 1983, Seiten 1075-1080; J. BOSCH et al.: "Studies on the synthesis of the benzo(a)quainolizidin-2-one ring system. Preparation of a 1,1-dimethyl derivative"**

(73) Patentinhaber: **BOEHRINGER INGELHEIM KG**

**D-55216 Ingelheim(DE)**
(84) Benannte Vertragsstaaten:
**AT BE CH DE ES FR GB GR IT LI LU NL SE**

(73) Patentinhaber: **BOEHRINGER INGELHEIM INTERNATIONAL GmbH**

**D-55216 Ingelheim(DE)**
(84) Benannte Vertragsstaaten:
**GB**

(72) Erfinder: **Lösel, Walter, Dr.**
**Im Herzenacker 26**
**D-6535 Gau-Algesheim(DE)**
Erfinder: **Roos, Otto, Dr.**
**Elsheimer Strasse 36**
**D-6501 Schwabenheim(DE)**
Erfinder: **Schnorrenberg, Gerd, Dr.**
**Ernst-Ludwig-Strasse 66a**
**D-6535 Gau-Algesheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

CHEMICAL AND PHARMACEUTICAL BULLETIN, Band 30, Nr. 2, 1982, Seiten 598-609; T. FUJI et al.: "Quinolizidines. VII. Structure of Q-methal psychotrine: the endocyclic versus the exocyclic double bond structure in the dihydroisoquinoline moiety"

CHEMICAL ABSTRACTS, Band 87, Nr. 17, 24. Qktober 1977, Seite 699, Spalte 1, Zusammenfassung-Nr. 134 980z, Columbus, Qhio, USA; J. KQBQR: "Study on isoquinoline compounds. Study on bis(6,7-dimethoxy-3,4-dihydro-1-isoquinolyl)-methane"

Erfinder: **Arndts, Dietrich, Dr.**
**Mühlstrasse 7**
**D-6531 Appenheim(DE)**
Erfinder: **Speck, Georg, Dr.**
**Rheinstrasse 13**
**D-6507 Ingelheim/Rhein(DE)**
Erfinder: **Streller, Ilse, Dr.**
**Waldstrasse 16**
**D-6534 Stromberg(DE)**
Erfinder: **Kuhn, Franz Josef, Dr.**
**Beethovenstrasse 11**
**D-6535 Gau-Algesheim(DE)**
Erfinder: **Schingnitz, Günther, Dr.**
**Unter den Gärten 18**
**D-6550 Bad Kreuznach 14(DE)**

**Beschreibung**

Die Erfindung betrifft neue Benzo- und Thieno-3,4-dihydro-pyridinderivate, Verfahren zu ihrer Herstellung und diese Verbindungen enthaltende Arzneimittel.

Die neuen Verbindungen haben die allgemeine Formel I

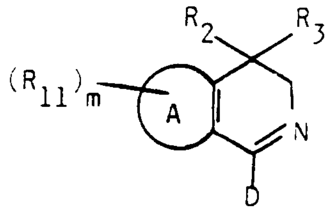

I

worin
A einen Benzo- oder Thienorest bedeutet;
$R_2$ und $R_3$ unabhängig voneinander Wasserstoff oder $(C_1\text{-}C_5)$Alkyl oder gemeinsam mit dem Kohlenstoffatom, an das sie gebunden sind, einen 5-oder 6-gliedrigen Carbocyclus bedeuten;
$R_{11}$ $(C_1\text{-}C_4)$Alkyl, Halogen (F, Cl, Br, J), Hydroxy, $(C_1\text{-}C_4)$Alkoxy, Amino, Thiomethyl, Methansulfonyloxy oder Methansulfonamido bedeutet, oder zwei benachbarte Substituenten $R_{11}$ zusammen $-O-CH_2-O-$ oder $-O-CH_2-CH_2-O-$ sind;
m 0, 1, 2 oder 3 bedeutet, wenn A ein Benzorest ist, und 0, 1 oder 2 bedeutet, wenn A ein Thienorest ist;
und D eine Gruppe der Formel Ia oder Ib bedeutet

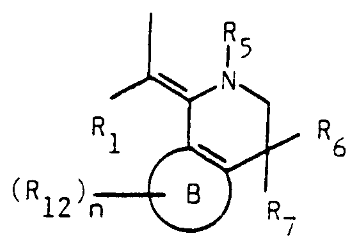

Ia

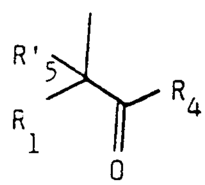

Ib

wobei in der Gruppe der Formel Ia
B einen Benzo- oder Thienorest bedeutet;
$R_1$ Wasserstoff, $(C_1\text{-}C_{10})$Alkyl, Phenyl, Phenyl$(C_1\text{-}C_5)$alkyl, $(C_1\text{-}C_4)$Alkoxy oder -NHCOX (worin X $(C_1\text{-}C_5)$-Alkyl ist) bedeutet;
$R_5$ Wasserstoff, $(C_1\text{-}C_4)$Alkyl oder Hydroxymethyl bedeutet;
$R_6$ und $R_7$ unabhängig voneinander Wasserstoff oder $(C_1\text{-}C_5)$Alkyl oder gemeinsam mit dem Kohlenstoffatom, an das sie gebunden sind, einen 5- oder 6-gliedrigen Carbocyclus bedeuten;
$R_{12}$ $(C_1\text{-}C_4)$Alkyl, Halogen (F, Cl, Br, J), Hydroxy, $(C_1\text{-}C_4)$Alkoxy, Amino, Thiomethyl, Methansulfonyloxy oder Methansulfonamido bedeutet, oder zwei benachbarte Substituenten
$R_{12}$ zusammen $-O-CH_2-O-$ oder $-O-CH_2-CH_2-O-$ sind;
und n 0, 1, 2 oder 3 bedeutet, wenn B ein Benzorest ist, und 0, 1 oder 2 bedeutet, wenn B ein Thienorest ist;
und in der Gruppe der Formel Ib
$R_1$ Wasserstoff, $(C_1\text{-}C_{10})$Alkyl, Phenyl$(C_1\text{-}C_5)$alkyl, $(C_1\text{-}C_4)$Alkoxy oder -NHCOX (worin X $(C_1\text{-}C_5)$Alkyl ist) bedeutet;
$R'_5$ Wasserstoff oder $(C_1\text{-}C_4)$Alkyl bedeutet;
$R_4$ eine $-NR_9R_{10}$-Gruppe bedeutet, worin
$R_9$ und $R_{10}$ unabhängig voneinander (a) Wasserstoff, (b) verzweigtes oder unverzweigtes Alkyl, Alkenyl oder Alkinyl mit 1-12 Kohlenstoffatomen (wobei das Alkyl substituiert sein kann durch Hydroxy, $(C_1\text{-}C_4)$-Alkoxy, Di$(C_1\text{-}C_4)$Alkylamino, Furyl, Pyrrolidinyl, Morpholinyl, Pyridinyl, Indolyl, oder die Gruppe

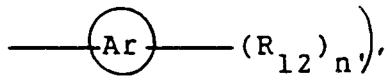

(c) Cycloalkyl mit 3-7 Ringgliedern, (d) Dimethylamino, (e) Amino($C_2$-$C_4$)alkyl (worin die Aminogruppe unsubstituiert sein kann oder Mono- oder Di-($C_1$-$C_4$)alkylamino ist), (f) Phenyl, (g) Morpholinyl, oder (h) Pyrridinyl bedeuten, wobei $R_9$ und $R_{10}$ nicht gleichzeitig Wasserstoff, Dimethylamino oder Di($C_1$-$C_4$)-alkylaminomethyl sein können;
oder $R_9$ und $R_{10}$ gemeinsam mit dem Stickstoffatom, an das sie gebunden sind, einen Pyrrolidinyl-, Piperidinyl-, Morpholinyl- oder Piperazinylrest bedeuten, wobei der Piperazinylring gegebenenfalls durch unsubstituiertes Phenyl, Mono- oder Di($C_1$-$C_4$)alkoxyphenyl, Pyrimidinyl oder Phenyl($C_1$-$C_4$)alkyl N-substituiert sein kann;
$R_{12}$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R_{12}$ zusammen -O-$CH_2$-O- oder -O-$CH_2$-$CH_2$-O- sind;
Ar Phenyl oder Thienyl ist;
$n'$ 0, 1, 2 oder 3 bedeutet, wenn Ar Phenyl ist und 0, 1 oder 2 bedeutet, wenn Ar Thienyl ist;
und ihre pharmazeutisch annehmbaren Salze mit anorganischen oder organischen Säuren;
ausgenommen die Verbindung der Formel I, worin D die Gruppe Ia ist und $R_1$, $R_2$, $R_3$, $R_5$, $R_6$ und $R_7$ Wasserstoff und A und B den Rest

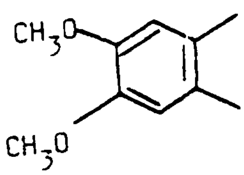

bedeuten;
und
ausgenommen die Verbindung der Formel I, worin D die Gruppe der Formel Ib ist und $R_1$, $R_2$, $R_3$ und $R'_5$ Wasserstoff, A den Rest

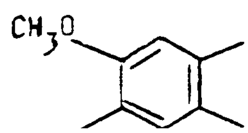

und $R_4$ die Gruppe

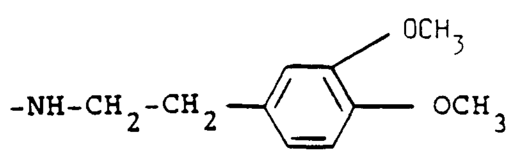

bedeuten.

Unter dem Carbocyclus, der von $R_2$ und $R_3$ beziehungsweise $R_6$ und $R_7$ und dem jeweiligen Kohlenstoffatom, an das sie gebunden sind, gebildet wird, ist vorzugsweise ein gesättigter 5- oder 6-gliedriger Carbocyclus zu verstehen.

Verbindungen der Formel I, worin D die Gruppe der Formel Ia ist, werden im folgenden als Verbindungen der Formel VIII bezeichnet.

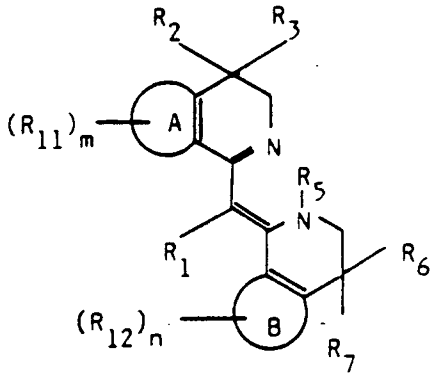

VIII

Verbindungen der Formel VIII, worin $R_5$ Wasserstoff ist, bilden Tautomere der Formeln VIIIa und VIIIb,

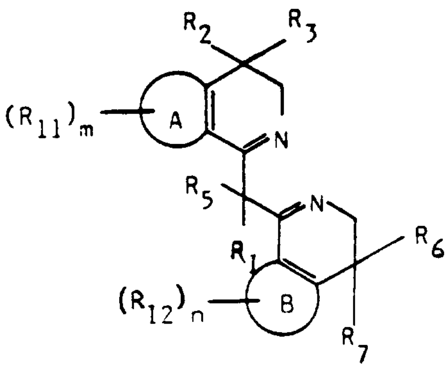

VIIIa

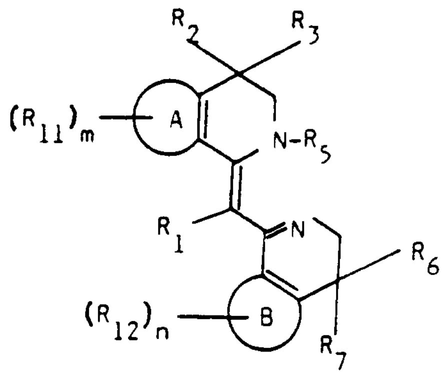

VIIIb

worin $R_5$ ebenfalls Wasserstoff ist. Die Definition der Formel I bzw. VIII umfaßt auch die genannten Tautomeren.

Verbindungen der Formel I, worin D die Gruppe der Formel Ib ist, werden im folgenden als Verbindungen der Formel IX bezeichnet.

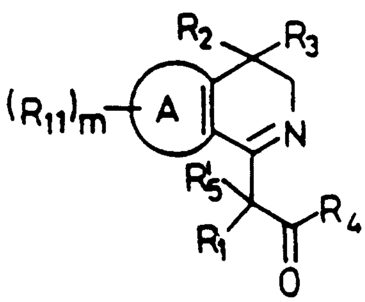

IX

Verbindungen der Formel IX, worin $R'_5$ Wasserstoff ist, bilden Tautomere der Formel IXa

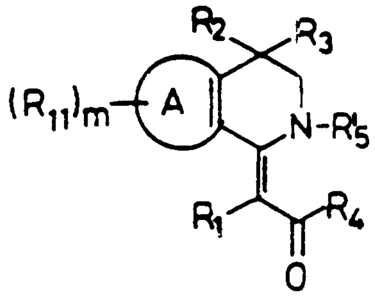

IXa

Die Tautomeren sind nach bekannten Methoden trennbar z.B. durch Säulenchromatographie oder selektive Reduktion ($NaBH_4$ beziehungsweise katalytische Reduktion). Verbindungen beider Strukturen, in denen $R_5$ -($C_1$-$C_4$)Alkyl bedeutet, sind stabil.

Die Definition der allgemeinen Formel I bzw. IX ist so zu verstehen, daß sie auch die Verbindungen der Struktur IXa, worin $R'_5$ Wasserstoff oder ($C_1$-$C_4$)Alkyl bedeutet, umfaßt.

Von Kobor Jeno wurde in Szegedi Tonarkepzo Foiskola Tud. Kozl. 1975 Seiten 145-153 (vgl. Chem. Abstr. 87; 134980Z) Verbindung der allgemeinen Formel VIII

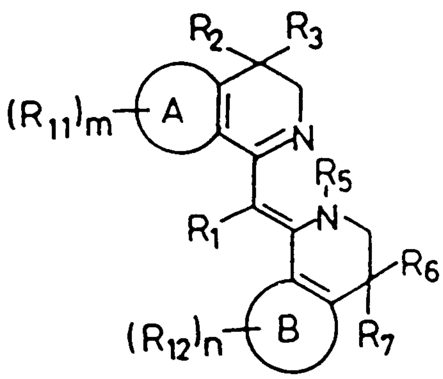

VIII

und ihre Herstellung beschrieben, worin $R_1$, $R_2$, $R_3$, $R_5$, $R_6$ und $R_7$ Wasserstoff bedeuten und die substituierten Gruppen A und B den Rest

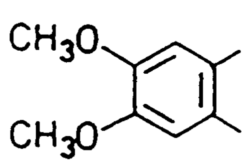

bedeuten. Diese Publikation enthält keine Angaben über physiologische Wirkungen dieser Verbindung. Verbindungen des Typs der Formel IX, worin jedoch $R_1$ Phenyl beziehungsweise $R_4$ Alkoxy sind in EP-A 037 934, EP-A 251 194, J. Org. Chem., 48, 1983, Seiten 1075-1080 und Chem. Pharm. Bull., 30, Nr. 2, 1982, Seiten 588-609 beschrieben worden.

Es wurde überraschenderweise gefunden, daß die neuen Verbindungen der allgemeinen Formel I und die oben genannte Verbindung sowie die dafür nötige Zwischenstufe der allgemeinen Formel II sowohl als Basen als auch in Form ihrer Salze wertvolle therapeutische Eigenschaften besitzen.

Ferner sind Verbindungen der allgemeinen Formel IX, worin $R_4$ die Gruppe der allgemeinen Formel X ist,

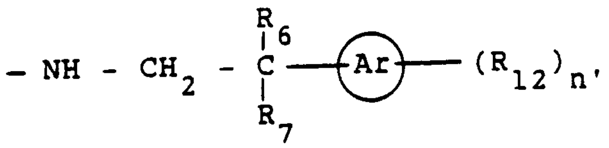

X

wichtige Zwischenstufen in der Herstellung von Verbindungen der allgemeinen Formel VIII, ihrer Tautomeren und Salze.

Von den Verbindungen der Formel VIII können folgende als besonders interessant bevorgehoben werden:

-) Verbindungen VIII, worin $R_1$ Wasserstoff, $(C_1-C_{10})$Alkyl, Phenyl$(C_1-C_5)$alkyl oder -NHCOX (worin X $(C_1-C_5)$Alkyl ist) bedeutet;

$R_{11}$ und $R_{12}$ unabhängig voneinander $(C_1-C_4)$Alkyl, Hydroxy, $(C_1-C_4)$Alkoxy, Methansulfonyloxy oder Methansulfonamido bedeuten, oder zwei benachbarte Substituenten $R_{11}$ beziehungsweise $R_{12}$ zusammen $-O-CH_2-O-$ oder $-O-CH_2-CH_2-O-$ sind;

-) Verbindungen VIII, worin $R_1$ Wasserstoff, $(C_1-C_{10})$Alkyl oder -NHCOX (worin X $(C_1-C_5)$Alkyl ist) bedeutet;

$R_2$, $R_3$, $R_6$ und $R_7$ unabhängig von einander Wasserstoff bedeuten oder $R_2$ mit $R_3$ und/oder $R_6$ mit $R_7$ und dem jeweiligen Kohlenstoffatom, an das sie gebunden sind, einen 5- oder 6-gliedrigen Carbocyclus bedeuten;

$R_{11}$ und $R_{12}$ unabhängig voneinander Hydroxy, $(C_1-C_4)$Alkoxy, Methansulfonyloxy oder Methansulfonamido bedeuten, oder zwei benachbarte Substituenten $R_{11}$ beziehungsweise $R_{12}$ zusammen $-O-CH_2-O-$ oder $-O-CH_2-CH_2-O-$ sind;

-) insbesondere Verbindungen, worin $R_1$ Wasserstoff, $(C_1-C_6)$Alkyl, oder $-NHCOCH_3$ bedeutet und/oder $R_5$ Wasserstoff, Methyl oder Hydroxymethyl bedeutet und/oder

$R_2$, $R_3$, $R_6$ und $R_7$ unabhängig voneinander Wasserstoff bedeuten oder $R_2$ mit $R_3$ und/oder $R_6$ mit $R_7$ und dem jeweiligen Kohlenstoffatom, an das sie gebunden sind, einen 5-gliedrigen Carbocyclus bedeuten und/oder

$R_{11}$ und $R_{12}$ unabhängig voneinander Hydroxy, Methoxy, Methansulfonyloxy oder Methansulfonamido bedeuten, oder zwei benachbarte Substituenten $R_{11}$ beziehungsweise $R_{12}$ zusammen $-O-CH_2-O-$ sind.

Bevorzugt sind Verbindungen VIII, worin m und/oder n 2 bedeuten, insbesondere solche in denen A und/oder B ein Benzorest ist, wobei vorzugsweise die beiden Substituenten $R_{11}$ und/oder $R_{12}$ im Benzorest in meta- beziehungsweise para-Position zu den Fusionspunkten des Restes A beziehungsweise B stehen.

Hervorzuheben sind Verbindungen, worin $R_{11}$ und $R_{12}$ Methoxy sind.

Beispiele spezifischer Verbindungen gemäß der Erfindung sind in den Tabellen weiter unten angeführt, hervorzuheben sind 1-(3,4-Dihydro -6,7-dimethoxy-isochinolin-1-yl)-1-(3,4-dihydro-6,7-dimethoxy-isochinolin-1-yliden)-ethan,

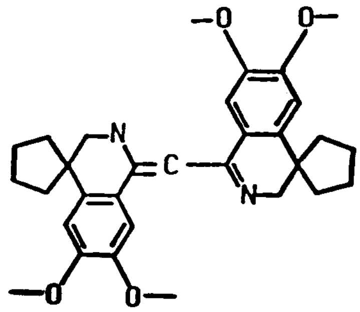

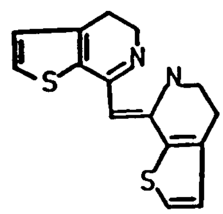

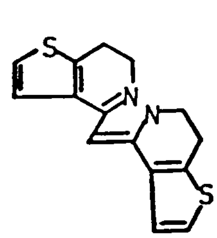

und

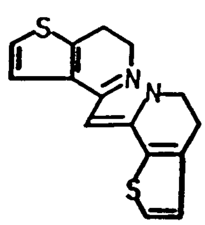

oder ihre physiologisch unbedenklichen Salze.

Von den Verbindungen der Formel IX können folgende als besonders interessant hervorgehoben werden:

Verbindungen

- worin $R_4$ eine $-NR_9R_{10}$-Gruppe bedeutet, worin $R_9$ und $R_{10}$ unabhängig voneinander (a) Wasserstoff, (b) Alkyl mit 1 bis 8 Kohlenstoffatomen, Alkenyl oder Alkinyl mit 2 oder 3 Kohlenstoffatomen (wobei das Alkyl substituiert sein kann durch Hydroxy, $(C_1$-$C_4)$Alkoxy, Di$(C_1$-$C_4)$Alkylamino, Furyl, Pyrrolidinyl, Morpholinyl, Pyridinyl oder die Gruppe

$$-\!\!\!\bigcirc\!\!\!\!\!\!\!\!\text{Ar}\;-(R_{12})_{n'},$$

worin Ar, $R_{12}$ und n' wie oben definiert sind), (d) Dimethylamino, (f) Phenyl, (g) Morpholinyl, (h) Pyrridinyl bedeuten, wobei $R_9$ und $R_{10}$ nicht gleichzeitig Wasserstoff, Dimethylamino oder Di$(C_1$-$C_4)$-alkylaminomethyl sein können;
oder $R_9$ und $R_{10}$ gemeinsam mit dem Stickstoffatom, an das sie gebunden sind, einen Pyrrolidinyl-, Morpholinyl- oder Piperazinylrest bedeuten, wobei der Piperazinylring gegebenenfalls durch unsubstituiertes Phenyl, Mono- oder Di($C_1$-$C_4$)alkoxyphenyl, Pyrimidinyl oder Phenyl($C_1$-$C_4$)alkyl N-substituiert sein kann;
insbesondere Verbindungen, worin $R_4$ eine -$NR_9R_{10}$-Gruppe ist, worin $R_9$ und/oder $R_{10}$ unsubsituiertes Phenyl, Fluorphenyl, Morpholino oder 2- oder 3-Pyridinyl bedeutet;
worin $R_4$ eine -$NR_9R_{10}$-Gruppe ist, worin $R_9$ und/oder $R_{10}$ ($C_1$-$C_4$)Alkyl, vorzugsweis Methyl oder Ethyl, bedeutet;
worin $R_4$ eine -$NR_9R_{10}$-Gruppe ist, worin $R_9$ und/oder $R_{10}$ ($C_2$ oder $C_3$)Alkyl bedeutet, das durch Hydroxy, Methoxy, Dimethylamin, Furyl, Morpholino, Pyrrolidine oder Pyridinyl substituiert ist;
worin $R_4$ eine -$NR_9R_{10}$-Gruppe ist, worin $R_9$ Wasserstoff ist.

Ferner sind Verbindungen (IX) hervorzuheben,
worin $R_4$ eine -$NR_9R_{10}$-Gruppe ist, worin $R_9$ Wasserstoff ist und $R_{10}$ ein substituiertes Alkyl der Formel VII ist,

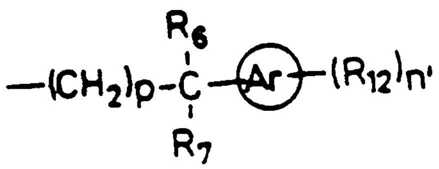

VII

worin p 0, 1 oder 2 ist;
$R_6$ und $R_7$ unabhängig voneinander Wasserstoff oder ($C_1$-$C_5$)Alkyl oder gemeinsam mit dem Kohlenstoffatom, an das sie gebunden sind, einen 5- oder 6-gliedrigen Carbocyclus bedeuten;
Ar Phenyl oder Thienyl bedeutet;
$R_{12}$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R_{11}$ zusammen -O-$CH_2$-O- oder -O-$CH_2$-$CH_2$-O- sind und n' O, 1, 2 oder 3 bedeutet, wenn Ar Phenyl ist, und O, 1 oder 2 bedeutet, wenn Ar Thienyl ist;
insbesondere Verbindungen
worin $R_{12}$ ($C_1$-$C_4$)Alkyl, Hydroxy, ($C_1$-$C_4$)Alkoxy, Methansulfonyloxy oder Methansulfonamido bedeutet, oder zwei benachbarte Substituenten $R_{12}$ zusammen -O-$CH_2$-O- oder -O-$CH_2$-$CH_2$-O- sind;
worin $R_{12}$ Hydroxy, ($C_1$-$C_4$)Alkoxy, Methansulfonyloxy oder Methansulfonamido bedeutet, oder zwei benachbarte Substituenten $R_{12}$ zusammen -O-$CH_2$-O- oder -O-$CH_2$-$CH_2$-O- sind;
worin $R_{12}$ Hydroxy, Methoxy, Methansulfonyloxy oder Merhansulfonamido bedeuten, oder zwei benachbarte Substituenten $R_{12}$ zusammen -O-$CH_2$-O- sind;
besonders Verbindungen
worin $R_{12}$ Methoxy ist;
ferner Verbindungen
worin n' null ist;
worin Ar Phenyl und n' zwei ist, vorzugsweise worin die beiden Sustituenten $R_{12}$ in den Positionen 2 und 3 sind;
und/oder worin p eins ist,
und/oder worin $R_6$ und $R_7$ Wasserstoff sind.

Hervorzuheben sind ferner Verbindungen (IX), worin $R_4$ eine -$NR_9R_{10}$-Gruppe ist, worin $R_9$ und $R_{10}$ gemeinsam mit dem Stickstoffatom, an das sie gebunden sind, Morpholino, Pyrrolidinyl oder Piperazinyl (das durch Methoxyphenyl, Phenethyl oder 2-Pyrimidinyl N-substituiert ist) bedeuten;

Von den genannten Verbindungsgruppen sind solche bevorzugt,

- worin $R_1$ Wasserstoff, ($C_1$-$C_{10}$)Alkyl, Phenyl ($C_1$-$C_5$)alkyl oder -NHCOX (worin X ($C_1$-$C_5$)Alkyl ist) bedeutet; und $R_{11}$ ($C_1$-$C_4$)Alkyl, Hydroxy, ($C_1$-$C_4$)Alkoxy, Methansulfonyloxy oder Methansulfonamido bedeutet, oder zwei benachbarte Substituenten $R_{11}$ zusammen -O-$CH_2$-O- oder -O-$CH_2$-$CH_2$-O- sind;
- worin $R_1$ Wasserstoff, ($C_1$-$C_{10}$)Alkyl oder -NHCOX (worin X ($C_1$-$C_5$ Alkyl ist) bedeutet;
  $R_2$ und $R_3$ Wasserstoff bedeuten oder zusammen mit dem Kohlenstoffatom, an das sie gebunden sind, einen 5- oder 6-gliedrigen gesättigten Carbocyclus bedeuten;
  $R_{11}$ Hydroxy, ($C_1$-$C_4$)Alkoxy, Methansulfonyloxy oder Methansulfonamido bedeutet, oder zwei benachbarte Substituenten $R_{11}$ zusammen -O-$CH_2$-O- oder -O-$CH_2$-$CH_2$-O- sind;
- worin $R_1$ Wasserstoff, ($C_1$-$C_6$)Alkyl, oder -$NHCOCH_3$ bedeutet;
- worin $R'_5$ Wasserstoff, Methyl oder Ethyl bedeutet;

- worin $R_2$ und $R_3$ Wasserstoff bedeuten oder gemeinsam mit dem Kohlenstoffatom, an das sie gebunden sind, einen 5-gliedrigen gesättigten Carbocyclus bedeuten;
- worin $R_{11}$ Hydroxy, Methoxy, Methansulfonyloxy oder Methansulfonamido bedeutet, oder zwei benachbarte Substituenten $R_{11}$ zusammen $-O-CH_2-O-$ sind;
- worin, wenn A ein Benzorest ist, m 2 bedeutet, und vorzugsweise die beiden Substituenten $R_{11}$ in meta-beziehungsweise para-Position zu den Fusionspunkten des Restes A stehen;
- worin $R_{11}$ Methoxy ist.

Bevorzugt sind Verbindungen, worin A Benzo, $R_{11}$ Methoxy, m zwei, $R_1$ Wasserstoff oder $(C_1$-$C_5)$Alkyl, $R_2$, $R_3$ und $R'_5$ Wasserstoff und $R_4$ Morpholino, Methylamino, Diethylamino oder Phenethylamino bedeuten.

Beispiele spezifischer Verbindungen gemäß der Erfindung sind in den Tabellen weiter unten angeführt, hervorzuheben sind
Morpholinocarbonylmethyl-6,7-dimethoxy-3,4-dihydro-isochinolinolin,
6,7-Dimethoxy-3,4-dihydro-isochinolinessigsäuremethylamid,
6,7-Dimethoxy-3,4-dihydro-isochinolinessigsäurediethylamid und
6,7-Dimethoxy-3,4-dihydro-isochinolinessigsäurephenylethylamid sowie ihr physiologisch unbbedenklichen Salze.

Die erfindungsgemäßen Verbindungen können in an sich bekannter Weise hergestellt werden.

Herstellung der Verbindung der Formel VIII:

A. In Gegenwart eines Kondensationsmittels kann ein Amid der allgemeinen Formel II,

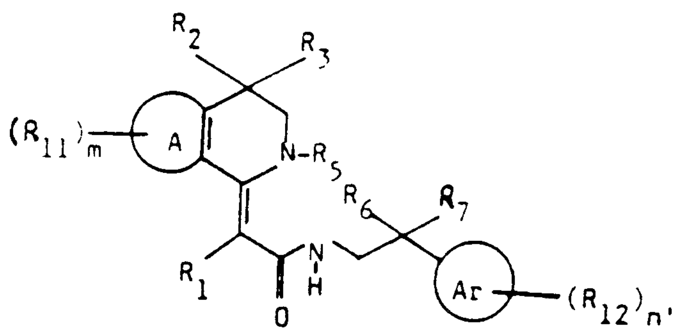

II

worin A, $R_1$, $R_2$, $R_3$, $R_6$, $R_7$, $R_{11}$, $R_{12}$ und m wie oben definiert sind, Ar Phenyl oder Thienyl bedeutet und n′ O, 1, 2 oder 3 bedeutet wenn Ar Phenyl ist, und O, 1 oder 2 bedeutet, wenn Ar Thienyl ist, und $R_5$ Wasserstoff oder $(C_1$-$C_4)$Alkyl bedeutet, zu einer entsprechenden Verbindung (VIII) cyclisiert werden.

Als Kondensationsmittel eignen sich starke Lewis-Säuren, wie z.B. Phorphoroxychlorid, Phosphorpentachlorid, Phosphortrichlorid, Phosphorpentoxid, Titantetrachlorid, Bortrifluorid, Zinntetrachlorid, aber auch anorganische Säuren, wie z.B. Polyphosphorsäure, Schwefelsäure, Fluorsulfonsäure und Fluorwasserstoffsäure, oder Gemische von Kondensationsmitteln wie z.B. ein Gemisch von Phosphoroxychlorid und Phosphorpentachlorid, oder ein Gemisch von Phosphorpentoxid und $(C_1$-$C_4)$ Alkylsulfonsäure z.B. mit einem $P_2O_5$-Anteil von ca. 10 Gewichtsprozenten.

Wird eine Verbindung II, worin $R_5$ Wasserstoff ist, in Gegenwart des Gemisches von Phosphorpentoxid und $(C_1$-$C_4)$Alkylsulfonsäure cyclisiert, so erhält man neben der entsprechenden Verbindung VIII, in der $R_5$ Wasserstoff ist, auch die analoge Verbindung VIII, in der $R_5$ $(C_1$-$C_4)$Alkyl ist. Vorzugsweise wird diese Verfahrensvariante mit Methansulfonsäure ausgeführt.

Die Cyclisierung kann in Gegenwart oder Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen, beispielsweise Benzol, Alkylbenzole (z.B. Toluol, Xylol), Chlorbenzole, Chloroform, Acetonitril, Dekalin. Eine bevorzugte Variante des Verfahrens besteht darin, das Kondensationsmittel, beispielsweise Phosphoroxychlorid oder ein $(C_1$-$C_4)$-Alkylsulfonsäure-/Phosphorpentoxid-Gemisch,ohne Zusatz von Lösungsmittel zu verwenden.

Bevorzugt erfolgt die Cyclisierung mit Phosphoroxychlorid oder in schwierigen Fällen mit einem Gemisch von Phosphorpentoxid und $(C_1$-$C_4)$Alkylsulfonsäure (bevorzugt Methansulfonsäure).

Die Umsetzung kann innerhalb eines großen Temperaturbereichs, vorzugsweise unter Erwärmen oder Erhitzen auf 50 °C bis etwa den Siedepunkt des Reaktionsgemisches, durchgeführt werden.

Die erforderliche Reaktionsdauer liegt je nach Ausgangsverbindung II zwischen mehreren Tagen und mehreren Stunden.

Die Verbindungen der oben definierten allgemeinen Formel II sind neue Verbindungen. Sie können durch Cyclisieren des entsprechenden Malonsäurediamids der allgemeinen Formel IIIa

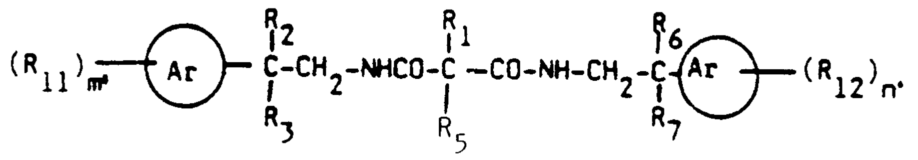

IIIa

hergestellt werden, in der $R_5$ Wasserstoff ist und $R_1$, $R_2$, $R_3$, $R_6$, $R_7$, $R_{11}$ und $R_{12}$ wie oben definiert sind, Ar Phenyl oder Thienyl bedeutet und m' und n' unabhängig voneinander O, 1, 2 oder 3 bedeuten, wenn das zugehörige Ar Phenyl ist, und O, 1 oder 2 bedeuten, wenn das zugehörige Ar Thienyl ist.

Die Umsetzung erfolgt wie oben für die Cyclisierung der Verbindung II zu Verbindung VIII beschrieben ist. Wird die Reaktion mit dem Gemisch von Phosphorpentoxid und ($C_1$-$C_4$)Alkylsulfonsäure ausgeführt so erhält man neben der entsprechenden Verbindung II, worin $R_5$ Wasserstoff ist, auch die analoge Verbindung II, worin $R_5$ ($C_1$-$C_4$)Alkyl ist. (Siehe dazu auch Verfahren C.)

B. Die Umsetzung, bei der von Verbindung IIIa ausgegangen wird, kann ohne Isolierung der Zwischenverbindung II in situ bis zur Herstellung der Verbindung VIII geführt werden.

Da bei manchen Verbindungen sich der Isochinolin- bzw. Thienopyridinring nur sehr schwer schließt, kann man, falls erforderlich oder erwünscht, die während der Cyclisierungsreaktion sich bildende Zwischenverbindung der allgemeinen Formel II oder deren Tautomeres isolieren, die Base freisetzen und diese der zweiten Cyclisierungsreaktion unterwerfen. In diesem Fall arbeitet man in der ersten Stufe bevorzugt mit Phosphoroxychlorid unter gelindem Erwärmen. In der zweiten Stufe schließt man den Ring mit Phorphorpentachlorid, einem Gemisch aus Phosphoroxychlorid und Phorphorpentachlorid oder mit einem Methansulfonsäure-/$P_2O_5$-Gemisch.

Die Verbindungen der allgemeinen Formel IIIa sind im wesentlichen bekannte Verbindungen und können nach an sich bekannten Verfahren hergestellt werden.

Herstellung der Verbindungen der Formel IX:

C. In Gegenwart eines Kondensationsmittels kann ein Malonsäurediamid der allgemeinen Formel III

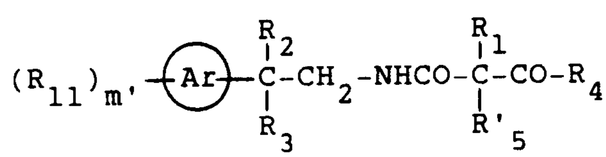

III

in der $R_1$, $R_2$, $R_3$, $R_4$, und $R_{11}$ wie oben definiert sind, $R'_5$ Wasserstoff ist, Ar Phenyl oder Thienyl bedeutet und m' O, 1, 2 oder 3 bedeutet, wenn Ar Phenyl ist, und O, 1 oder 2 bedeutet, wenn Ar Thienyl ist, zu entsprechenden Verbindungen IX und IXa cyclisiert werden. Auf die Ausführung des Verfahrens wird weiter unten näher eingegangen. Wird die Reaktion mit einem Gemisch von Phosphorpentoxid und ($C_1$-$C_4$)Alkylsulfonsäure ausgeführt, so erhält man neben den entsprechenden Verbindungen IX und IXa, worin $R'_5$ Wasserstoff ist, auch die analogen Verbindungen IX und IXa, worin $R'_5$ ($C_1$-$C_4$)Alkyl ist.

Als Kondensationsmittel für dieses Verfahren eignen sich starke Lewis-Säuren, wie z.B. Phorphoroxychlorid, Phosphorpentachlorid, Phosphortrichlorid, Phosphorpentoxid, Titantetrachlorid, Bortrifluorid, Zinntetrachlorid, aber auch anorganische Säuren, wie z.B. Polyphosphorsäure, Schwefelsäure, Fluorsulfonsäure und Fluorwasserstoffsäure, oder Gemische von Kondensationsmitteln wie z.B. ein Gemisch von Phosphoroxychlorid und Phosphorpentachlorid, oder ein Gemisch von Phosphorpentoxid und ($C_1$-$C_4$) Alkylsulfonsäure z.B. mit einem $P_2O_5$-Anteil von ca. 10 Gewichtsprozenten.

Wird eine Verbindung III, worin $R'_5$ Wasserstoff ist, in Gegenwart des Gemisches von Phosphorpentoxid und ($C_1$-$C_4$)Alkylsulfonsäure cyclisiert, so erhält man, wie oben erwähnt worden ist, neben der entsprechenden Verbindungen IX und IXa, in denen $R'_5$ Wasserstoff ist, auch die analogen Verbindungen IX und IXa, in denen $R'_5$ ($C_1$-$C_4$)Alkyl ist. Vorzugsweise wird diese Verfahrensvariante mit Methansulfonsäure ausgeführt.

Die Cyclisierung kann in Gegenwart oder Abwesenheit eines Lösungsmittels durchgeführt werden. Geeignet sind alle inerten Lösungsmittel, soweit sie eine ausreichende Löslichkeit für die Reaktionspartner besitzen und einen ausreichend hohen Siedepunkt aufweisen, beispielsweise Benzol, Alkylbenzole (z.B. Toluol, Xylol), Chlorbenzole, Chloroform, Acetonitril, Dekalin. Eine bevorzugte Variante des Verfahrens besteht darin, das Kondensationsmittel, beispielsweise Phosphoroxychlorid oder ein ($C_1$-$C_4$)-Alkylsulfonsäure-/Phosphorpentoxid-Gemisch,ohne Zusatz von Lösungsmittel zu verwenden.

Bevorzugt erfolgt die Cyclisierung mit Phosphoroxychlorid oder in schwierigen Fällen mit einem Gemisch von Phosphorpentoxid und ($C_1$-$C_4$)Alkylsulfonsäure (bevorzugt Methansulfonsäure).
Die Umsetzung kann innerhalb eines großen Temperaturbereichs, vorzugsweise unter Erwärmen oder Erhitzen auf 50 °C bis etwa den Siedepunkt des Reaktionsgemisches, durchgeführt werden.

Die erforderliche Reaktionsdauer liegt je nach Ausgangsverbindung III zwischen 2 und 15 Stunden.

Die Tautomeren der allgemeinen Formeln IX und IXa, worin $R'_5$ Wasserstoff ist, können nach bekannten Verfahren getrennt werden, wie z.B. durch Säulenchromatographie oder selektive Reduktion mit z.B. $NaBH_4$ (reduziert Tautomere IX). Durch katalytische Reduktion werden die Tautomeren IX und IXa reduziert.

Verbindungen der Formel I, worin $R_5$ Wasserstoff ist, werden gegebenenfalls N-alkyliert. Für die N-Alkylierung eignen sich im Prinzip alle bekannten Alkylierungsmittel, soweit sie eine ausreichende Reaktivität besitzen, z.B. aktive Alkylester, wie Dialkylsulfat, Toluolsulfonsäurealkylester oder Fluormethansulfonsäurealkylester. Die Reation erfolgt bei Temperaturen bis zum Siedepunkt des Reaktionsgemisches (Alkyl steht hier für ($C_1$-$C_4$) Alkyl).

Die N-Hydroxymethylierung erfolgt unter den Bedingungen der Aminoalkylierung nach Leuckart-Wallach (Ber. dtsch. Chem. Ges. 18, (1885) 2341) oder Eschweiler-Clarke (Teilheimer 2, (1948) Nr. 352; 4 (1950) Nr. 378). Im allgemeinen wird die Substanz mit einer z.B. 30%-igen Formalinlösung in Gegenwart von Ameisensäure bei Raumtemperatur behandelt.

Die Überführung der freien Base der allgemeinen Formel I in ihre Säureadditionssalze erfolgt in an sich bekannter Weise.

Zur Salzbildung geeignete Säuren sind beispielsweise Salzsäure, Bromwasserstoffsäure, Schwefelsäure, Phosphorsäure, Salpetersäure, Essigsäure, Propionsäure, Buttersäure, Oxalsäure, Malonsäure, Bernsteinsäure, Maleinsäure, Fumarsäure, Milchsäure, Weinsäure, Zitronensäure Apfelsäure, Benzoesäure, Zimtsäure, Ascorbinsäure, Methansulfonsäure.

Die neuen Bis-(3,4-dihydro-1-pyridinyl)-methane der allgemeinen Formel I besitzen, wie eingangs erwähnt, sowohl als Basen als auch in Form ihrer Salze wertvolle therapeutische Eigenschaften.

Insbesondere weisen diese Substanzen eine deutliche cardioprotektive Wirkung auf, die wie folgt bestimmt wurde:
Bekanntlich ist der myocardiale Ca(2+)-Gehalt ein Maß für die hypoxische bzw. durch toxische Catecholamindosen hervorgerufene Herzschädigung (Higgins et al., Mol. Cell. Cardiol. 10, 427-438, 1984; Nakanishi et al., Am. J. Physiol. 242, 437-449, 1982; Fleckenstein, A. Vorträge der Erlanger Physiol. Tagung 1970, Edit. Keidel, Springer Verlag Berlin, Heidelberg, New York, 1971). Umgekehrt ist die Inhibition der hypoxischen oder Isoprenalin-bedingten myocardialen Calciumaufnahme ein Maß für die cardioprotektive Effektivität von Calciumantagonisten (Fleckenstein, s.o.), von Calmodulininhibitoren (Higgins) und anderen Pharmaka, z.B. Betaadrenolytika (Arndts, Arzneimittelforschung 25, 1279-1284, 1975). Die cardioprotektive Wirkung wurde an wachen Ratten nach subcutaner oder peroraler Wirkstoffabgabe anhand der von Arndts (s.o.) beschriebenen Methode festgestellt und die Wirkungsstärke der Testsubstanzen als $H_{50}$-Wert angegeben. Dieser Wert entspricht der Dosis, die die durch eine Gabe von 30 mg/kg s.c. Isoprenalin bedingte myocardiale Radiocalciumaufnahme zu 50 % hemmt.

Hier erwiesen sich die untersuchten neuen Verbindungen als bis zu fünfmal wirksamer als das bekannte Handelsprodukt Propranolol.

| Verbindung | $H_{50}$-Wert* |
|---|---|
| A | 1.25 |
| B | 2.26 |
| C | 1.34 |
| D | 2.25 |
| E | 2.29 |
| F | 1.41 |
| G | 2.23 |
| H | 2.15 |

*perorale Wirkstoffabgabe

Wird ein längere Zeit unter ischämischen Bedingungen gehaltenes, isoliertes Herz wieder normal perfundiert, so tritt keine sofortige Normalisierung der Herzfunktion ein. Es findet sich vielmehr eine vorübergehende Periode, die durch Kontraktur und Arrhytmien gekennzeichnet ist. Diese Arrhythmiephase ist durch Veränderungen von Funktion und Struktur der Myocardzelle mit intrazellulärer Calciumüberladung bedingt (Hess u. Manson, J. Mol. Cell. Cardiol. 16, 969, 1984). Cardioprotektiv wirksame Verbindungen wie Verapamil und Diltiazem verringern die Calciumüberladung und verbessern das Kontraktionsverhalten bei Reperfusion (Watts et al. Am. J. Physiol. 238, H 909, 1980; Meno et al. Am. J. Physiol. 247, H 380, 1984). Die cardioprotektive Wirkung wurde an isolierten Rattenherzen unter Ischämie und anschließender Reperfusion untersucht. Unter Kontrollbedingungen findet sich nach einstündiger Ischämie (Fluß 0,15 ml/min) eine Periode unregelmäßiger Herztätigkeit von 10-15 Minuten. Infusion von cardioprotektiven Verbindungen bewirkt eine signifikante Verkürzung dieser Periode.

| Verbindung | konz. µg/ml | Verkürzung der Arrhythmiephase von 11-13 min auf |
|---|---|---|
| A | 3,3 | 4,0 min |
| I | 13,3 | 3,5 min |
| K | 6,6 | 3,1 min |
| L | 3,3 | 5,0 min |

Verbindungen der Testergebnisse

Verbindung A: Verbindung der Tabelle 1

| | | | |
|---|---|---|---|
| $R_{11}'$ | : $CH_3O$ | $R_5$ | : H |
| $R_{11}''$ | : $CH_3O$ | $R_{12}'$ | : $CH_3O$ |
| $R_1$ | : H | $R_{12}''$ | : $CH_3O$ |
| $R_2$-$R_3$ | : $-(CH_2)_4-$ | $R_6$-$R_7$ | : $-(CH_2)_4-$ |

Fp°C/Salzform : 119/BS

Verbindungen der Tabelle 4 (Strukturtyp I)

| Verb. | $R'_{11}$ | $R''_{11}$ | $R_1$ | $R_2$ | $R_3$ | $R_4$ | Salzform |
|---|---|---|---|---|---|---|---|
| B | $CH_3O$ | $CH_3O$ | H | H | H | $NH-N-$ $-(CH_3)_2$ | HCl |
| C | $CH_3O$ | $CH_3O$ | $CH_3-$ $-(CH_2)_3$ | H | H | $NH-CH_2-$ $-CH(CH_3)_2$ | BS |
| D | $CH_3O$ | $CH_3O$ | $CH_3-$ $-(CH_2)_3$ | H | H | $NH-CH-$ $-(CH_3)_2$ | BS |
| E | $CH_3O$ | $CH_3O$ | $CH_3-$ $-(CH_2)_3$ | H | H | $NH-$ $-(CH_2)_2-$ 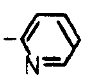 | BS |
| F | $CH_3O$ | $CH_3O$ | $CH_3-$ $-(CH_2)_3$ | H | H | $NHCH_3$ | BS |
| G | $CH_3O$ | $CH_3O$ | $CH_3-$ $-(CH_2)_3$ | H | H | $NH-$ $-(CH_2)_2-OCH_3$ | BS |
| H | $CH_3O$ | $CH_3O$ | $CH_3-$ $-(CH_2)_3$ | H | H | $NH-CH_2-$ $-CH(OH)-CH_3$ | HCl |
| I | $CH_3O$ | $CH_3O$ | H | H | H | 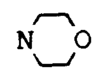 | |
| K | $CH_3O$ | $CH_3O$ | $CH_3-$ $-(CH_2)_3$ | H | H | $NH-CH(CH_3)-$ $-(CH_2)_3-$ $-CH(CH_3)_2$ | BS |
| L | $CH_3O$ | $CH_3O$ | $C_6H_5-$ $-(CH_2)_2$ | H | H | $NH-$ $(CH_2)_2-$ 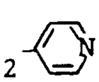 | BS |

In vitro-Untersuchungen an der glatten Muskulatur (Aortenstreifen) haben ergeben, daß es sich bei den erfindungsgemäßen Verbindungen um Calciumantagonisten mit einem neuen Wirkungsmechanismus handelt:

Calciumantagonisten hemmen den transmembranären Calciumioneneinstrom in die Zelle. Diese Hemmung betrifft den spannungsabhängigen (langsamen) Calciumkanal in der Zellmembran. Die Bestimmung

transmembraner Calciumionenströme an Gewebestreifen unter Kaliumdepolarisation nach der von van Breemen beschriebenen Methode weist Calciumantagonisten eindeutig nach (van Breemen et al., Chest. 78, 157 S - 165 S, 1980; van Breemen et al., Am J. Cardiol. 49, 507 - 510, 1982; Casteels et al. Pflügers Arch. 392, 139 - 145, 1981; Deth. und van Breemen, J. Membrane Biol. 30, 363 - 380, 1977). In diesen Untersuchungen zeigt sich, daß die erfindungsgemäßen Verbindungen keine klassischen Calciumantagonisten sind.

Aufgrund dieser Befunde kommen die Verbindungen der allgemeinen Formel I bzw. ihre Säureadditionssalze als Wirkstoffe für Arzneimittel zur Behandlung der koronaren Herzkrankheit und des akuten Myocardinfarktes in Frage.

Bei der Prüfung der Überlebensfähigkeit von Tieren in einer geschlossenen Kammer (Hypoxietoleranztest), welche mit einem Gasgemisch, bestehend aus 96,5 % Stickstoff und 3,5 % Sauerstoff durchströmt wurde, wiesen die mit den erfindungsgemäßen Substanzen vorbehandelten Tiere eine statistisch hoch signifikant größere Überlebensfähigkeit auf als Kontrolltiere bzw. mit Diltiazem, Nifedipin oder Verapamil vorbehandelten Tiere. Die mit dieser Methode geprüfte hirnprotektive Wirkung war bereits bei einer Dosis von 5 mg/kg p.o. ausgeprägt. Damit sind die erfindungsgemäßen Verbindungen sowohl hinsichtlich der wirksamen Dosis als auch der tierexperimentell erzielten Leistungsverbesserung den genannten bekannten Substanzen deutlich überlegen.

Aufgrund dieser Befunde können die Verbindungen der allgemeinen Formel I beziehungsweise ihre Säureadditionssalze als Wirkstoffe für Arzneimittel gegen Herzinsuffizienz und cerebrale Stoffwechselstörungen bzw. das hirnorganische Psychosyndrom sowie posttraumatische und alkoholische Hirnschädigungen Verwendung finden.

Ein weiterer Gegenstand der Erfindung sind Arzneimittel, welche die Substanzen der allgemeinen Formel I sowie ihre Salze mit anorganischen oder organischen Sauren enthalten. Die Arzneimittel sind für orale oder parenterale Verabreichung geeignet. Als Arzneimittelformen dienen vorwiegend Tabletten, Dragees, Ampullen und Saftzubereitungen. Die Einzeldosis zu diesen Arzneimittelformen beträgt zwischen 1,0 und 200 mg, vorzugsweise 20 und 50 mg pro 75 kg Körpergewicht. Je nach der Schwere des Falles sind täglich im allgemeinen 1 bis 3 Einzeldosen zu verabreichen.

Die folgenden Beispiele sollen die Erfindung näher erläutern:

Beispiel 1

1-(3,4-Dihydro-6,7-dimethoxy-isochinolin-1-yl)-1-(1,2,3,4-tetrahydro-6,7-dimethoxy-isochinolin-1-yliden)-pentan und

1-(3,4-Dihydro-6,7-dimethoxy-isochinolin-1-yl)-1-(1,2,3,4-tetrahydro-6,7-dimethoxy-2-N-methyl-isochinolin-1-yliden)-pentan

4,9 g n-Butylmalonsäure di-N-[2-(3,4-dimethoxy-phenyl)-ethyl]-amid werden in 20 ml eines Methansulfonsäure-$P_2O_5$-Gemisches(10 Gewichtsprozent $P_2O_5$-Anteil) 1-2 Stunden auf 100 °C erhitzt. Nach beendeter Umsetzung (Dünnschichtchromatographie-Kontrolle) wird das Reaktionsgemisch auf Eis gegossen, mit gesättigter Sodalösung alkalisch gestellt und mit Methylenchlorid extrahiert. Die organische Phase wird mit Wasser gewaschen, über Natriumsulfat getrocknet, im Vakuum eingedampft und der Rückstand an Kieselgel (Eluent: Methylenchlorid: Methanol = 100:5, V:V) aufgetrennt. Dabei wird zuerst die N-H-Verbindung eluiert.

N-H-Verbindung: Fp. = 158-159 °C (Hydrochlorid)
N-$CH_3$-Verbindung: Fp. = 136-137 °C (Hydrochlorid)

Beispiel 2

1-(3,4-Dihydro-6,7-dimethoxy-isochinolin-1-yl)-1-(3,4-dihydro-6,7-dimethoxy-2-N-methyl-isochinolin-1-yliden)-pentan-hydrochlorid

1 g des nach Beispiel 1 dargestellten 1-(3,4-Dihydro-6,7-dimethoxy-isochinolin-1-yl)-1-(1,2,3,4-tetrahydro-6,7-dimethoxy-isochinolin-1-yliden)-pentan werden in 2 ml frisch destilliertem Dimethylsulfat 6 Stunden zum Sieden erhitzt. Nach der üblichen Aufarbeitung wird an Kieselgel (Eluent: $CH_2Cl_2$: MeOH = 100:5, V:V) chromatographiert, das Hydrochlorid gebildet und aus Ethanol/Ether kristallisiert.

Das Produkt ist mit dem nach Beispiel 1 hergestellten identisch.

Beispiel 3

1-(3,4-Dihydro-6,7-dimethoxy-isochinolin-1-yl)-1-(3,4-dihydro-6,7-dimethoxy-1-N-hydroxymethyl-isochinolin-1-yliden)-ethan-hydrochlorid

12 g 1-(3,4-Dihydro-6,7-dimethoxy-isochinolin-1-yl)-1-(3,4-dihydro-6,7-dimethoxy-isochinolin-1-yliden)-ethan werden in einem Gemisch bestehend aus 20 ml 30 %iger Formalinlösung und 10 ml Ameisensäure (98 %ig) 20 Stunden bei Raumtemperatur stehengelassen. Es wird im Wasserstrahlvakuum zur Trockne eingedampft, das Umsetzungsprodukt in $CH_2Cl_2$ aufgenommen, mit verdünnter Sodalösung und nachfolgend mit Wasser gewaschen, die organische Phase über $Na_2SO_4$ getrocknet, das Lösungsmittel im Vakuum abgezogen, der Rückstand in der eben zusreichenden Menge Ethanol aufgenommen und unter Zugabe von etherischer Salzsäure als Hydrochlorid gefällt. Fp = 155 °C

Beispiel 4

$\alpha$-(3,4-Dihydro-6,7-dimethoxy-isochinolin-1-yl)-1,2,3,4-tetrahydro-6,7-dimethoxy-1-benzyliden-isochinolin

4,9 g 1,2,3,4-Tetrahydro-6,7-dimethoxy-1-$\alpha$-{[2-(3,4-diemethoxyphenyl)-ethyl]-aminocarbonyl}-benzyl-isochinolin werden in 20 ml frisch destilliertem Phosphoroxychlorid 4 Stunden zum Sieden erhitzt. Nach beendeter Umsetzung (Dünnschichtchromatographie-Kontrolle) wird überschüssiges $POCl_3$ abdestilliert, der Rückstand zwischen $CH_2Cl_2$ und verdünnter Sodalösung verteilt, die organische Phase wird mit Wasser gewaschen, über $Na_2SO_4$ getrocknet und eingeengt. Der Rückstand wird an Kieselgel (Eluent: $CH_2Cl_2$:MeOH = 100:10, V:V) chromatographiert. Die rasch laufende gelbe Fraktion liefert $\alpha$-(3,4-Dihydro-6,7-dimethoxy-isochinolin-1-yl)-1,2,3,4- tetrahydro-6,7-dimethoxy-1-benzyliden-2-N-phosphono- isochinolin (Fp. > 270 °C (Hydrochlorid)), die nachfolgende rote Zone die N-H-Verbindung (Fp = 95-100 °C), die im Titel angegeben ist.

Beispiel 5

(4,5-Dihydro-thieno[2,3-c]pyridin-1-yl)-4,5,6,7-tetrahydro-1-methyliden-thieno[2,3-c]pyridin

19 g Malonsäure-di-N-[2-(3-thieno-)ethyl]amid werden in 25 ml Phosphoroxychlorid 3 Stunden zum Sieden erhitzt. Sobald kein Ausgangsmaterial mehr nachweisbar ist, wird wie üblich aufgearbeitet, das Reaktionsprodukt an $Al_2O_3$ neutral, Aktivitätsstufe III (Fa. Woelm) (Eluens:$CH_2Cl_2$) gereinigt und das Hydrochlorid gebildet. (Fp. = 233-235 °C)

Beispiel 6

$\alpha$-Isobutylaminocarbonyl-1-pentyl-6,7-dimethoxy-3,4-dihydro-isochinolin

3,8 g (10 mmol) $\alpha$-Isobutylaminocarbonylcapronsäure -N-[2-(3,4-dimethoxyphenyl)-ethyl]-amid werden in 120 ml Acetonitril gelöst und mit 18 ml Phosphoroxychlorid versetzt. Das Reaktionsgemisch wird ca. 2 Stunden auf Rückflußtemperatur erhitzt. Danach wird eingeengt, der Rückstand in 200 ml Methylenchlorid aufgenommen und durch Einrühren in eine Eiswasser-Pottasche Lösung alkalisch gestellt. Nach der üblichen Aufarbeitung - Ausschütteln mit Methylenchlorid, Trocknen der organischen Phase über $Na_2SO_4$, Abziehen des Lösungsmittels etc. - wird über eine Kieselgelsäule ($CH_2Cl_2$/MeOH = 100:2) gereinigt. Fp.: 158-160 °.

Beispiel 7

2-Ethyl-2-(3,4-dihydro-5,6-dimethoxy-1-isochinolinyl)-butan-carbonsäuremethylester

22 g 2-{[2-(3,4-Dimethoxyphenyl)-ethyl]-aminocarbonyl}-2-ethyl-butancarbonsäuremethylester werden in einem Gemisch aus 100 ml Acetonitril und 12 ml Phosphoroxychlorid bis zum völligen Stoffumsatz zum Sieden erhitzt (ca. 2 Stunden). Danach wird wie üblich aufgearbeitet, das Umsetzungsprodukt an Kieselgel (Eluens: $CH_2Cl_2$ : $CH_3OH$ = 100 : 2) gereinigt und das Hydrochlorid gebildet.
Fp = 141-143 °C (Ethanol/Ether)

Beispiele erfindungsgemäßer Verbindungen werden in den folgenden Tabellen zusammengefaßt, die auch analog zu den oben beschriebenen Beispielen hergestellt werden können.

Tabelle 1

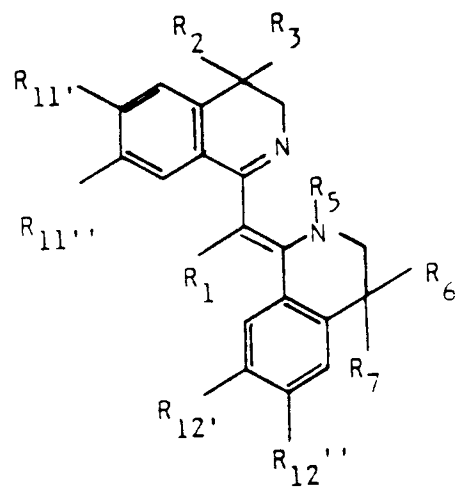

| $R_{11}'$ | $R_{11}''$ | $R_1$ | $R_2$ | $R_3$ | $R_5$ | $R_{12}'$ | $R_{12}''$ | $R_6$ | $R_7$ | Fp°C/Salzform |
|---|---|---|---|---|---|---|---|---|---|---|
| $CH_3O$ | $CH_3O$ | H | $-(CH_2)_4-$ | | H | $CH_3O$ | $CH_3O$ | $-(CH_2)_4-$ | | 119/BS |
| $CH_3SO_2NH$ | $CH_3O$ | H | H | H | H | $CH_3O$ | $CH_3SO_2NH$ | H | H | 233-238 (Z)/BS |
| $CH_3O$ | $CH_3O$ | $n-C_4H_9$ | H | H | $CH_3$ | $CH_3O$ | $CH_3O$ | H | H | 136-137/Cl |
| $CH_3O$ | $CH_3O$ | $n-C_4H_9$ | H | H | H | $CH_3O$ | $CH_3O$ | H | H | 158-159/Cl |
| $CH_3O$ | $CH_3O$ | $CH_3$ | H | H | H | $CH_3O$ | $CH_3O$ | H | H | 180/Cl |
| $CH_3O$ | $CH_3O$ | $CH_3$ | H | H | $CH_3$ | $CH_3O$ | $CH_3O$ | H | H | 177/BS |
| $CH_3O$ | $CH_3O$ | H | H | H | H | $CH_3SO_2O$ | HO | H | H | 182-186/BS |
| $CH_3O$ | $CH_3O$ | H | H | H | $CH_3$ | $CH_3O$ | $CH_3O$ | H | H | 138-140/BS |
| $CH_3O$ | $CH_3SO_2$ | H | H | H | $CH_3$ | HO | $CH_3O$ | H | H | 222-225/BS |
| $CH_3O$ | $CH_3O$ | $CH_3$ | H | H | $CH_2OH$ | $CH_3O$ | $CH_3O$ | H | H | 155/Cl |
| $CH_3O$ | $CH_3O$ | $n-C_5H_{11}$ | H | H | $CH_3$ | $CH_3O$ | $CH_3O$ | H | H | 75-79(amorph)/BS |
| $CH_3O$ | $CH_3O$ | $C_2H_5$ | H | H | $CH_3$ | $CH_3O$ | $CH_3O$ | H | H | 80 (amorph)/BS |
| $CH_3O$ | $CH_3O$ | $C_6H_5$ | H | H | H | $CH_3O$ | $CH_3O$ | H | H | 95-100/BS |
| $CH_3O$ | $CH_3O$ | $CH_3$ | H | H | H | $-O—CH_2—O-$ | | H | H | 156-158/Cl |
| $CH_3O$ | $CH_3O$ | $C_2H_5$ | H | H | H | $-O—CH_2—O-$ | | H | H | 148-155/Cl |
| $CH_3O$ | $CH_3O$ | $C_2H_5$ | H | H | H | $CH_3O$ | $CH_3O$ | H | H | 177-179/Cl |
| $CH_3O$ | $CH_3O$ | H | H | H | H | $-O—CH_2—O-$ | | H | H | 251-253/Cl |
| $-O—CH_2—O-$ | | H | H | H | H | $-O—CH_2—O-$ | | H | H | 251-253(Z)/Cl |
| $CH_3O$ | $CH_3O$ | $CH_3CONH$ | H | H | H | $CH_3O$ | $CH_3O$ | H | H | 171-172/Cl |
| $-O—CH_2—O-$ | | $C_6H_5$ | H | H | H | $-O—CH_2—O-$ | | H | H | 191-195/BS |

BS = Base
Cl = Chlorid
Z = Zersetzung

Tabelle 2

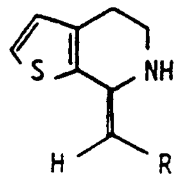

| R | Salzform | Fp(°C) |
|---|---|---|
| 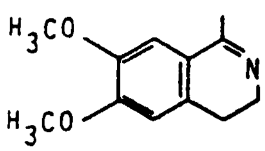 | Cl | 218-222 |
| 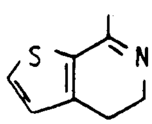 | Cl | 239-235 |

Tabelle 3

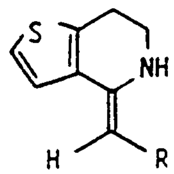

| R | Salzform | Fp(°C) |
|---|---|---|
| 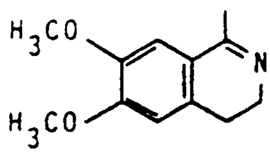 | Cl | 162-172 |
| 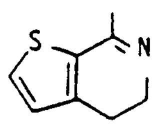 | Cl | 237-239 |
| 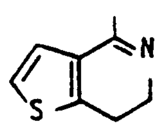 | Cl | 250-253 |

Tabelle 4

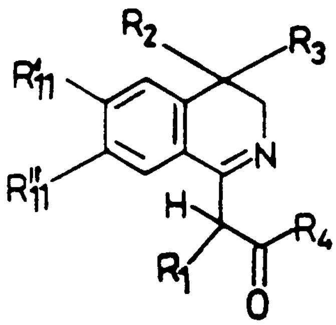

Strukturtyp I

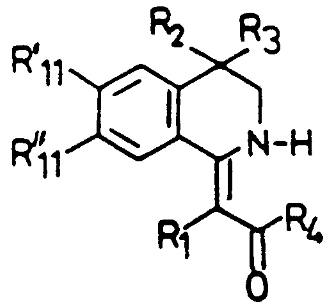

Strukturtyp II

BS = Base

| $R'_{11}$ | $R''_{11}$ | $R_1$ | $R_2$ | $R_3$ | $R_4$ | Struktur-typ | Salz-form | Fp. ($^{o}$ C) |
|---|---|---|---|---|---|---|---|---|
| $CH_3O$ | $CH_3O$ | H | H | H | $NHCH_3$ | I | HCl | 196-197 |
| $CH_3O$ | $CH_3O$ | H | H | H | $N(CH_3)_2$ | I | HCl | 149-150 |
| $CH_3O$ | $CH_3O$ | H | H | H | $NHC_2H_5$ | I | HCl | 212-217 |
| $CH_3O$ | $CH_3O$ | H | H | H | $NH-(CH_2)_2-CH_3$ | I | HCl | 199-204 |
| $CH_3O$ | $CH_3O$ | H | H | H | $N((CH_2)_2-CH_3)_2$ | I | HCl | 94- 98 |
| $CH_3O$ | $CH_3O$ | H | H | H | $NH(CH_2)_3-CH_3$ | I | HCl | 196-198 |
| $CH_3O$ | $CH_3O$ | H | H | H | $NH-(CH_2)_4-CH_3$ | I | BS | 126-128 |
| $CH_3O$ | $CH_3O$ | H | H | H | $NH-CH(CH_3)_2$ | I | HCl | 198-199 |
| $CH_3O$ | $CH_3O$ | H | H | H | $NH-CH_2-CH(CH_3)_2$ | I | HCl | 221-222 |
| $CH_3O$ | $CH_3O$ | H | H | H | $NH-(CH_2)_2-CH(CH_3)_2$ | I | HCl | |
| $CH_3O$ | $CH_3O$ | H | H | H | $NH-CH_2-C=CH_2$ | I | HCl | 113-114 |

| $R'_{11}$ | $R''_{11}$ | $R_1$ | $R_2$ | $R_3$ | $R_4$ | Struktur-typ | Salz-form | Fp. ($^{o}$ C) |
|---|---|---|---|---|---|---|---|---|
| $CH_3O$ | $CH_3O$ | H | H | H | $NH-CH_2-C \equiv CH$ | I | HCl | 216-217 |
| $CH_3O$ | $CH_3O$ | H | H | H | $N(CH_3)-CH_2-CH_2OH$ | I | HCl | 202-203 |
| $CH_3O$ | $CH_3O$ | H | H | H | $NH(CH_2)_3$ 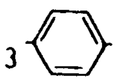 $OCH_3$ | I | | |
| $CH_3O$ | $CH_3O$ | H | H | H | $NH-N(CH_3)_2$ | I | HCl | 132-134 |
| $CH_3O$ | $CH_3O$ | H | H | H | $NHCH_3$ | II | BS | 196-198 |
| $CH_3O$ | $CH_3O$ | H | H | H | $N(CH_3)_2$ | II | HCl | 224-226 |
| $CH_3O$ | $CH_3O$ | H | H | H | $N(C_2H_5)_2$ | II | BS | 88- 91 |
| $CH_3O$ | $CH_3O$ | H | H | H | $NH(CH_2)_4-CH_3$ | II | BS | 115-121 |
| $CH_3O$ | $CH_3O$ | H | H | H | $NH-(CH_2)_3-OCH_3$ | II | BS | 135-136 |
| $CH_3O$ | $CH_3O$ | H | H | H | $NH-(CH_2)_3-N(CH_3)_2$ | II | BS | 144-147 |

| $R'_{11}$ | $R''_{11}$ | $R_1$ | $R_2$ | $R_3$ | $R_4$ | Strukturtyp | Salzform | Fp. ($^0$ C) |
|---|---|---|---|---|---|---|---|---|
| $CH_3O$ | HO | H | H | H | $N(C_2H_5)_2$ | I | BS | 178-180 |
| $CH_3O$ | HO | H | H | H | $NH(CH_2)_2N(CH_3)_2$ | I | BS | 158-161 |
| $CH_3O$ | HO | H | H | H | $NH(CH_2)_3N(CH_3)_2$ | I | HCl | 160-164 |
| $CH_3O$ | HO | H | H | H | $NH(CH_2)_3OCH_3$ | I | BS | 197-199 |
| $CH_3O$ | HO | H | H | H | 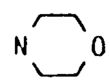 | I | BS | 207-208 |
| $CH_3O$ | HO | H | H | H | $NH(CH_2)_2-CH_3$ | II | BS | 173-180 |
| $CH_3O$ | HO | H | H | H | $NH(CH_2)_3-CH_3$ | II | BS | 148-155 |
| $CH_3O$ | HO | H | H | H | $NH(CH_2)_4-CH_3$ | II | BS | 152-155 |
| $CH_3O$ | HO | H | H | H | $NH(CH_2)_2OCH_3$ | II | BS | 181-182 |
| $CH_3O$ | HO | H | H | H | 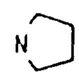 | II | BS | 245-248 |

| R'11 | R''11 | R1 | R2 | R3 | R4 | Struktur-typ | Salz-form | Fp. (° C) |
|---|---|---|---|---|---|---|---|---|
| $O-CH_2-O$ | | H | H | H | $NH-CH_2-CH(OH)-CH_3$ | II | BS | 134-146 |
| $O-CH_2-O$ | | H | H | H | 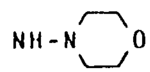 | I | HCl | 180-193 |
| $O-CH_2-O$ | | H | H | H | $NH(CH_2)_3OH$ | II | BS | 133-134 |
| $O-CH_2-O$ | | H | H | H | $NH(CH_2)_2-CH(CH_3)_2$ | II | BS | 129-133 |
| $O-CH_2-O$ | | H | H | H | 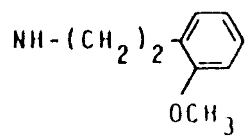 | II | BS | 142-146 |
| $O-CH_2-O$ | | H | H | H | 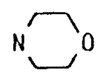 | II | BS | 89-105 (7) |
| $NHSO_2CH_3$ | $CH_3O$ | H | H | H | 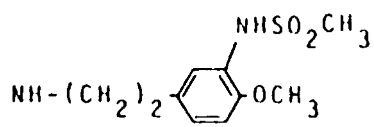 | II | BS | 176 |

| R'11 | R''11 | $R_1$ | $R_2$ | $R_3$ | $R_4$ | Struktur-typ | Salz-form | Fp. ($^{o}$C) |
|---|---|---|---|---|---|---|---|---|
| $CH_3O$ | $CH_3O$ | $CH_3$ | H | H | $NH-CH_2-CH(CH_3)_2$ | I | BS | 138-141 |
| $CH_3O$ | $CH_3O$ | $CH_3$ | H | H | $NH-(CH_2)_4-CH_3$ | I | BS | 114-116 |
| $CH_3O$ | $CH_3O$ | $CH_3$ | H | H | $N[(CH_2)_2CH_3]_2$ | I | BS | ölig |
| $CH_3O$ | $CH_3O$ | $CH_3$ | H | H | $N[(CH_2)_3-CH_3]_2$ | I | BS | ölig |
| $CH_3O$ | $CH_3O$ | $CH_3$ | H | H | $NH-CH(CH_3)-(CH_2)_3-CH(CH_3)_2$ | I | BS | ölig |
| $CH_3O$ | $CH_3O$ | $CH_3$ | H | H | $NH-(CH_2)_2-OCH_3$ | I | BS | 136-139 |
| $CH_3O$ | $CH_3O$ | $CH_3$ | H | H | $NH-CH_2$ 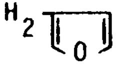 | I | HCl | 189-193 |
| $CH_3O$ | $CH_3O$ | $CH_3$ | H | H | NH- 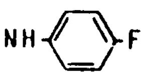 | I | BS | 146-148 |
| $CH_3O$ | $CH_3O$ | $CH_3$ | H | H | $NH-(CH_2)_2-$ 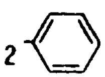 | I | BS | 115-119 |
| $CH_3O$ | $CH_3O$ | $CH_3$ | H | H | $NH-(CH_2)_2-$ 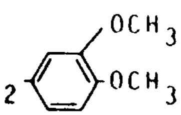 | I | BS | 128-132 |
| $CH_3O$ | $CH_3O$ | $CH_3$ | H | H | $NH-(CH_2)_2-$ 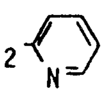 | I | HCl | 185-190 |
| $CH_3O$ | $CH_3O$ | $CH_3$ | H | H | $NH-(CH_2)_2-$ 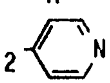 | I | BS | 131-133 |
| $CH_3O$ | $CH_3O$ | $CH_3$ | H | H | $NH-(CH_2)_3-N(CH_3)_2$ | I | BS | 102-105 |

| R'11 | R''11 | R1 | R2 | R3 | R4 | Struktur-typ | Salz-form | Fp. ($^{o}$ C) |
|---|---|---|---|---|---|---|---|---|
| $CH_3O$ | $CH_3O$ | $C_2H_5$ | H | H | $NH-(CH_2)_4-CH_3$ | I | BS | 99-103 |
| $CH_3O$ | $CH_3O$ | $C_2H_5$ | H | H | $NH-CH_2-CH(CH_3)_2$ | I | BS | 111-115 |
| $CH_3O$ | $CH_3O$ | $C_2H_5$ | H | H | $NH-CH_2-CH_2-$ 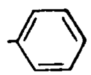 | I | BS | 85- 95 |
| $CH_3O$ | $CH_3O$ | $C_2H_5$ | H | H | $NH-CH_2-CH_2-$ 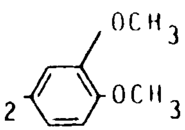 | I | BS | 121-125 |
| $CH_3O$ | $CH_3O$ | $C_2H_5$ | H | H | NH 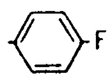 | I | BS | 137-139 |
| $CH_3O$ | $CH_3O$ | $C_2H_5$ | H | H | $NH-CH_2-CH_2-OCH_3$ | I | BS | 110-113 |
| $CH_3O$ | $CH_3O$ | $C_2H_5$ | H | H | $NH-(CH_2)_3-N(CH_3)_2$ | I | BS | 108-148 |
| $CH_3O$ | $CH_3O$ | $C_2H_5$ | H | H | $NH-CH_2$ 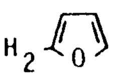 | I | BS | 182-184 |
| $CH_3O$ | $CH_3O$ | $C_2H_5$ | H | H | $NH-(CH_2)_2-$ 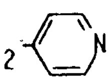 | I | BS | 218-220 |
| $CH_3O$ | $CH_3O$ | $C_2H_5$ | H | H | $NH-(CH_2)_2$ 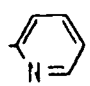 | I | BS | 198-204 |
| $CH_3O$ | $CH_3O$ | $C_2H_5$ | H | H | $NH-CH(CH_3)-(CH_2)_3-CH(CH_3)_2$ | I | BS | 85- 95 |

| $R'_{11}$ | $R''_{11}$ | $R_1$ | $R_2$ | $R_3$ | $R_4$ | Struktur-typ | Salz-form | Fp. ($^{o}$ C) |
|---|---|---|---|---|---|---|---|---|
| $CH_3O$ | $CH_3O$ | $CH_3(CH_2)_3$ | H | H | $NH(CH_2)_3OH$ | I | HCl | 101-103 |
| $CH_3O$ | $CH_3O$ | $CH_3(CH_2)_3$ | H | H | $NH-CH_2-CH(OH)-CH_3$ | I | HCl | 156-158 |
| $CH_3O$ | $CH_3O$ | $CH_3(CH_2)_3$ | H | H | $NH-(CH_2)_2-OCH_3$ | I | BS | 93- 96 |
| $CH_3O$ | $CH_3O$ | $CH_3(CH_2)_3$ | H | H | $NH-(CH_2)_3-OCH_3$ | I | BS | 93- 94 |
| $CH_3O$ | $CH_3O$ | $CH_3(CH_2)_3$ | H | H | $NH-(CH_2)_2-N(CH_3)_2$ | I | BS | 87- 90 |
| $CH_3O$ | $CH_3O$ | $CH_3(CH_2)_3$ | H | H | $NH-(CH_2)_3-N(CH_3)_2$ | I | BS | 87- 90 |
| $CH_3O$ | $CH_3O$ | $CH_3(CH_2)_3$ | H | H | $NH-CH_2-$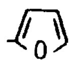 | I | BS | 76- 80 |
| $CH_3O$ | $CH_3O$ | $CH_3(CH_2)_3$ | H | H | $NH-(CH_2)_2-$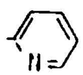 | I | BS | 115-117 |
| $CH_3O$ | $CH_3O$ | $CH_3(CH_2)_3$ | H | H | $N(CH_3)_2$ | I | BS | 108-109 |
| $CH_3O$ | $CH_3O$ | $CH_3(CH_2)_3$ | H | H | $N(C_2H_5)_2$ | I | HCl | 175-177 |
| $CH_3O$ | $CH_3O$ | $CH_3(CH_2)_3$ | H | H | $N((CH_2)_2CH_3)_2$ | I | HCl | 107-110 |
| $CH_3O$ | $CH_3O$ | $CH_3(CH_2)_3$ | H | H | $N((CH_2)_3-CH_3)_2$ | I | HCl | amorph |
| $CH_3O$ | $CH_3O$ | $CH_3(CH_2)_3$ | H | H | 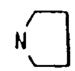 | I | HCl | 193-195 |
| $CH_3O$ | $CH_3O$ | $CH_3(CH_2)_3$ | H | H | 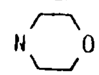 | I | HCl | 195-198 |
| $CH_3O$ | $CH_3O$ | $CH_3(CH_2)_3$ | H | H | 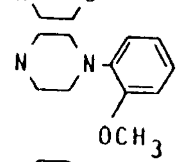 | I | HCl | 103-128 (amorph) |
| $CH_3O$ | $CH_3O$ | $CH_3(CH_2)_3$ | H | H | 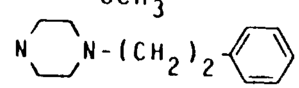 | I | HCl | 93-106 (amorph) |
| $CH_3O$ | $CH_3O$ | $CH_3(CH_2)_3$ | H | H | 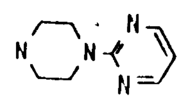 | I | HCl | 92-116 (amorph) |

| $R'_{11}$ | $R''_{11}$ | $R_1$ | $R_2$ | $R_3$ | $R_4$ | Struktur-typ | Salz-form | Fp. ($^oC$) |
|---|---|---|---|---|---|---|---|---|
| $CH_3O$ | $CH_3O$ | $CH_3(CH_2)_3$ | H | H | $NHCH_3$ | I | BS | 94-102 |
| $CH_3O$ | $CH_3O$ | $CH_3(CH_2)_3$ | H | H | $NHC_2H_5$ | I | BS | 119-123 |
| $CH_3O$ | $CH_3O$ | $CH_3(CH_2)_3$ | H | H | $NH(CH_2)_2-CH_3$ | I | BS | 100-103 |
| $CH_3O$ | $CH_3O$ | $CH_3(CH_2)_3$ | H | H | $NH(CH_2)_3-CH_3$ | I | BS | 110-112 |
| $CH_3O$ | $CH_3O$ | $CH_3(CH_2)_3$ | H | H | $NH(CH_2)_4-CH_3$ | I | BS | 108-109 |
| $CH_3O$ | $CH_3O$ | $CH_3(CH_2)_3$ | H | H | $NH-CH(CH_3)_2$ | I | BS | 126-128 |
| $CH_3O$ | $CH_3O$ | $CH_3(CH_2)_3$ | H | H | $NH-CH_2-CH(CH_3)_2$ | I | BS | 158-160 |
| $CH_3O$ | $CH_3O$ | $CH_3(CH_2)_3$ | H | H | $NH(CH_2)_2CH(CH_3)_2$ | I | BS | 93- 95 |
| $CH_3O$ | $CH_3O$ | $CH_3(CH_2)_3$ | H | H | $NH-CH(C_2H_5)-CH_3$ | I | BS | 110-112 |
| $CH_3O$ | $CH_3O$ | $CH_3(CH_2)_3$ | H | H | $NH-CH(CH_3)-(CH_2)_3-CH(CH_3)_2$ | I | BS | 108-110 |
| $CH_3O$ | $CH_3O$ | $CH_3(CH_2)_3$ | H | H | $NHC(CH_3)_3$ | I | BS | 88- 92 |
| $CH_3O$ | $CH_3O$ | $CH_3(CH_2)_3$ | H | H | $NH-(CH_2)_2$- 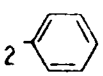 | I | BS | 77- 80 |
| $CH_3O$ | $CH_3O$ | $CH_3(CH_2)_3$ | H | H | $NH-(CH_2)_2$- 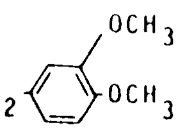 | I | BS | 111-113 |
| $CH_3O$ | $CH_3O$ | $CH_3(CH_2)_3$ | H | H | $NH-(CH_2)_2$- 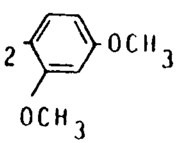 | I | BS | 120-125 |
| $CH_3O$ | $CH_3O$ | $CH_3(CH_2)_3$ | H | H | $NH-CH_2-CH=CH_2$ | I | HCl | 158-160 |
| $CH_3O$ | $CH_3O$ | $CH_3(CH_2)_3$ | H | H | $NH-(CH_2)_2-OH$ | I | BS | 105-107 |

| $R'_{11}$ | $R''_{11}$ | $R_1$ | $R_2$ | $R_3$ | $R_4$ | Struktur-typ | Salz-form | Fp. ($^{o}$ C) |
|---|---|---|---|---|---|---|---|---|
| $CH_3O$ | $CH_3O$ | $CH_3(CH_2)_3$ | H | H | NH- 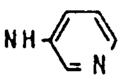 | II | HCl | 108-156 (amorph) |
| $CH_3O$ | $CH_3O$ | $CH_3(CH_2)_3$ | H | H | NH- 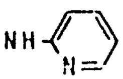 | II | BS | 85- 87 |
| $CH_3O$ | H | $CH_3(CH_2)_3$ | H | H | $NH(CH_2)_4-CH_3$ | I | BS | 83 |
| $CH_3O$ | $CH_3O$ | H | H | H | 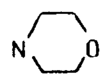 | | | |

| R'11 | R''11 | R1 | R2 | R3 | R4 | Struktur-typ | Salz-form | Fp. (°C) |
|---|---|---|---|---|---|---|---|---|
| $CH_3O$ | $CH_3O$ | $CH_3(CH_2)_4$ | H | H | $NH-(CH_2)_4-CH_3$ | I | BS | 100-102 |
| $CH_3O$ | $CH_3O$ | $CH_3(CH_2)_4$ | H | H | $NH-CH_2-CH(CH_3)_2$ | I | BS | 76- 79 |
| $CH_3O$ | $CH_3O$ | $CH_3(CH_2)_4$ | H | H | $NH-(CH_2)_2-CH(CH_3)_2$ | I | BS | 92- 96 |
| $CH_3O$ | $CH_3O$ | $CH_3(CH_2)_4$ | H | H | $NH-CH(CH_3)-(CH_2)_3-CH(CH_3)_2$ | I | BS | amorph |
| $CH_3O$ | $CH_3O$ | $CH_3(CH_2)_4$ | H | H | NH- 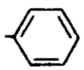 | I | BS | 111 |
| $CH_3O$ | $CH_3O$ | $CH_3(CH_2)_4$ | H | H | $NH-CH_2-$ 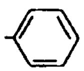 | I | BS | 102-103 |
| $CH_3O$ | $CH_3O$ | $CH_3(CH_2)_4$ | H | H | $NH-(CH_2)_2-$ 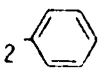 | I | BS | 80 |
| $CH_3O$ | $CH_3O$ | $CH_3(CH_2)_4$ | H | H | $NH-CH_2-CH=CH_2$ | I | BS | 88- 90 |
| $CH_3O$ | $CH_3O$ | $CH_3(CH_2)_4$ | H | H | $NH-CH_2-$ 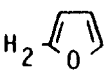 | I | BS | 141-142 |
| $CH_3O$ | $CH_3O$ | $CH_3(CH_2)_4$ | H | H | $NH-(CH_2)_2-$ 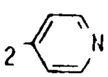 | I | BS | 74- 78 |
| $CH_3O$ | $CH_3O$ | $CH_3(CH_2)_4$ | H | H | $NH-(CH_2)_2-$ 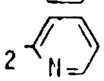 | I | BS | 102-104 |
| $CH_3O$ | $CH_3O$ | $CH_3(CH_2)_4$ | H | H | $NH-(CH_2)_3-N(CH_3)_2$ | I | BS | amorph |
| $CH_3O$ | $CH_3O$ | $CH_3(CH_2)_4$ | H | H | $NH-(CH_2)_2-$ 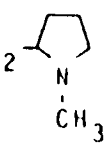 | I | BS | 865- 87 |
| $CH_3O$ | $CH_3O$ | $CH_3(CH_2)_4$ | H | H | $N((CH_2)_3CH_3)_2$ | I | BS | 157-160 |

| $R'_{11}$ | $R''_{11}$ | $R_1$ | $R_2$ | $R_3$ | $R_4$ | Struktur-typ | Salz-form | Fp. ($^o$ C) |
|---|---|---|---|---|---|---|---|---|
| $CH_3O$ | $CH_3O$ | $C_6H_5-(CH_2)_2$ | H | H | $NH-(CH_2)_2-$ 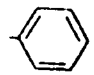 | I | BS | 133-135 |
| $CH_3O$ | $CH_3O$ | $C_6H_5-(CH_2)_2$ | H | H | NH 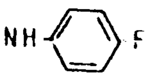 | I | BS | 133 |
| $CH_3O$ | $CH_3O$ | $C_6H_5-(CH_2)_2$ | H | H | $NH-CH_2-$ 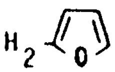 | I | BS | 152-153 |
| $CH_3O$ | $CH_3O$ | $C_6H_5-(CH_2)_2$ | H | H | NH 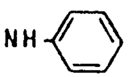 | I | BS | 137-139 |
| $CH_3O$ | $CH_3O$ | $C_6H_5-(CH_2)_2$ | H | H | $NH-CH_2-$ 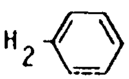 | I | BS | 128-130 |
| $CH_3O$ | $CH_3O$ | $C_6H_5-(CH_2)_2$ | H | H | $NH-(CH_2)_2-$ 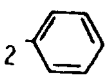 | I | BS | 107-108 |
| $CH_3O$ | $CH_3O$ | $C_6H_5-(CH_2)_2$ | H | H | $NH-(CH_2)_2-$ 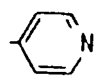 | I | BS | 85- 86 |
| $CH_3O$ | $CH_3O$ | $C_6H_5-(CH_2)_2$ | H | H | $NH(CH_2)_2-$ 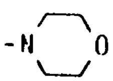 | I | BS | 86- 87 |
| $CH_3O$ | $CH_3O$ | $C_6H_5-(CH_2)_2$ | H | H | $NH-CH_2-$ 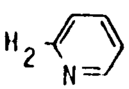 | I | BS | 141-142 |
| $CH_3O$ | $CH_3O$ | $C_6H_5-(CH_2)_2$ | H | H | $NH-CH_2-CH_2OH$ | I | BS | 99-101 |

Tabelle 5

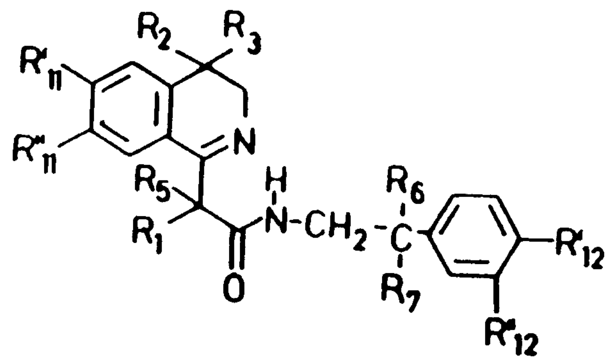

IV

| $R_{11}'$ | $R_{11}''$ | $R_1$ | $R_2$ | $R_3$ | $R_5$ | $R_{12}'$ | $R_{12}''$ | $R_6$ | $R_7$ |
|---|---|---|---|---|---|---|---|---|---|
| $CH_3O$ | $CH_3O$ | H | $-(CH_2)_4-$ | | H | $CH_3O$ | $CH_3O$ | $-(CH_2)_4-$ | |
| $CH_3O$ | $CH_3O$ | $n-C_4H_9$ | H | H | $CH_3$ | $CH_3O$ | $CH_3O$ | H | H |
| $CH_3O$ | $CH_3O$ | $CH_3$ | H | H | $CH_3$ | $CH_3O$ | $CH_3O$ | H | H |
| $CH_3O$ | $CH_3O$ | H | H | H | H | $CH_3SO_2O$ | HO | H | H |
| $CH_3O$ | $CH_3O$ | H | H | H | $CH_3$ | $CH_3O$ | $CH_3O$ | H | H |
| $CH_3O$ | $CH_3SO$ | H | H | H | $CH_3$ | HO | $CH_3O$ | H | H |
| $CH_3O$ | $CH_3O$ | $n-C_5H_{11}$ | H | H | $CH_3$ | $CH_3O$ | $CH_3O$ | H | H |
| $CH_3O$ | $CH_3O$ | $C_2H_5$ | H | H | $CH_3$ | $CH_3O$ | $CH_3O$ | H | H |
| $CH_3O$ | $CH_3O$ | $CH_3$ | H | H | H | $-O-CH_2-O-$ | | H | H |
| $CH_3O$ | $CH_3O$ | $C_2H_5$ | H | H | H | $-O-CH_2-O-$ | | H | H |
| $CH_3O$ | $CH_3O$ | H | H | H | H | $-O-CH_2-O-$ | | H | H |
| $-O-CH_2-O-$ | | H | H | H | H | $-O-CH_2-O-$ | | H | H |
| $CH_3O$ | $CH_3O$ | $CH_3CONH$ | H | H | H | $CH_3O$ | $CH_3O$ | H | H |

Tabelle 6

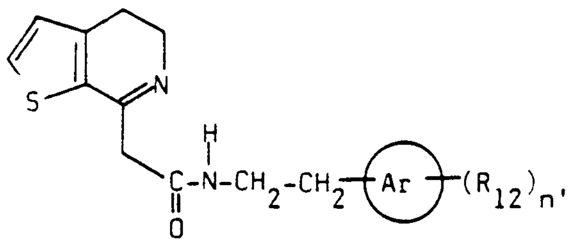

V

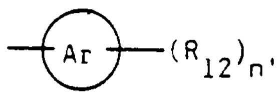

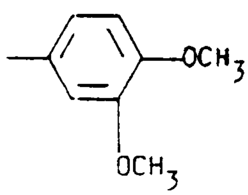

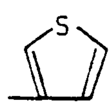

Tabelle 7

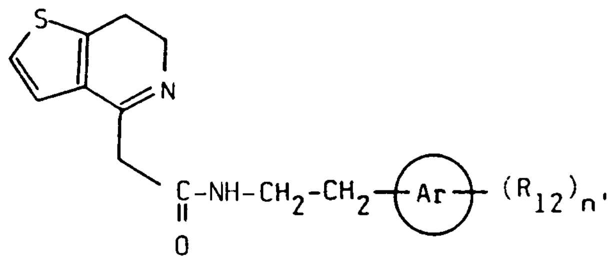

VI

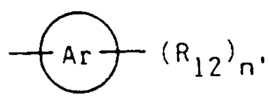

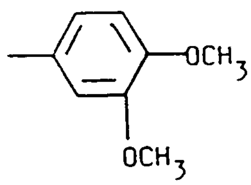

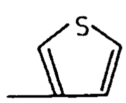

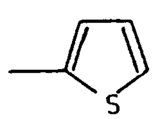

Pharmazeutische Anwendungsbeispiele

a) Dragees

1 Drageekern enthält:

| | |
|---|---|
| Wirkstoff der allgemeinen Formel I | 30,0 mg |
| Milchzucker | 100,0 mg |
| Maisstärke | 75,0 mg |
| Gelatine | 3,0 mg |
| Magnesiumstearat | 2,0 mg |
| | 210,0 mg |

Herstellung

Die Mischung der Wirksubstanz mit Milchzucker und Maisstärke wird mit einer 10%-igen wässerigen Gelatinelösung durch ein Sieb mit 1 mm Maschenweite granuliert, bei 40 °C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässerigen Suspension von Zucker, Titandioxyd, Talkum und Gummi arabicum aufgebracht wird. Die fertigen Dragees werden mit Bienenwachs poliert.

b) Tabletten

| | |
|---|---|
| Wirkstoff der allgemeinen Formel I | 30,0 mg |
| Milchzucker | 100,0 mg |
| Maisstärke | 70,0 mg |
| löslich Stärke | 7,0 mg |
| Magnesiumstearat | 3,0 mg |
| | 210,0 mg |

Herstellung

Wirkstoff und Magnesiumstearat werden mit einer wässerigen Lösung der löslichen Stärke granuliert, das Granulat getrocknet und innig mit Milchzucker und Maisstärke vermischt. Das Gemisch wird sodann zu Tabletten von 210 mg Gewicht verpreßt.

| c) Kapseln | |
|---|---|
| Wirkstoff gemäß Anspruch 1 | 20,0 mg |
| Milchzucker | 230,0 mg |
| Maisstärke | 40,0 mg |
| Talk | 10,0 mg |
| | 300,0 mg |

Herstellung

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

In diesen Beispielen kann als Wirkstoff z.B. 1-(3,4-Dihydro -6,7-dimethoxyisochinolin-1-yl)-1-(3,4-dihydro-6,7-dimethoxyisochinolin-1-yliden)-ethan, Morpholinocarbonylmethyl-6,7-dimethoxy-3,4-dihydroisochinolin,
6,7-Dimethoxy-3,4-dihydro-isochinolinessigsäuremethylamid,
6,7-Dimethoxy-3,4-dihydro-isochinolinessigsäurediethylamid, oder
6,7-Dimethoxy-3,4-dihydro-isochinolinessigsäurephenylethylamid,

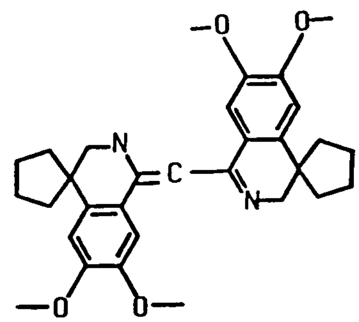

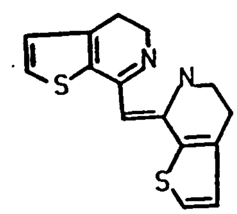

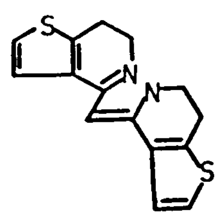

oder

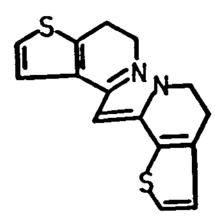

oder ein pharmazeutisch annehmbares Salz davon verwendet werden.

**Patentansprüche**

**Patentansprüche für folgende Vertragsstaaten : AT, BE, CH, DE, FR, GB, IT, LI, LU, NL, SE**

1. Verbindung der allgemeinen Formel I

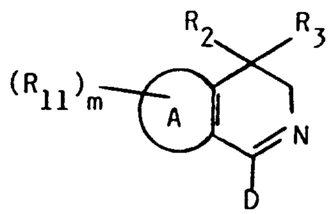

I

worin
A einen Benzo- oder Thienorest bedeutet;
$R_2$ und $R_3$ unabhängig voneinander Wasserstoff oder $(C_1$-$C_5)$Alkyl oder gemeinsam mit dem Kohlenstoffatom, an das sie gebunden sind, einen 5- oder 6-gliedrigen Carbocyclus bedeuten;
$R_{11}$ $(C_1$-$C_4)$Alkyl, Halogen (F, Cl, Br, J), Hydroxy, $(C_1$-$C_4)$Alkoxy, Amino, Thiomethyl, Methansulfonyloxy oder Methansulfonamido bedeutet, oder zwei benachbarte Substituenten $R_{11}$ zusammen $-O-CH_2-O-$ oder $-O-CH_2-CH_2-O-$ sind;
m 0, 1, 2 oder 3 bedeutet, wenn A ein Benzorest ist, und 0, 1 oder 2 bedeutet, wenn A ein Thienorest ist;
und D eine Gruppe der Formel Ia oder Ib bedeutet

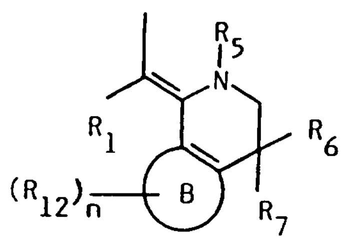

Ia

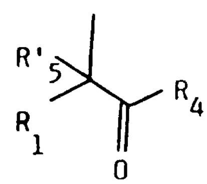

Ib

wobei in der Gruppe der Formel Ia
B einen Benzo- oder Thienorest bedeutet;
$R_1$ Wasserstoff, $(C_1$-$C_{10})$Alkyl, Phenyl, Phenyl$(C_1$-$C_5)$alkyl, $(C_1$-$C_4)$Alkoxy oder -NHCOX (worin X $(C_1$-$C_5)$Alkyl ist) bedeutet;
$R_5$ Wasserstoff, $(C_1$-$C_4)$Alkyl oder Hydroxymethyl bedeutet;
$R_6$ und $R_7$ unabhängig voneinander Wasserstoff oder $(C_1$-$C_5)$Alkyl oder gemeinsam mit dem Kohlenstoffatom, an das sie gebunden sind, einen 5- oder 6-gliedrigen Carbocyclus bedeuten;
$R_{12}$ $(C_1$-$C_4)$Alkyl, Halogen (F, Cl, Br, J), Hydroxy, $(C_1$-$C_4)$Alkoxy, Amino, Thiomethyl, Methansulfonyloxy oder Methansulfonamido bedeutet, oder zwei benachbarte Substituenten $R_{12}$ zusammen $-O-CH_2-O-$ oder $-O-CH_2-CH_2-O-$sind;
und n 0, 1, 2 oder 3 bedeutet, wenn B ein Benzorest ist, und 0, 1 oder 2 bedeutet, wenn B ein Thienorest ist:
und in der Gruppe der Formel Ib
$R_1$
Wasserstoff, $(C_1$-$C_{10})$Alkyl, Phenyl$(C_1$-$C_5)$alkyl, $(C_1$-$C_4)$Alkoxy oder -NHCOX (worin X $(C_1$-$C_5)$Alkyl ist) bedeutet;
$R'_5$ Wasserstoff oder $(C_1$-$C_4)$Alkyl bedeutet;
$R_4$ eine $-NR_9R_{10}$-Gruppe bedeutet, worin
$R_9$ und $R_{10}$ unabhängig voneinander (a) Wasserstoff, (b) verzweigtes oder unverzweigtes Alkyl, Alkenyl oder Alkinyl mit 1-12 Kohlenstoffatomen (wobei das Alkyl substituiert sein kann durch Hydroxy, $(C_1$-$C_4)$-Alkoxy, Di$(C_1$-$C_4)$Alkylamino, Furyl, Pyrrolidinyl, Morpholinyl, Pyridinyl, Indolyl, oder die Gruppe

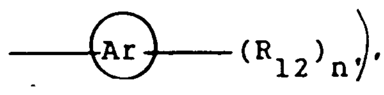

(c) Cycloalkyl mit 3-7 Ringgliedern, (d) Dimethylamino, (e) Amino($C_2$-$C_4$)alkyl (worin die Aminogruppe unsubstituiert sein kann oder Mono- oder Di-($C_1$-$C_4$)alkylamino ist), (f) Phenyl, (g) Morpholinyl, oder (h) Pyrridinyl bedeuten, wobei $R_9$ und $R_{10}$ nicht gleichzeitig Wasserstoff, Dimethylamino oder Di($C_1$-$C_4$)-alkylaminomethyl sein können;

oder $R_9$ und $R_{10}$ gemeinsam mit dem Stickstoffatom, an das sie gebunden sind, einen Pyrrolidinyl-, Piperidinyl-, Morpholinyl- oder Piperazinylrest bedeuten, wobei der Piperazinylring gegebenenfalls durch unsubstituiertes Phenyl, Mono- oder Di($C_1$-$C_4$)alkoxyphenyl, Pyrimidinyl oder Phenyl($C_1$-$C_4$)alkyl N-substituiert sein kann;

$R_{12}$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R_{12}$ zusammen -O-$CH_2$-O- oder -O-$CH_2$-$CH_2$-O- sind;

Ar Phenyl oder Thienyl ist;

n' 0, 1, 2 oder 3 bedeutet, wenn Ar Phenyl ist und 0, 1 oder 2 bedeutet, wenn Ar Thienyl ist;

und ihre pharmazeutisch annehmbaren Salze mit anorganischen oder organischen Säuren;

ausgenommen die Verbindung der Formel I, worin D die Gruppe Ia ist und $R_1$, $R_2$, $R_3$, $R_5$, $R_6$ und $R_7$ Wasserstoff und A und B den Rest

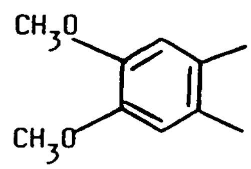

bedeuten;

und

ausgenommen die Verbindung der Formel I, worin D die Gruppe der Formel Ib ist und $R_1$, $R_2$, $R_3$ und $R'_5$ Wasserstoff, A den Rest

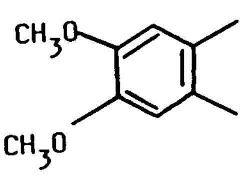

und $R_4$ die Gruppe

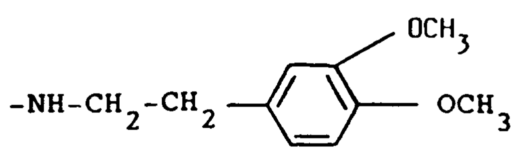

bedeuten.

**2.** Verbindung nach Anspruch 1, worin D die Gruppe der Formel Ia bedeutet; $R_1$ Wasserstoff, ($C_1$-$C_{10}$)-Alkyl, Phenyl($C_1$-$C_5$)alkyl oder -NHCOX (worin X ($C_1$-$C_5$)Alkyl ist) bedeutet;

$R_{11}$ und $R_{12}$ unabhängig voneinander ($C_1$-$C_4$)Alkyl, Hydroxy, ($C_1$-$C_4$)Alkoxy, Methansulfonyloxy oder Methansulfonamido bedeuten, oder zwei benachbarte Substituenten $R_{11}$ beziehungsweise $R_{12}$ zusammen -O-$CH_2$-O- oder -O-$CH_2$-$CH_2$-O- sind;

$R_2$, $R_3$, $R_6$ und $R_7$ unabhängig voneinander Wasserstoff bedeuten oder $R_2$ mit $R_3$ und/oder $R_6$ mit $R_7$ und dem jeweiligen Kohlenstoffatom, an das sie gebunden sind, einen 5- oder 6-gliedrigen Carbocyclus bedeuten;
$R_{11}$ und $R_{12}$ unabhängig voneinander Hydroxy, $(C_1-C_4)$Alkoxy, Methansulfonyloxy oder Methansulfonamido bedeuten, oder zwei benachbarte Substituenten $R_{11}$ beziehungsweise $R_{12}$ zusammen $-O-CH_2-O-$ oder $-O-CH_2-CH_2-O-$ sind.

**3.** Verbindung nach Anspruch 2, worin $R_1$ Wasserstoff, $(C_1-C_{10})$Alkyl oder -NHCOX (worin X $(C_1-C_5)$Alkyl ist) bedeutet.

**4.** Verbindung nach Anspruch 2,
worin $R_1$ Wasserstoff, $(C_1-C_6)$Alkyl, oder $-NHCOCH_3$ bedeutet und/oder
$R_5$ Wasserstoff, Methyl oder Hydroxymethyl bedeutet und/oder
$R_2$, $R_3$, $R_6$ und $R_7$ unabhängig voneinander Wasserstoff bedeuten oder $R_2$ mit $R_3$ und/oder $R_6$ mit $R_7$ und dem jeweiligen Kohlenstoffatom, an das sie gebunden sind, einen 5-gliedrigen Carbocyclus bedeuten und/oder
$R_{11}$ und $R_{12}$ unabhängig voneinander Hydroxy, Methoxy, Methansulfonoxy oder Methansulfonamido bedeuten, oder zwei benachbarte Substituenten $R_{11}$ beziehungsweise $R_{12}$ zusammen $-O-CH_2-O-$ sind.

**5.** Verbindung nach einem der Ansprüche 1 bis 4, worin D die Gruppe der Formel Ia bedeutet und m und/oder n 2 bedeuten.

**6.** Verbindung nach Anspruch 5, worin D die Gruppe der Formel Ia bedeutet und, wenn A und/oder B ein Benzorest ist, die beiden Substituenten $R_{11}$ und/oder $R_{12}$ im Benzorest in metabeziehungsweise para-Position zu den Fusionspunkten des Restes A beziehungsweise B stehen und worin vorzugsweise $R_{11}$ und $R_{12}$ Methoxy sind.

**7.** Verbindung nach Anspruch 1, nämlich

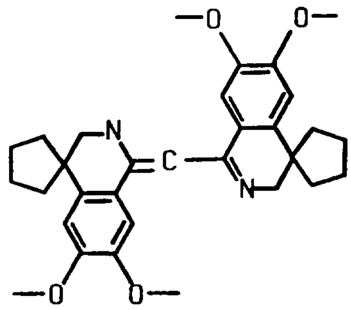

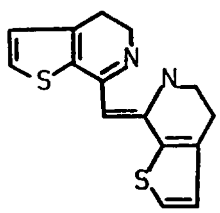

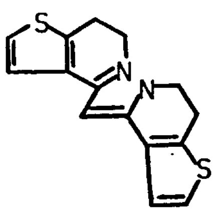

oder

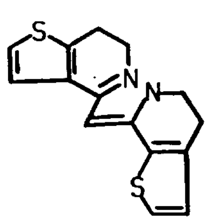

oder ihr physiologisch unbedenkliches Salz.

**8.** Verbindung nach Anspruch 1, worin D die Gruppe der Formel Ib bedeutet und $R_4$ eine $-NR_9R_{10}$-Gruppe bedeutet, worin
$R_9$ und $R_{10}$ unabhängig voneinander (a) Wasserstoff, (b) Alkyl mit 1 bis 8 Kohlenstoffatomen, Alkenyl oder Alkinyl mit 2 oder 3 Kohlenstoffatomen (wobei das Alkyl substituiert sein kann durch Hydroxy, ($C_1$-$C_4$)Alkoxy, Di($C_1$-$C_4$)Alkylamino, Furyl, Pyrrolidine, Morpholinyl, Pyridinyl oder die Gruppe

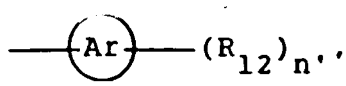

worin Ar, $R_{12}$ und n' wie in Anspruch 1 definiert sind), (d) Dimethylamino, (f) Phenyl, (g) Morpholinyl, (h) Pyrridinyl bedeuten, wobei $R_9$ und $R_{10}$ nicht gleichzeitig Wasserstoff. Dimethylamino oder Di($C_1$-$C_4$)alkylaminomethyl sein können;

oder $R_9$ und $R_{10}$ gemeinsam mit dem Stickstoffatom, an das sie gebunden sind, einen Pyrrolidinyl-, Morpholinyl- oder Piperazinylrest bedeuten, wobei der Piperazinylring gegebenenfalls durch unsubstituiertes Phenyl, Mono- oder Di($C_1$-$C_4$)alkoxyphenyl, Pyrimidinyl oder Phenyl ($C_1$-$C_4$)alkyl N-substituiert sein kann.

**9.** Verbindung nach Anspruch 8, worin $R_4$ eine $-NR_9R_{10}$-Gruppe ist, worin $R_9$ Wasserstoff ist und $R_{10}$ ein substituiertes Alkyl der Formel VII ist,

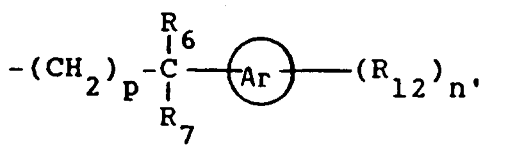

VII

worin p O, 1 oder 2 ist;

$R_6$ und $R_7$ unabhängig voneinander Wasserstoff oder ($C_1$-$C_5$)Alkyl oder gemeinsam mit dem Kohlenstoffatom, an das sie gebunden sind, einen 5- oder 6-gliedrigen Carbocyclus bedeuten;

Ar, Phenyl oder Thienyl bedeutet;

$R_{12}$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R_{12}$ zusammen $-O-CH_2-O-$ oder $-O-CH_2-CH_2-O-$ sind und

n' 0, 1, 2 oder 3 bedeutet, wenn Ar Phenyl ist, und O 1 oder 2 bedeutet, wenn Ar Thienyl ist.

**10.** Verbindung nach einem der Ansprüche 1, 8 und 9, worin D die Gruppe der Formel Ib bedeutet, $R_1$ Wasserstoff, ($C_1$-$C_6$)Alkyl oder $-NHCOCH_3$ bedeutet;

$R_{11}$ Hydroxy, ($C_1$-$C_4$)Alkoxy, Methansulfonyloxy oder Methansulfonamido bedeutet, oder zwei benachbarte Substituenten $R_{11}$ zusammen $-O-CH_2-O-$ oder $-O-CH_2-CH_2-O-$ sind;

und

$R_2$ und $R_3$ Wasserstoff bedeuten oder zusammen mit dem Kohlenstoffatom, an das sie gebunden sind, einen 5- oder 6-gliedrigen Carbocyclus bedeuten;

und $R'_5$ Wasserstoff, Methyl oder Ethyl bedeutet.

**11.** Verbindung nach einem der Ansprüche 1 und 8 bis 10 worin A ein Benzorest ist, m 2 bedeutet und die beiden Substituenten $R_{11}$ in meta- beziehungsweise para-Position zu den Fusionspunkten des Restes A stehen, worin $R_{11}$ vorzugsweise Methoxy ist.

**12.** Verbindung nach Anspruch 1, worin D die Gruppe der Formel Ib ist, A Benzo, $R_{11}$ Methoxy, m zwei, $R_1$ Wasserstoff oder ($C_1$-$C_5$)alkyl, $R_2$, $R_3$ und $R'_5$ Wasserstoff und $R_4$ Morpholino, Methylamino, Diethylamino oder Phenethylamino bedeuten.

**13.** Verbindung nach Anspruch 1, nämlich Morpholinocarbonylmethyl-6,7-dimethoxy-3,4-dihydro-isochinolin,
6,7-Dimethoxy-3,4-dihydro-isochinolinessigsäuremethylamid,
6,7-Dimethoxy-3,4-dihydro-isochinolinessigsäurediethylamid oder
6,7-Dimethoxy-3,4-dihydro-isochinolinessigsäurephenylethylamid.

**14.** Verfahren zur Herstellung einer Verbindung nach einem der Ansprüche 1 bis 13, dadurch gekennzeichnet, daß man

A. (zur Herstellung einer Verbindung der Formel I, worin D die Gruppe der Formel Ia ist) eine Verbindung (II),

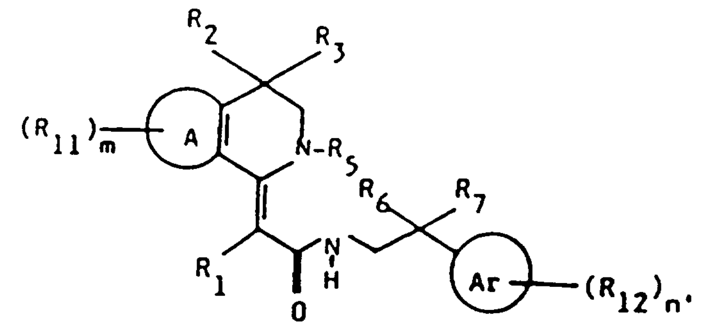

II

worin A, $R_1$, $R_2$, $R_3$, $R_6$, $R_7$, $R_{11}$, $R_{12}$ und m wie in einem der Ansprüche 1 bis 7 definiert sind, Ar Phenyl oder Thienyl bedeutet und n', O, 1, 2 oder 3 bedeutet wenn Ar Phenyl ist, und 0, 1 oder 2 bedeutet, wenn Ar Thienyl ist, und $R_5$ Wasserstoff oder $(C_1-C_4)$Alkyl bedeutet,
in Gegenwart eines Kondensationsmittels kondensiert;
B. (zur Herstellung einer Verbindung der Formel I, worin D die Gruppe der Formel Ia ist) eine Verbindung (IIIa),

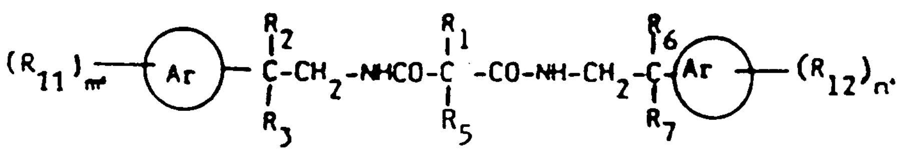

IIIa

in der $R_5$ Wasserstoff ist, $R_1$, $R_2$, $R_3$, $R_6$, $R_7$, $R_{11}$ und $R_{12}$ wie in einem der Ansprüche 1 bis 7 definiert sind, Ar Phenyl oder Thienyl bedeutet und m' und n' unabhängig voneinander 0, 1, 2 oder 3 bedeuten, wenn das zugehörige Ar Phenyl ist, und O, 1 oder 2 bedeuten, wenn das zugehörige Ar Thienyl ist;
in Gegenwart eines Kondensationsmittels kondensiert ohne das Zwischenprodukt der allgemeinen Formel (II) zu isolieren;
C. (zur Herstellung einer Verbindung der Formel I, worin D die Gruppe der Formel Ib ist) eine Verbindung III

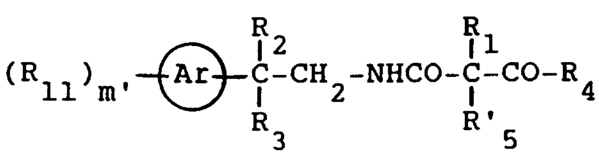

III

in der $R'_5$ Wasserstoff ist, $R_1$, $R_2$, $R_3$ und $R_{11}$ wie in einem der Ansprüche 1 und 8 bis 13 definiert sind, Ar Phenyl oder Thienyl bedeutet und m' 0, 1, 2 oder 3 bedeutet, wenn Ar Phenyl ist, und 0, 1 oder 2 bedeutet, wenn Ar Thienyl ist;
in Gegenwart eines Kondensationsmittels kondensiert;
und daß man gegebenenfalls eine oder mehrere der folgenden Nachbehandlungen anschließt:
Alkylieren einer Verbindung (I), in der $R_5$ beziehungsweise $R'_5$ Wasserstoff ist, zu einer Verbindung (I) in der $R_5$ beziehungsweise $R'_5$ $(C_1-C_4)$Alkyl ist;
Hydroxymethylieren einer Verbindung (I), in der D die Gruppe der Formel Ia ist und $R_5$ Wasserstoff ist, zu einer Verbindung, in der $R_5$ Hydroxymethylen ist;
Isolieren der einzelnen von der Formel I umfaßten Tautomeren;
Isolieren der freien Verbindung (I) aus ihrem Salz;
Umsetzen einer Verbindung (I) zu ihrem pharmazeutisch annehmbaren Salz.

**15.** Pharmazeutische Zubereitung, enthaltend als Wirkstoff eine oder mehrere Verbindungen der allgemeinen Formel I

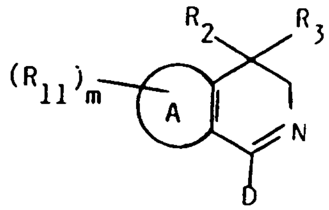

I

worin
A einen Benzo- oder Thienorest bedeutet;
$R_2$ und $R_3$ unabhängig voneinander Wasserstoff oder $(C_1\text{-}C_5)$Alkyl oder gemeinsam mit dem Kohlenstoffatom, an das sie gebunden sind, einen 5- oder 6-gliedrigen Carbocyclus bedeuten;
$R_{11}$ $(C_1\text{-}C_4)$Alkyl, Halogen (F, Cl, Br, J), Hydroxy, $(C_1\text{-}C_4)$Alkoxy, Amino, Thiomethyl, Methansulfonyloxy oder Methansulfonamido bedeutet, oder zwei benachbarte Substituenten $R_{11}$ zusammen $-O-CH_2-O-$ oder $-O-CH_2-CH_2-O-$ sind;
m 0, 1, 2 oder 3 bedeutet, wenn A ein Benzorest ist, und 0, 1 oder 2 bedeutet, wenn A ein Thienorest ist;
und D eine Gruppe der Formel Ia oder Ib bedeutet

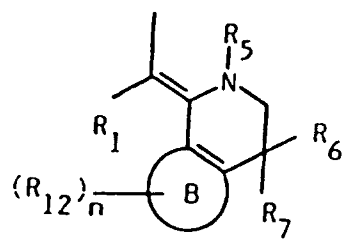

Ia

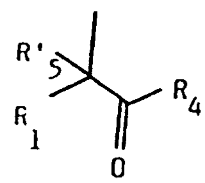

Ib

wobei in der Gruppe der Formel Ia
B einen Benzo- oder Thienorest bedeutet;
$R_1$ Wasserstoff, $(C_1\text{-}C_{10})$Alkyl, Phenyl, Phenyl$(C_1\text{-}C_5)$alkyl, $(C_1\text{-}C_4)$Alkoxy oder -NHCOX (worin X $(C_1\text{-}C_5)$Alkyl ist) bedeutet;
$R_5$ Wasserstoff, $(C_1\text{-}C_4)$Alkyl oder Hydroxymethyl bedeutet;
$R_6$ und $R_7$ unabhängig voneinander Wasserstoff oder $(C_1\text{-}C_5)$Alkyl oder gemeinsam mit dem Kohlenstoffatom, an das sie gebunden sind, einen 5- oder 6-gliedrigen Carbocyclus bedeuten;
$R_{12}$ $(C_1\text{-}C_4)$Alkyl, Halogen (F, Cl, Br, J), Hydroxy, $(C_1\text{-}C_4)$Alkoxy, Amino, Thiomethyl, Methansulfonyloxy oder Methansulfonamido bedeutet, oder zwei benachbarte Substituenten $R_{12}$ zusammen $-O-CH_2-O-$ oder $-O-CH_2-CH_2-O-$sind;
und n 0, 1, 2 oder 3 bedeutet, wenn B ein Benzorest ist, und 0, 1 oder 2 bedeutet, wenn B ein Thienorest ist;
und in der Gruppe der Formel Ib
$R_1$
Wasserstoff, $(C_1\text{-}C_{10})$Alkyl,
Phenyl$(C_1\text{-}C_5)$alkyl, $(C_1\text{-}C_4)$Alkoxy oder -NHCOX (worin X $(C_1\text{-}C_5)$Alkyl ist) bedeutet;
$R'_5$ Wasserstoff oder $(C_1\text{-}C_4)$Alkyl bedeutet;
$R_4$ eine $-NR_9R_{10}$-Gruppe bedeutet, worin
$R_9$ und $R_{10}$ unabhängig voneinander (a) Wasserstoff, (b) verzweigtes oder unverzweigtes Alkyl, Alkenyl oder Alkinyl mit 1-12 Kohlenstoffatomen (wobei das Alkyl substituiert sein kann durch Hydroxy, $(C_1\text{-}C_4)$-Alkoxy, Di$(C_1\text{-}C_4)$Alkylamino, Furyl, Pyrrolidinyl, Morpholinyl, Pyridinyl, Indolyl, oder die Gruppe

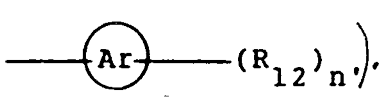

(c) Cycloalkyl mit 3-7 Ringgliedern, (d) Dimethylamino, (e) Amino$(C_2\text{-}C_4)$alkyl (worin die Aminogruppe

unsubstituiert sein kann oder Mono- oder Di-($C_1$-$C_4$)alkylamino ist), (f) Phenyl, (g) Morpholinyl, oder (h) Pyrridinyl bedeuten, wobei $R_9$ und $R_{10}$ nicht gleichzeitig Wasserstoff, Dimethylamino oder Di($C_1$-$C_4$)-alkylaminomethyl sein können;
oder $R_9$ und $R_{10}$ gemeinsam mit dem Stickstoffatom, an das sie gebunden sind, einen Pyrrolidinyl-, Piperidinyl-, Morpholinyl- oder Piperazinylrest bedeuten, wobei der Piperazinylring gegebenenfalls durch unsubstituiertes Phenyl, Mono- oder Di($C_1$-$C_4$)alkoxyphenyl, Pyrimidinyl oder Phenyl($C_1$-$C_4$)alkyl N-substituiert sein kann;
$R_{12}$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R_{12}$ zusammen -0-$CH_2$-O- oder -O-$CH_2$-$CH_2$-O- sind;
Ar Phenyl oder Thienyl ist;
n', 0, 1, 2 oder 3 bedeutet, wenn Ar Phenyl ist und 0,1 oder 2 bedeutet, wenn Ar Thienyl ist oder deren pharmazeutisch annehmbare(s) Salze(e).

**16.** Pharmazeutische Zubereitung nach Anspruch 15, enthaltend eine Verbindung nach einem der Ansprüche 2 bis 13.

**17.** Verwendung einer Verbindung der allgemeinen Formel I, definiert wie in einem der Ansprüche 1 bis 13 und 15 oder deren pharmazeutisch annehmbare(s) Salz(e), für die Herstellung eines Medikaments zu der Behandlung der koronaren Herzkrankheit, des akuten Myocardinfarktes, zur Cardio- und/oder Hirnprotektion.

**Patentansprüche für folgende Vertragsstaaten : ES, GR**

**1.** Verfahren zur Herstellung einer Verbindung der allgemeinen Formel I

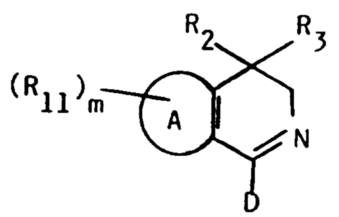

I

worin
A einen Benzo- oder Thienorest bedeutet;
$R_2$ und $R_3$ unabhängig voneinander Wasserstoff oder ($C_1$-$C_5$)Alkyl oder gemeinsam mit dem Kohlenstoffatom, an das sie gebunden sind, einen 5- oder 6-gliedrigen Carbocyclus bedeuten;
$R_{11}$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R_{11}$ zusammen -O-$CH_2$-O- oder -O-$CH_2$-$CH_2$-O- sind;
m 0, 1, 2 oder 3 bedeutet, wenn A ein Benzorest ist, und 0, 1 oder 2 bedeutet, wenn A ein Thienorest ist;
und D eine Gruppe der Formel Ia oder Ib bedeutet

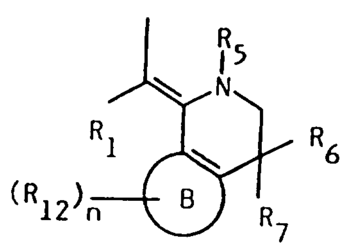

Ia

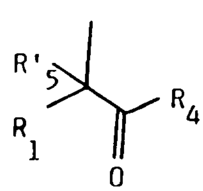

Ib

wobei in der Gruppe der Formel Ia

B einen Benzo- oder Thienorest bedeutet;

$R_1$ Wasserstoff, ($C_1$-$C_{10}$)Alkyl, Phenyl, Phenyl($C_1$-$C_5$)alkyl, ($C_1$-$C_4$)Alkoxy oder -NHCOX (worin X ($C_1$-$C_5$)Alkyl ist) bedeutet;

$R_5$ Wasserstoff, ($C_1$-$C_4$)Alkyl oder Hydroxymethyl bedeutet;

$R_6$ und $R_7$ unabhängig voneinander Wasserstoff oder ($C_1$-$C_5$)Alkyl oder gemeinsam mit dem Kohlenstoffatom, an das sie gebunden sind, einen 5- oder 6-gliedrigen Carbocyclus bedeuten;

$R_{12}$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R_{12}$ zusammen -O-$CH_2$-O- oder -O-$CH_2$-$CH_2$-O-sind;

und n 0, 1, 2 oder 3 bedeutet, wenn B ein Benzorest ist, und 0, 1 oder 2 bedeutet, wenn B ein Thienorest ist;

und in der Gruppe der Formel Ib

$R_1$

Wasserstoff, ($C_1$-$C_{10}$)Alkyl, Phenyl($C_1$-$C_5$)alkyl, ($C_1$-$C_4$)Alkoxy oder -NHCOX (worin X ($C_1$-$C_5$)Alkyl ist) bedeutet;

$R'_5$ Wasserstoff oder ($C_1$-$C_4$)Alkyl bedeutet;

$R_4$ oder eine -$NR_9R_{10}$-Gruppe bedeutet, worin

$R_9$ und $R_{10}$ unabhängig voneinander (a) Wasserstoff, (b) verzweigtes oder unverzweigtes Alkyl, Alkenyl oder Alkinyl mit 1-12 Kohlenstoffatomen (wobei das Alkyl substituiert sein kann durch Hydroxy, ($C_1$-$C_4$)-Alkoxy, Di($C_1$-$C_4$)Alkylamino, Furyl, Pyrrolidinyl, Morpholinyl, Pyridinyl, Indolyl, oder die Gruppe

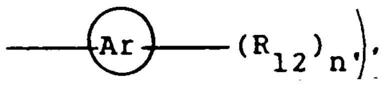

(c) Cycloalkyl mit 3-7 Ringgliedern, (d) Dimethylamino, (e) Amino($C_2$-$C_4$)alkyl (worin die Aminogruppe unsubstituiert sein kann oder Mono- oder Di-($C_1$-$C_4$)alkylamino ist), (f) Phenyl, (g) Morpholinyl, oder (h) Pyrridinyl bedeuten, wobei $R_9$ und $R_{10}$ nicht gleichzeitig Wasserstoff, Dimethylamino oder Di($C_1$-$C_4$)-alkylaminomethyl sein können;

oder $R_9$ und $R_{10}$ gemeinsam mit dem Stickstoffatom, an das sie gebunden sind, einen Pyrrolidinyl-, Piperidinyl-, Morpholinyl- oder Piperazinylrest bedeuten, wobei der Piperazinylring gegebenenfalls durch unsubstituiertes Phenyl, Mono- oder Di($C_1$-$C_4$)alkoxyphenyl, Pyrimidinyl oder Phenyl($C_1$-$C_4$)alkyl N-substituiert sein kann;

$R_{12}$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R_{12}$ zusammen -O-$CH_2$-O- oder -O-$CH_2$-$CH_2$-O- sind;

Ar Phenyl oder Thienyl ist;

n' 0, 1, 2 oder 3 bedeutet, wenn Ar Phenyl ist und 0, 1 oder 2 bedeutet, wenn Ar Thienyl ist;

und ihre pharmazeutisch annehmbaren Salze mit anorganischen oder organischen Säuren;

ausgenommen die Verbindung der Formel I, worin D die Gruppe Ia ist und $R_1$, $R_2$, $R_3$, $R_5$, $R_6$ und $R_7$ Wasserstoff und A und B den Rest

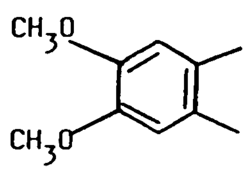

bedeuten;

und

ausgenommen die Verbindung der Formel I, worin D die Gruppe der Formel Ib ist und $R_1$, $R_2$, $R_3$ und $R'_5$ Wasserstoff, A den Rest

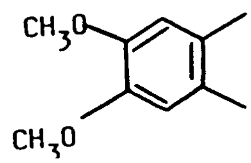

und $R_4$ die Gruppe

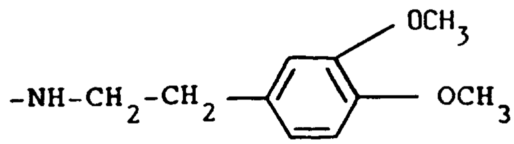

bedeuten,

dadurch gekennzeichnet, daß man

A. (zur Herstellung einer Verbindung der Formel I, worin D die Gruppe der Formel Ia ist) eine Verbindung (II),

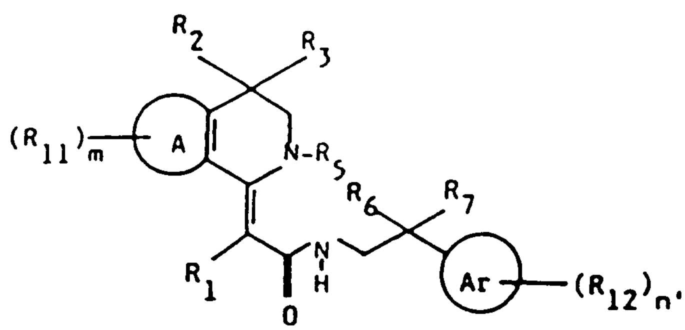

II

worin A, $R_1$, $R_2$, $R_3$, $R_6$, $R_7$, $R_{11}$, $R_{12}$ und m wie oben definiert sind, Ar Phenyl oder Thienyl bedeutet und n', O, 1, 2 oder 3 bedeutet wenn Ar Phenyl ist, und 0, 1 oder 2 bedeutet, wenn Ar Thienyl ist, und $R_5$ Wasserstoff oder $(C_1-C_4)$Alkyl bedeutet, in Gegenwart eines Kondensationsmittels kondensiert;

B. (zur Herstellung einer Verbindung der Formel I, worin D die Gruppe der Formel Ia ist) eine Verbindung (IIIa),

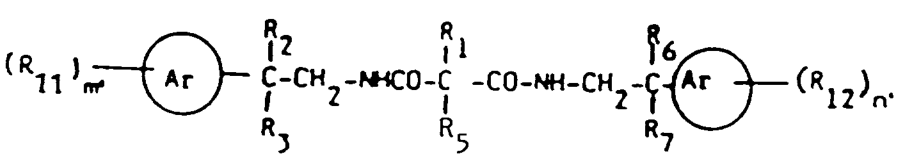

IIIa

in der $R_5$ Wasserstoff ist, $R_1$, $R_2$, $R_3$, $R_6$, $R_7$, $R_{11}$ und $R_{12}$ wie oben definiert sind, Ar Phenyl oder Thienyl bedeutet und m' und n' unabhängig voneinander 0, 1, 2 oder 3 bedeuten, wenn das zugehörige Ar Phenyl ist, und O, 1 oder 2 bedeuten, wenn das zugehörige Ar Thienyl ist;

in Gegenwart eines Kondensationsmittels kondensiert ohne das Zwischenprodukt der allgemeinen Formel (II) zu isolieren:

C. (zur Herstellung einer Verbindung der Formel I, worin D die Gruppe der Formel Ib ist) eine Verbindung III

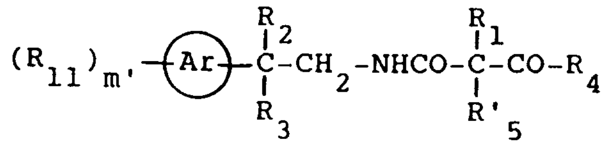

III

in der $R'_5$ Wasserstoff ist, $R_1$, $R_2$, $R_3$ und $R_{11}$ wie oben definiert sind, Ar Phenyl oder Thienyl bedeutet und m' 0, 1, 2 oder 3 bedeutet, wenn Ar Phenyl ist, und 0, 1 oder 2 bedeutet, wenn Ar Thienyl ist;
in Gegenwart eines Kondensationsmittels kondensiert;
und daß man gegebenenfalls eine oder mehrere der folgenden Nachbehandlungen anschließt:
Alkylieren einer Verbindung (I), in der $R_5$ beziehungsweise $R'_5$ Wasserstoff ist, zu einer Verbindung (I) in der $R_5$ beziehungsweise $R'_5$ $(C_1\text{-}C_4)$Alkyl ist;
Hydroxymethylieren einer Verbindung (I), in der D die Gruppe der Formel Ia ist und $R_5$ Wasserstoff ist, zu einer Verbindung, in der $R_5$ Hydroxymethylen ist;
Isolieren der einzelnen von der Formel I umfaßten Tautomeren;
Isolieren der freien Verbindung (I) aus ihrem Salz;
Umsetzen einer Verbindung (I) zu ihrem pharmazeutisch annehmbaren Salz.

**2.** Verfahren nach Anspruch 1, dadurch gekennzeichnet, daß man eine Verbindung herstellt worin D die Gruppe der Formel Ia bedeutet; $R_1$ Wasserstoff, $(C_1\text{-}C_{10})$Alkyl, Phenyl$(C_1\text{-}C_5)$alkyl oder -NHCOX (worin X $(C_1\text{-}C_5)$Alkyl ist) bedeutet;
$R_{11}$ und $R_{12}$ unabhängig voneinander $(C_1\text{-}C_4)$Alkyl, Hydroxy, $(C_1\text{-}C_4)$Alkoxy, Methansulfonyloxy oder Methansulfonamido bedeuten, oder zwei benachbarte Substituenten $R_{11}$ beziehungsweise $R_{12}$ zusammen $-O\text{-}CH_2\text{-}O-$ oder $-O\text{-}CH_2\text{-}CH_2\text{-}O-$ sind;
$R_2$, $R_3$, $R_6$ und $R_7$ unabhängig voneinander Wasserstoff bedeuten oder $R_2$ mit $R_3$ und/oder $R_6$ mit $R_7$ und dem jeweiligen Kohlenstoffatom, an das sie gebunden sind, einen 5- oder 6-gliedrigen Carbocyclus bedeuten;
$R_{11}$ und $R_{12}$ unabhängig voneinander Hydroxy, $(C_1\text{-}C_4)$Alkoxy, Methansulfonyloxy oder Methansulfonamido bedeuten, oder zwei benachbarte Substituenten $R_{11}$ beziehungsweise $R_{12}$ zusammen $-O\text{-}CH_2\text{-}O-$ oder $-O\text{-}CH_2\text{-}CH_2\text{-}O-$ sind.

**3.** Verfahren nach Anspruch 2, dadurch gekennzeichnet, daß man eine Verbindung herstellt worin $R_1$ Wasserstoff, $(C_1\text{-}C_{10})$Alkyl oder -NHCOX (worin X $(C_1\text{-}C_5)$Alkyl ist) bedeutet.

**4.** Verfahren nach Anspruch 2, dadurch gekennzeichnet, daß man eine Verbindung herstellt worin $R_1$ Wasserstoff, $(C_1\text{-}C_6)$Alkyl, oder $-NHCOCH_3$ bedeutet und/oder
$R_5$ Wasserstoff, Methyl oder Hydroxymethyl bedeutet und/oder
$R_2$, $R_3$, $R_6$ und $R_7$ unabhängig voneinander Wasserstoff bedeuten oder $R_2$ mit $R_3$ und/oder $R_6$ mit $R_7$ und dem jeweiligen Kohlenstoffatom, an das sie gebunden sind, einen 5-gliedrigen Carbocyclus bedeuten und/oder
$R_{11}$ und $R_{12}$ unabhängig voneinander Hydroxy, Methoxy, Methansulfonoxy oder Methansulfonamido bedeuten, oder zwei benachbarte Substituenten $R_{11}$ beziehungsweise $R_{12}$ zusammen $-O\text{-}CH_2\text{-}O-$ sind.

**5.** Verfahren nach einem der Ansprüche 1 bis 4, dadurch gekennzeichnet, daß man eine Verbindung herstellt, worin D die Gruppe der Formel Ia bedeutet und m und/oder n 2 bedeuten.

**6.** Verfahren nach Anspruch 5, worin D die Gruppe der Formel Ia bedeutet und, wenn A und/oder B ein Benzorest ist, die beiden Substituenten $R_{11}$ und/oder $R_{12}$ im Benzorest in meta-beziehungsweise para-Position zu den Fusionspunkten des Restes A beziehungsweise B stehen und worin vorzugsweise $R_{11}$ und $R_{12}$ Methoxy sind.

7. Verfahren nach Anspruch 1, dadurch gekennzeichnet, daß man eine Verbindung der Formel

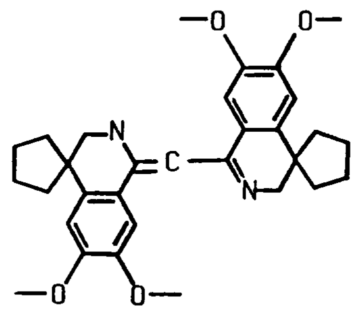

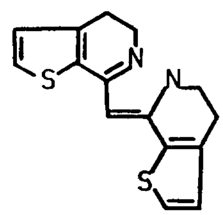

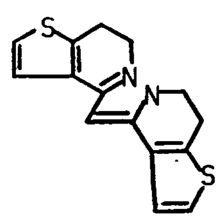

oder

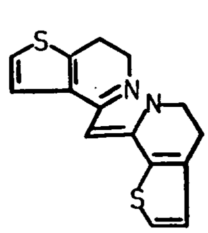

oder ihr physiologisch unbedenkliches Salz herstellt.

8. Verfahren nach Anspruch 1, dadurch gekennzeichnet, daß man eine Verbindung herstellt, worin D die Gruppe der Formel Ib bedeutet und $R_4$ eine $-NR_9R_{10}$-Gruppe bedeutet, worin $R_9$ und $R_{10}$ unabhängig voneinander (a) Wasserstoff, (b) Alkyl mit 1 bis 8 Kohlenstoffatomen, Alkenyl oder Alkinyl mit 2 oder 3 Kohlenstoffatomen (wobei das Alkyl substituiert sein kann durch Hydroxy, $(C_1-C_4)$Alkoxy, Di$(C_1-C_4)$Alkylamino, Furyl, Pyrrolidine, Morpholinyl, Pyridinyl oder die Gruppe

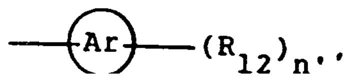

worin Ar, $R_{12}$ und n' wie in Anspruch 1 definiert sind), (d) Dimethylamino, (f) Phenyl, (g) Morpholinyl, (h) Pyrridinyl bedeuten, wobei $R_9$ und $R_{10}$ nicht gleichzeitig Wasserstoff, Dimethylamino oder Di($C_1$-$C_4$)alkylaminomethyl sein können;
oder $R_9$ und $R_{10}$ gemeinsam mit dem Stickstoffatom, an das sie gebunden sind, einen Pyrrolidinyl-, Morpholinyl- oder Piperazinylrest bedeuten, wobei der Piperazinylring gegebenenfalls durch unsubstituiertes Phenyl, Mono- oder Di($C_1$-$C_4$)alkoxyphenyl, Pyrimidinyl oder Phenyl ($C_1$-$C_4$)alkyl N-substituiert sein kann.

**9.** Verfahren nach Anspruch 8, dadurch gekennzeichnet, daß man eine Verbindung herstellt, worin $R_4$ eine -$NR_9R_{10}$-Gruppe ist, worin $R_9$ Wasserstoff ist und $R_{10}$ ein substituiertes Alkyl der Formel VII ist,

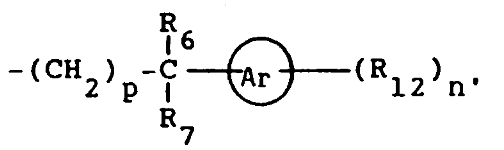

VII

worin p O, 1 oder 2 ist;
$R_6$ und $R_7$ unabhängig voneinander Wasserstoff oder ($C_1$-$C_5$)Alkyl oder gemeinsam mit dem Kohlenstoffatom, an das sie gebunden sind, einen 5- oder 6-gliedrigen Carbocyclus bedeuten;
Ar, Phenyl oder Thienyl bedeutet;
$R_{12}$ ($C_1$-$C_4$)Alkyl, Halogen (F, Cl, Br, J), Hydroxy, ($C_1$-$C_4$)Alkoxy, Amino, Thiomethyl, Methansulfonyloxy oder Methansulfonamido bedeutet, oder zwei benachbarte Substituenten $R_{12}$ zusammen -O-$CH_2$-O- oder -O-$CH_2$-$CH_2$-O- sind und
n' 0, 1, 2 oder 3 bedeutet, wenn Ar Phenyl ist, und O 1 oder 2 bedeutet, wenn Ar Thienyl ist.

**10.** Verfahren nach einem der Ansprüche 1, 8 und 9, dadurch gekennzeichnet, daß man eine Verbindung herstellt, worin D die Gruppe der Formel Ib bedeutet, $R_1$ Wasserstoff ($C_1$-$C_6$)Alkyl oder -$NHCOCH_3$ bedeutet;
$R_{11}$ Hydroxy, ($C_1$-$C_4$)Alkoxy, Methansulfonyloxy oder Methansulfonamido bedeutet, oder zwei benachbarte Substituenten $R_{11}$ zusammen -O-$CH_2$-O- oder -O-$CH_2$-$CH_2$-O- sind; und
$R_2$ und $R_3$ Wasserstoff bedeuten oder zusammen mit dem Kohlenstoffatom, an das sie gebunden sind, einen 5- oder 6-gliedrigen Carbocyclus bedeuten;
und $R'_5$ Wasserstoff, Methyl oder Ethyl bedeutet.

**11.** Verfahren nach einem der Ansprüche 1 und 8 bis 10, dadurch gekennzeichnet, daß man eine Verbindung herstellt, worin A ein Benzorest ist, m 2 bedeutet und die beiden Substituenten $R_{11}$ in meta- beziehungsweise para-Position zu den Fusionspunkten des Restes A stehen, worin $R_{11}$ vorzugsweise Methoxy ist.

**12.** Verfahren nach Anspruch 1, dadurch gekennzeichnet, daß man eine Verbindung herstellt, worin D die Gruppe der Formel Ib ist, A Benzo, $R_{11}$ Methoxy, m zwei, $R_1$ Wasserstoff oder ($C_1$-$C_5$)alkyl, $R_2$, $R_3$ und $R'_5$ Wasserstoff und $R_4$ Morpholino, Methylamino, Diethylamino oder Phenethylamino bedeuten.

**13.** Verfahren nach Anspruch 1, dadurch gekennzeichnet, daß man Morpholinocarbonylmethyl-6,7-dimethoxy-3,4-dihydro-isochinolin,
6,7-Dimethoxy-3,4-dihydro-isochinolinessigsäuremethylamid,
6,7-Dimethoxy-3,4-dihydro-isochinolinessigsäurediethylamid oder
6,7-Dimethoxy-3,4-dihydro-isochinolinessigsäurephenylethylamid herstellt.

**14.** Verfahren zur Herstellung einer pharmazeutischen Zubereitung, dadurch gekennzeichnet, daß man eine Verbindung, die wie in einem der Ansprüche 1 bis 13 definiert ist mit einem oder mehreren pharmazeutisch annehmbaren Trägerstoffen mischt.

## Claims

**Claims for the following Contracting States : AT, BE, CH, DE, FR, GB, IT, LI, LU, NL, SE**

**1.** Compound of general formula I

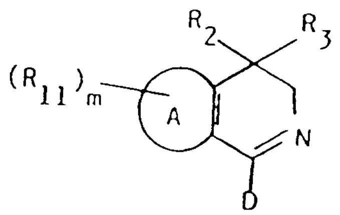

I

wherein
A denotes a benzo- or thieno group;
$R_2$ and $R_3$ independently of each other represent hydrogen or $(C_{1-5})$alkyl or together with the carbon atom to which they are bound they represent a 5- or 6-membered carbocyclic ring;
$R_{11}$ denotes $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{11}$ together represent $-O-CH_2-O-$ or $-O-CH_2-CH_2-O-$;
m denotes 0, 1, 2 or 3 if A is a benso group and 0, 1 or 2 if A is a thieno group;
and D denotes a group of formula Ia or Ib

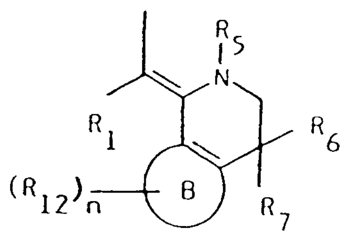

Ia

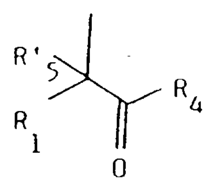

Ib

wherein, in the group of formula Ia
B denotes a benzo- or thieno group;
$R_1$ denotes hydrogen, $(C_{1-10})$alkyl, phenyl, phenyl$(C_{1-5})$alkyl, $(C_{1-4})$alkoxy or -NHCOX (wherein X is $(C_{1-5})$alkyl);
$R_5$ denotes hydrogen, $(C_{1-4})$alkyl or hydroxymethyl;
$R_6$ and $R_7$ independently of each other denote hydrogen or $(C_{1-5})$alkyl or together with the carbon atom to which they are bound they represent a 5- or 6-membered carbocyclic group;
$R_{12}$ denotes $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{12}$ together represent $-O-CH_2-O-$ or $-O-CH_2-CH_2-O-$;
and n denotes 0, 1, 2 or 3 if B is a benzo group and 0, 1 or 2 if B is a thieno group;
and in the group of formula Ib
$R_1$ denotes hydrogen, $(C_{1-10})$alkyl, phenyl$(C_{1-5})$alkyl, $(C_{1-4})$alkoxy or -NHCOX (wherein X is $(C_{1-5})$-alkyl);
$R'_5$ denotes hydrogen or $(C_{1-4})$alkyl;
$R_4$ denotes an $-NR_9R_{10}$ group wherein
$R_9$ and $R_{10}$ independently of each other represent (a) hydrogen, (b) branched or unbranched alkyl, alkenyl or alkynyl with 1-12 carbon atoms (wherein the alkyl may be substituted by hydroxy, $(C_{1-4})$-alkoxy, di$(C_{1-4})$alkylamino, furyl, pyrrolidinyl, morpholinyl, pyridinyl, indolyl, or the group

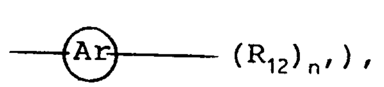

(c) cycloalkyl with 3-7 ring members, (d) dimethylamino, (e) amino($C_{2-4}$)alkyl (wherein the amino group may be unsubstituted or is mono- or di($C_{1-4}$)alkylamino), (f) phenyl, (g) morpholinyl, or (h) pyrridinyl, whilst $R_9$ and $R_{10}$ cannot simultaneously represent hydrogen, dimethylamino or di($C_{1-4}$)-alkylaminomethyl;
or $R_9$ and $R_{10}$ together with the nitrogen atom to which they are bound represent a pyrrolidinyl, piperidinyl, morpholinyl or piperazinyl group, whilst the piperazinyl ring may optionally be N-substituted by unsubstituted phenyl, mono- or di-($C_{1-4}$)alkoxyphenyl, pyrimidinyl or phenyl($C_{1-4}$)alkyl;
$R_{12}$ represents ($C_{1-4}$)alkyl, halogen (F, Cl, Br, I), hydroxy, ($C_{1-4}$)alkoxy, amino, thiomethyl, methanesulphonyloxy or methanesulphonamido, or two adjacent substituents $R_{12}$ together represent $-O-CH_2-O-$ or $-O-CH_2-CH_2-O-$;
Ar is phenyl or thienyl;
n' represents 0, 1, 2 or 3 if Ar is phenyl and 0, 1 or 2, if Ar is thienyl;
and the physiologically acceptable salts thereof with inorganic and organic acids; with the exception of the compound of formula I wherein D is the group Ia and $R_1$, $R_2$, $R_3$, $R_5$, $R_6$ and $R_7$ represent hydrogen and A and B represent the group

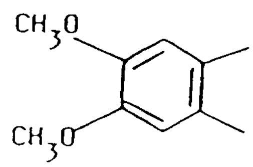

and with the exception of the compound of formula I wherein D is the group of formula Ib and $R_1$, $R_2$, $R_3$ and $R'_5$ denote hydrogen, A represents the group

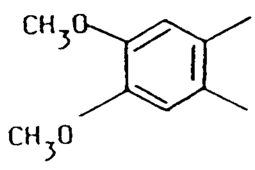

and $R_4$ represents the group

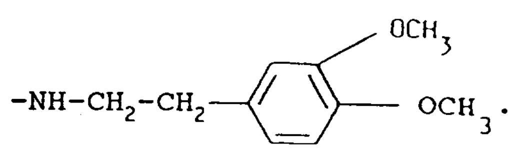

**2.** Compound according to claim 1, wherein D denotes the group of formula Ia; $R_1$ represents hydrogen, ($C_{1-10}$)alkyl, phenyl($C_{1-5}$)alkyl or -NHCOX (wherein X is ($C_{1-5}$)alkyl);
$R_{11}$ and $R_{12}$ independently of each other represent ($C_{1-4}$)alkyl, hydroxy, ($C_{1-4}$)alkoxy, methanesulphonyloxy or methanesulphonamido, or two adjacent substituents $R_{11}$ and $R_{12}$ together represent $-O-CH_2-O-$ or $-O-CH_2-CH_2-O-$;
$R_2$, $R_3$, $R_6$ and $R_7$ independently of each other represent hydrogen or $R_2$ together with $R_3$ and/or $R_6$ together with $R_7$ and the particular carbon atom to which they are bound represent a 5- or 6-membered carbocyclic group;
$R_{11}$ and $R_{12}$ independently of each other represent hydroxy, ($C_{1-4}$)alkoxy, methanesulphonyloxy or methanesulphonamido, or two adjacent substituents $R_{11}$ and $R_{12}$ together represent $-O-CH_2-O-$ or $-O-CH_2-CH_2-O-$.

**3.** Compound according to claim 2, wherein $R_1$ represents hydrogen, ($C_{1-10}$)alkyl or -NHCOX (wherein X is ($C_{1-5}$)alkyl).

**4.** Compound according to claim 2, wherein $R_1$ represents hydrogen, $(C_{1-6})$alkyl or $-NHCOCH_3$ and/or $R_5$ represents hydrogen, methyl or hydroxymethyl and/or
$R_2$, $R_3$, $R_6$ and $R_7$ independently of each other represent hydrogen or $R_2$ with $R_3$ and/or $R_6$ with $R_7$ and the particular carbon atom to which they are bound represent a 5-membered carbocyclic group, and/or
$R_{11}$ and $R_{12}$ independently of each other represent hydroxy, methoxy, methanesulphonoxy or methanesulphonamido, or two adjacent substituents $R_{11}$ and $R_{12}$ together represent $-O-CH_2-O-$.

**5.** Compound according to one of claims 1 to 4, wherein D represents the group of formula Ia and m and/or n represents 2.

**6.** Compound according to claim 5, wherein D represents the group of formula Ia and, if A and/or B is a benzo group, the two substituents $R_{11}$ and/or $R_{12}$ in the benzo group are in the meta- and para-positions respectively, to the fusion points of group A or B respectively and wherein preferably $R_{11}$ and $R_{12}$ represent methoxy.

**7.** Compound according to claim 1, namely

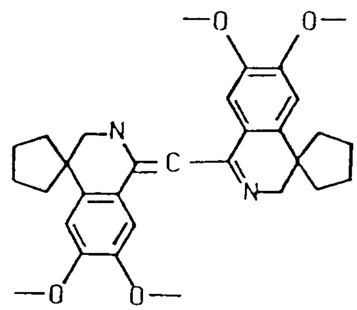

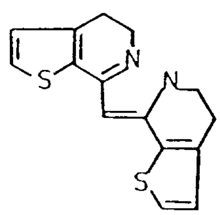

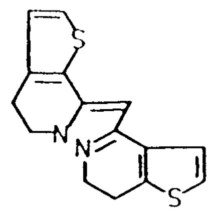

or

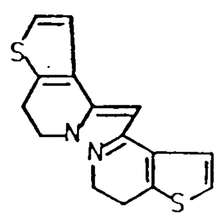

or a physiologically acceptable salt thereof.

**8.** Compound according to claim 1, wherein D represents the group of formula Ib and $R_4$ represents an $-NR_9R_{10}$-group, wherein
$R_9$ and $R_{10}$ independently of each other represent (a) hydrogen, (b) alkyl with 1 to 8 carbon atoms, alkenyl or alkynyl with 2 or 3 carbon atoms (whilst the alkyl may be substituted by hydroxy, $(C_{1-4})$-alkoxy, di$(C_{1-4})$alkylamino, furyl, pyrrolidinyl, morpholinyl, pyridinyl or the group

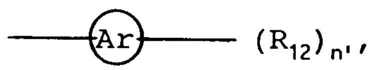

wherein Ar, $R_{12}$ and n' are defined as in claim 1), (d) dimethylamino, (f) phenyl, (g) morpholinyl, (h) pyridinyl, whilst $R_9$ and $R_{10}$ cannot simultaneously represent hydrogen, dimethylamino or di$(C_{1-4})$-alkylaminomethyl;
or $R_9$ and $R_{10}$ together with the nitrogen atom to which they are bound represent a pyrrolidinyl, morpholinyl or piperazinyl group, whilst the piperazinyl ring may optionally be N-substituted by unsubstituted phenyl, mono- or di$(C_{1-4})$alkoxyphenyl, pyrimidinyl or phenyl$(C_{1-4})$alkyl.

**9.** Compound as claimed in claim 8, wherein $R_4$ is an $-NR_9R_{10}$ group wherein $R_9$ is hydrogen and $R_{10}$ is a substituted alkyl of formula VII

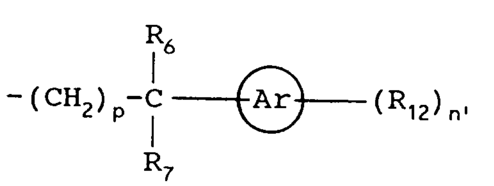

VII

wherein p is 0, 1 or 2;
$R_6$ and $R_7$ independently of each other represent hydrogen or $(C_{1-5})$alkyl or together with the carbon atom to which they are bound they represent a 5- or 6-membered carbocyclic ring;
Ar represents phenyl or thienyl;
$R_{12}$ represents $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{12}$ together represent

$-O-CH_2-O-$ or $-O-CH_2-CH_2-O-$ and
n' represents 0, 1, 2 or 3, if Ar is phenyl, and 0, 1 or 2, if Ar is thienyl.

**10.** Compound according to one of claims 1, 8 and 9, wherein D represents the group of formula Ib, $R_1$ represents hydrogen, $(C_{1-6})$alkyl or $-NHCOCH_3$;
$R_{11}$ represents hydroxy, $(C_{1-4})$alkoxy, methanesulphonyloxy or methanesulphonamido, or two adjacent substituents $R_{11}$ together represent $-O-CH_2-O-$ or $-O-CH_2-CH_2-O-$; and
$R_2$ and $R_3$ represent hydrogen or together with the carbon atom to which they are bound they represent a 5- or 6-membered carbocyclic group;
and $R'_5$ represents hydrogen, methyl or ethyl.

**11.** Compound according to one of claims 1 and 8 to 10 wherein A is a benzo group, m represents 2 and the two substituents $R_{11}$ are in the meta- and para-positions, respectively, to the fusion points of group A, wherein $R_{11}$ is preferably methoxy.

**12.** Compound according to claim 1, wherein D is the group of formula Ib, A represents benzo, $R_{11}$ represents methoxy, m represents two, $R_1$ represents hydrogen or $(C_{1-5})$alkyl, $R_2$, $R_3$ and $R'_5$ represent hydrogen and $R_4$ represents morpholino, methylamino, diethylamino or phenethylamino.

**13.** Compound according to claim 1, namely morpholinocarbonylmethyl-6,7-dimethoxy-3,4-dihydro-isoquinoline,
6,7-dimethoxy-3,4-dihydro-isoquinoline acetic acid methylamide,
6,7-dimethoxy-3,4-dihydro-isoquinoline acetic acid diethylamide or
6,7-dimethoxy-3,4-dihydro-isoquinoline acetic acid phenylethylamide.

**14.** Process for preparing a compound according to one of claims 1 to 13, characterised in that
A. (in order to prepare a compound of formula I wherein D is the group of formula Ia) a compound

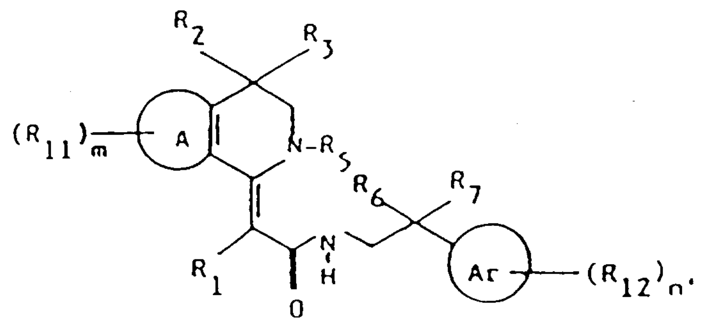

II

wherein A, $R_1$, $R_2$, $R_3$, $R_6$, $R_7$, $R_{11}$, $R_{12}$ and m are defined as in one of claims 1 to 7, Ar represents phenyl or thienyl and n' represents 0, 1, 2 or 3 if Ar is phenyl and 0, 1 or 2 if Ar is thienyl, and $R_5$ represents hydrogen or $(C_{1-4})$alkyl,
is condensed in the presence of a condensing agent;
B. (in order to prepare a compound of formula I wherein D is the group of formula Ia) a compound (IIIa)

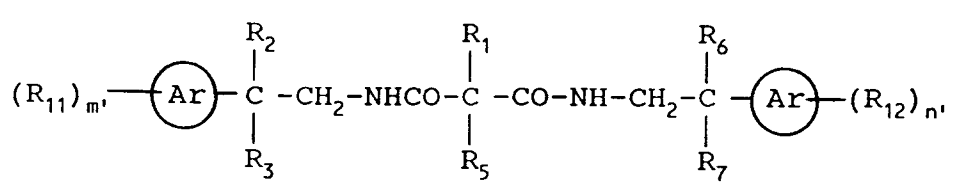

IIIa

wherein $R_5$ is hydrogen, $R_1$, $R_2$, $R_3$, $R_6$, $R_7$, $R_{11}$ and $R_{12}$ are defined as in one of claims 1 to 7, Ar represents phenyl or thienyl and m' and n' independently of each other represent 0, 1, 2 or 3 if the

associated Ar is phenyl and 0, 1 or 2, if the associated Ar is thienyl;
is condensed in the presence of a condensing agent without isolating the intermediate product of general formula (II);
C. (in order to prepare a compound of formula I wherein D is the group of formula Ib) a compound III

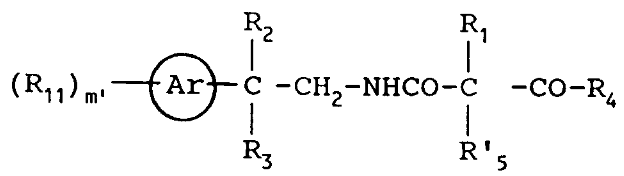

III

wherein $R'_5$ is hydrogen, $R_1$, $R_2$, $R_3$ and $R_{11}$ are defined as in one of claims 1 and 8 to 13, Ar denotes phenyl or thienyl and m' denotes 0, 1, 2 or 3 if Ar is phenyl and 0, 1 or 2 if Ar is thienyl;
is condensed in the presence of a condensing agent;
and optionally one or more of the following after-treatments is added:
alkylation of a compound (I) wherein $R_5$ or $R'_5$ is hydrogen, to yield a compound (I) wherein $R_5$ or $R'_5$ is $(C_{1-4})$alkyl;
hydroxymethylation of a compound (I) wherein D is the group of formula Ia and $R_5$ is hydrogen, to yield a compound wherein $R_5$ is hydroxymethylene;
isolation of the individual tautomers included in formula I;
isolation of the free compound (I) from its salt;
reaction of a compound (I) to obtain the pharmaceutically acceptable salt thereof.

**15.** Pharmaceutical preparation containing as active substance one or more compounds of general formula I

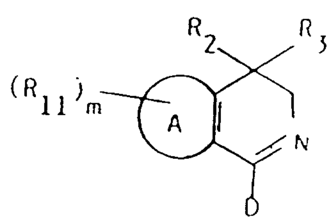

I

wherein
A denotes a benzo- or thieno group;
$R_2$ and $R_3$ independently of each other represent hydrogen or $(C_{1-5})$alkyl or together with the carbon atom to which they are bound they represent a 5- or 6-membered carbocyclic ring;
$R_{11}$ denotes $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{11}$ together represent $-O-CH_2-O-$ or $-O-CH_2-CH_2-O-$;
m denotes 0, 1, 2 or 3 if A is a benzo group and 0, 1 or 2 if A is a thieno group;
and D denotes a group of formula Ia or Ib

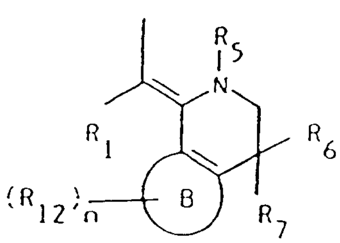

Ia

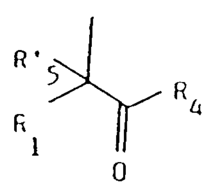

Ib

wherein, in the group of formula Ia

B denotes a benzo- or thieno group;
$R_1$ denotes hydrogen, $(C_{1-10})$alkyl, phenyl, phenyl$(C_{1-5})$alkyl, $(C_{1-4})$alkoxy or -NHCOX (wherein X is $(C_{1-5})$alkyl);
$R_5$ denotes hydrogen, $(C_{1-4})$alkyl or hydroxymethyl;
$R_6$ and $R_7$ independently of each other denote hydrogen or $(C_{1-5})$alkyl or together with the carbon atom to which they are bound they represent a 5- or 6-membered carbocyclic group;
$R_{12}$ denotes $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{12}$ together represent $-O-CH_2-O-$ or $-O-CH_2-CH_2-O-$;
and n denotes 0, 1, 2 or 3 if B is a benzo group and 0, 1 or 2 if B is a thieno group;
and in the group of formula Ib
$R_1$ denotes hydrogen, $(C_{1-10})$alkyl, phenyl$(C_{1-5})$alkyl, $(C_{1-4})$alkoxy or -NHCOX (wherein X is $(C_{1-5})$-alkyl);
$R'_5$ denotes hydrogen or $(C_{1-4})$alkyl;
$R_4$ denotes an $-NR_9R_{10}$ group wherein
$R_9$ and $R_{10}$ independently of each other represent (a) hydrogen, (b) branched or unbranched alkyl, alkenyl or alkynyl with 1-12 carbon atoms (wherein the alkyl may be substituted by hydroxy, $(C_{1-4})$-alkoxy, di$(C_{1-4})$alkylamino, furyl, pyrrolidinyl, morpholinyl, pyridinyl, indolyl, or the group

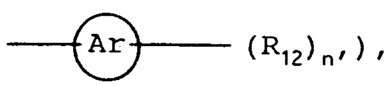

(c) cycloalkyl with 3-7 ring members, (d) dimethylamino, (e) amino$(C_{2-4})$alkyl (wherein the amino group may be unsubstituted or is mono- or di$(C_{1-4})$alkylamino), (f) phenyl, (g) morpholinyl, or (h) pyrridinyl, whilst $R_9$ and $R_{10}$ cannot simultaneously represent hydrogen, dimethylamino or di$(C_{1-4})$-alkylaminomethyl;
or $R_9$ and $R_{10}$ together with the nitrogen atom to which they are bound represent a pyrrolidinyl, piperidinyl, morpholinyl or piperazinyl group, whilst the piperazinyl ring may optionally be N-substituted by unsubstituted phenyl, mono- or di-$(C_{1-4})$alkoxyphenyl, pyrimidinyl or phenyl$(C_{1-4})$alkyl;
$R_{12}$ represents $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{12}$ together represent $-O-CH_2-O-$ or $-O-CH_2-CH_2-O-$;
Ar is phenyl or thienyl;
n' represents 0, 1, 2 or 3 if Ar is phenyl and 0, 1 or 2, if Ar is thienyl or the pharmaceutically acceptable salts thereof.

**16.** Pharmaceutical preparation according to claim 15, containing a compound according to one of claims 2 to 13.

**17.** Use of a compound of general formula I defined as in one of claims 1 to 13 and 15 or the pharmaceutically acceptable salts thereof, for preparing a drug for treating coronary heart disease, acute myocardial infarction, or for cardio- and/or cerebroprotection.

**Claims for the following Contracting States : ES, GR**

**1.** Process for preparing a compound of general formula I

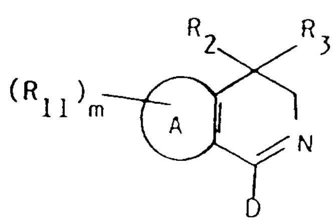

I

wherein
A denotes a benzo- or thieno group;
$R_2$ and $R_3$ independently of each other represent hydrogen or $(C_{1-5})$alkyl or together with the carbon atom to which they are bound they represent a 5- or 6-membered carbocyclic ring;
$R_{11}$ denotes $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{11}$ together represent $-O-CH_2-O-$ or $-O-CH_2-CH_2-O-$;
m denotes 0, 1, 2 or 3 if A is a benzo group and 0, 1 or 2 if A is a thieno group;
and D denotes a group of formula Ia or Ib

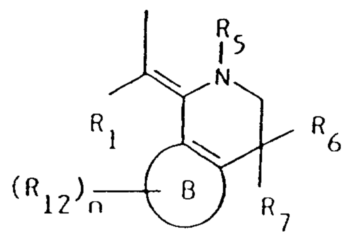 Ia 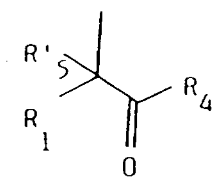 Ib

wherein, in the group of formula Ia
B denotes a benzo- or thieno group;
$R_1$ denotes hydrogen, $(C_{1-10})$alkyl, phenyl, phenyl$(C_{1-5})$alkyl, $(C_{1-4})$alkoxy or -NHCOX (wherein X is $(C_{1-5})$alkyl);
$R_5$ denotes hydrogen, $(C_{1-4})$alkyl or hydroxymethyl;
$R_6$ and $R_7$ independently of each other denote hydrogen or $(C_{1-5})$alkyl or together with the carbon atom to which they are bound they represent a 5- or 6-membered carbocyclic group;
$R_{12}$ denotes $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{12}$ together represent $-O-CH_2-O-$ or $-O-CH_2-CH_2-O-$;
and n denotes 0, 1, 2 or 3 if B is a benzo group and 0, 1 or 2 if B is a thieno group;
and in the group of formula Ib
$R_1$ denotes hydrogen, $(C_{1-10})$alkyl, phenyl$(C_{1-5})$alkyl, $(C_{1-4})$alkoxy or -NHCOX (wherein X is $(C_{1-5})$-alkyl);
$R'_5$ denotes hydrogen or $(C_{1-4})$alkyl;
$R_4$ denotes an $-NR_9R_{10}$ group wherein
$R_9$ and $R_{10}$ independently of each other represent (a) hydrogen, (b) branched or unbranched alkyl, alkenyl or alkynyl with 1-12 carbon atoms (wherein the alkyl may be substituted by hydroxy, $(C_{1-4})$-alkoxy, di$(C_{1-4})$alkylamino, furyl, pyrrolidinyl, morpholinyl, pyridinyl, indolyl, or the group

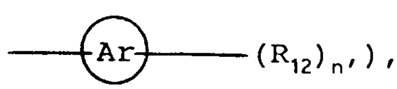

(c) cycloalkyl with 3-7 ring members, (d) dimethylamino, (e) amino$(C_{2-4})$alkyl (wherein the amino group may be unsubstituted or is mono- or di$(C_{1-4})$alkylamino), (f) phenyl, (g) morpholinyl, or (h) pyrridinyl, whilst $R_9$ and $R_{10}$ cannot simultaneously represent hydrogen, dimethylamino or di$(C_{1-4})$-alkylaminomethyl;
or $R_9$ and $R_{10}$ together with the nitrogen atom to which they are bound represent a pyrrolidinyl, piperidinyl, morpholinyl or piperazinyl group, whilst the piperazinyl ring may optionally be N-substituted by unsubstituted phenyl, mono- or di-$(C_{1-4})$alkoxyphenyl, pyrimidinyl or phenyl$(C_{1-4})$alkyl;
$R_{12}$ represents $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{12}$ together represent $-O-CH_2-O-$ or $-O-CH_2-CH_2-O-$;
Ar is phenyl or thienyl;
n' represents 0, 1, 2 or 3 if Ar is phenyl and 0, 1 or 2, if Ar is thienyl;
and the physiologically acceptable salts thereof with inorganic and organic acids; with the exception of

the compound of formula I wherein D is the group Ia and $R_1$, $R_2$, $R_3$, $R_5$, $R_6$ and $R_7$ represent hydrogen and A and B represent the group

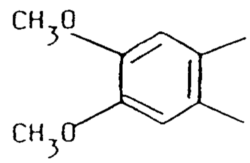

and with the exception of the compound of formula I wherein D is the group of formula Ib and $R_1$, $R_2$, $R_3$ and $R'_5$ denote hydrogen, A represents the group

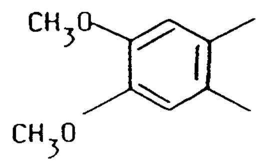

and $R_4$ represents the group

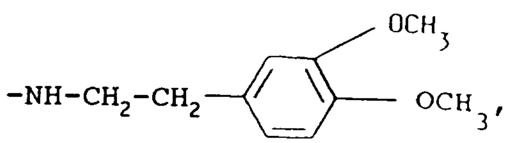

characterised in that

A. (in order to prepare a compound of formula I wherein D is the group of formula Ia) a compound (II),

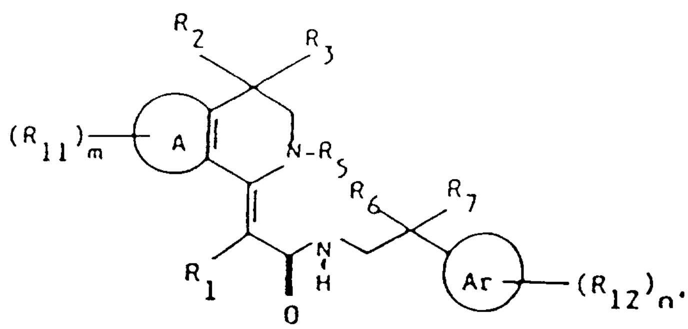

II

wherein A, $R_1$, $R_2$, $R_3$, $R_6$, $R_7$, $R_{11}$, $R_{12}$ and m are defined as hereinbefore, Ar represents phenyl or thienyl and n' represents 0, 1, 2 or 3 if Ar is phenyl and 0, 1 or 2 if Ar is thienyl, and $R_5$ represents hydrogen or $(C_{1-4})$alkyl,

is condensed in the presence of a condensing agent;

B. (in order to prepare a compound of formula I wherein D is the group of formula Ia) a compound (IIIa)

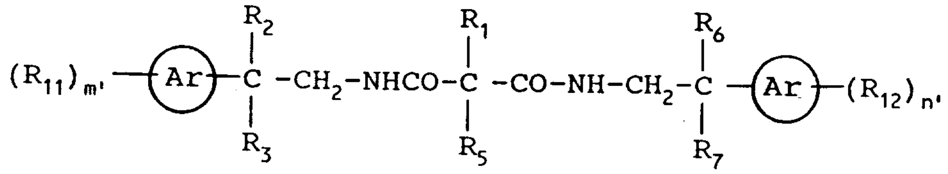 IIIa

wherein $R_5$ is hydrogen, $R_1$, $R_2$, $R_3$, $R_6$, $R_7$, $R_{11}$ and $R_{12}$ are defined as hereinbefore, Ar represents phenyl or thienyl and m' and n' independently of each other represent 0, 1, 2 or 3 if the associated Ar is phenyl and 0, 1 or 2, if the associated Ar is thienyl;
is condensed in the presence of a condensing agent without isolating the intermediate product of general formula (II);
C. (in order to prepare a compound of formula I wherein D is the group of formula Ib) a compound III

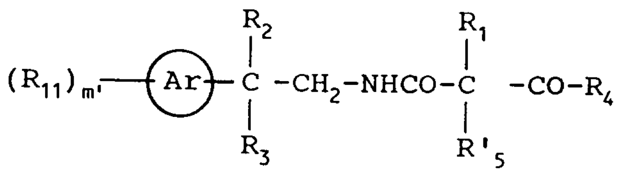 III

wherein $R'_5$ is hydrogen, $R_1$, $R_2$, $R_3$ and $R_{11}$ are defined as hereinbefore, Ar denotes phenyl or thienyl and m' denotes 0, 1, 2 or 3 if Ar is phenyl and 0, 1 or 2 if Ar is thienyl;
is condensed in the presence of a condensing agent;
and optionally one or more of the following after-treatments is added:
alkylation of a compound (I) wherein $R_5$ or $R'_5$ is hydrogen, to yield a compound (I) wherein $R_5$ or $R'_5$ is $(C_{1-4})$alkyl;
hydroxymethylation of a compound (I) wherein D is the group of formula Ia and $R_5$ is hydrogen, to yield a compound wherein $R_5$ is hydroxymethylene;
isolation of the individual tautomers included in formula I;
isolation of the free compound (I) from its salt;
reaction of a compound (I) to obtain the pharmaceutically acceptable salt thereof.

2. Process according to claim 1, characterised in that a compound is prepared wherein D represents the group of formula Ia; $R_1$ represents hydrogen, $(C_{1-10})$alkyl, phenyl$(C_{1-5})$alkyl or -NHCOX (wherein X is $(C_{1-5})$alkyl);
$R_{11}$ and $R_{12}$ independently of each other represent $(C_{1-4})$alkyl, hydroxy, $(C_{1-4})$alkoxy, methanesulphonyloxy or methanesulphonamido, or two adjacent substituents $R_{11}$ and $R_{12}$ together represent $-O-CH_2-O-$ or $-O-CH_2-CH_2-O-$;
$R_2$, $R_3$, $R_6$ and $R_7$ independently of each other represent hydrogen or $R_2$ together with $R_3$ and/or $R_6$ together with $R_7$ and the particular carbon atom to which they are bound represent a 5- or 6-membered carbocyclic group;
$R_{11}$ and $R_{12}$ independently of each other represent hydroxy, $(C_{1-4})$alkoxy, methanesulphonyloxy or methanesulphonamido, or two adjacent substituents $R_{11}$ and $R_{12}$ together represent $-O-CH_2-O-$ or $-O-CH_2-CH_2-O-$.

3. Process according to claim 2, characterized in that a compound is prepared wherein $R_1$ denotes hydrogen, $(C_{1-10})$alkyl or -NHCOX (wherein X is $(C_{1-5})$alkyl).

4. Process according to claim 2, characterised in that a compound is prepared wherein $R_1$ represents hydrogen, $(C_{1-6})$alkyl or $-NHCOCH_3$ and/or
$R_5$ represents hydrogen, methyl or hydroxymethyl and/or
$R_2$, $R_3$, $R_6$ and $R_7$ independently of each other represent hydrogen or $R_2$ with $R_3$ and/or $R_6$ with $R_7$ and the particular carbon atom to which they are bound represent a 5-membered carbocyclic group, and/or
$R_{11}$ and $R_{12}$ independently of each other represent hydroxy, methoxy, methanesulphonoxy or

methanesulphonamido, or two adjacent substituents $R_{11}$ and $R_{12}$ together represent -O-$CH_2$-O-.

**5.** Process according to one of claims 1 to 4, characterised in that a compound is prepared wherein D denotes the group of formula Ia and m and/or n represents 2.

**6.** Process according to claim 5, wherein D represents the group of formula Ia and, if A and/or B is a benzo group, the two substituents $R_{11}$ and/or $R_{12}$ in the benzo group are in the meta- and para-positions respectively, to the fusion points of group A or B respectively and wherein preferably $R_{11}$ and $R_{12}$ represent methoxy.

**7.** Process according to claim 1, characterised in that a compound of formula

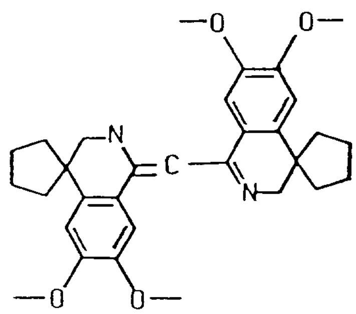

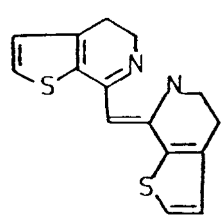

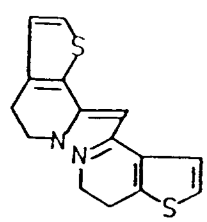

or

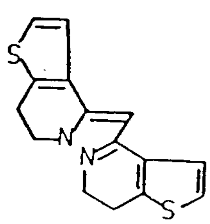

or a physiologically acceptable salt thereof is prepared.

**8.** Process according to claim 1, characterised in that a compound is prepared wherein D represents the group of formula Ib and $R_4$ represents an $-NR_9R_{10}-$ group, wherein $R_9$ and $R_{10}$ independently of each other represent (a) hydrogen, (b) alkyl with 1 to 8 carbon atoms, alkenyl or alkynyl with 2 or 3 carbon atoms (whilst the alkyl may be substituted by hydroxy, $(C_{1-4})$alkoxy, di$(C_{1-4})$alkylamino, furyl, pyrrolidinyl, morpholinyl, pyridinyl or the group

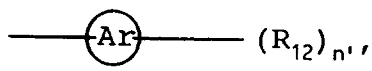

wherein Ar, $R_{12}$ and n' are defined as in claim 1), (d) dimethylamino, (f) phenyl, (g) morpholinyl, (h) pyridinyl, whilst $R_9$ and $R_{10}$ cannot simultaneously represent hydrogen, dimethylamino or di$(C_{1-4})$-alkylaminomethyl;
or $R_9$ and $R_{10}$ together with the nitrogen atom to which they are bound represent a pyrrolidinyl, morpholinyl or piperazinyl group, whilst the piperazinyl ring may optionally be N-substituted by unsubstituted phenyl, mono- or di$(C_{1-4})$alkoxypheny], pyrimidinyl or phenyl$(C_{1-4})$alkyl.

**9.** Process according to claim 8, characterised in that a compound is prepared wherein $R_4$ is an $-NR_9R_{10}$ group wherein $R_9$ is hydrogen and $R_{10}$ is a substituted alkyl of formula VII

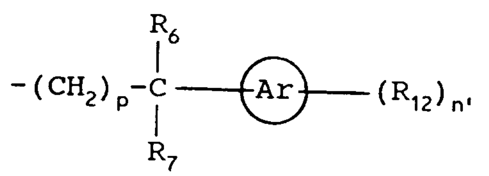

VII

wherein p is 0, 1 or 2;
$R_6$ and $R_7$ independently of each other represent hydrogen or $(C_{1-5})$alkyl or together with the carbon atom to which they are bound they represent a 5- or 6-membered carbocyclic ring;
Ar represents phenyl or thienyl;
$R_{12}$ represents $(C_{1-4})$alkyl, halogen (F, Cl, Br, I), hydroxy, $(C_{1-4})$alkoxy, amino, thiomethyl, methanesulphonyloxy or methanesulphonamido, or two adjacent substituents $R_{12}$ together represent $-O-CH_2-O-$ or $-O-CH_2-CH_2-O-$ and
n' represents 0, 1, 2 or 3, if Ar is phenyl, and 0, 1 or 2, if Ar is thienyl.

**10.** Process according to one of claims 1, 8 and 9, characterised in that a compound is prepared wherein D represents the group of formula Ib, $R_1$ represents hydrogen, $(C_{1-6})$alkyl or $-NHCOCH_3$;
$R_{11}$ represents hydroxy, $(C_{1-4})$alkoxy, methanesulphonyloxy or methanesulphonamido, or two adjacent substituents $R_{11}$ together represent $-O-CH_2-O-$ or $-O-CH_2-CH_2-O-$; and
$R_2$ and $R_3$ represent hydrogen or together with the carbon atom to which they are bound they represent a 5- or 6-membered carbocyclic group;
and $R'_5$ represents hydrogen, methyl or ethyl.

**11.** Process according to one of claims 1 and 8 to 10, characterised in that a compound is prepared wherein A is a benzo group, m represents 2 and the two substituents $R_{11}$ are in the meta- and para-positions, respectively, to the fusion points of group A, wherein $R_{11}$ is preferably methoxy.

**12.** Process according to claim 1, characterised in that a compound is prepared wherein D is the group of formula Ib, A represents benzo, $R_{11}$ represents methoxy, m represents two, $R_1$ represents hydrogen or $(C_{1-5})$alkyl, $R_2$, $R_3$ and $R'_5$ represent hydrogen and $R_4$ represents morpholino, methylamino, diethylamino or phenethylamino.

**13.** Process according to claim 1, characterised in that morpholinocarbonylmethyl-6,7-dimethoxy-3,4-dihydro-isoquinoline,
6,7-dimethoxy-3,4-dihydro-isoquinoline acetic acid methylamide,
6,7-dimethoxy-3,4-dihydro-isoquinoline acetic acid diethylamide or
6,7-dimethoxy-3,4-dihydro-isoquinoline acetic acid phenylethylamide
is prepared.

**14.** Process for preparing a pharmaceutical preparation, characterised in that a compound which is defined as in one of claims 1 to 13 is mixed with one or more pharmaceutically acceptable carriers.

**Revendications**

**Revendications pour les Etats contractants suivants : AT, BE, CH, DE, FR, GB, IT, LI, LU, NL, SE**

**1.** Composé de formule générale (I)

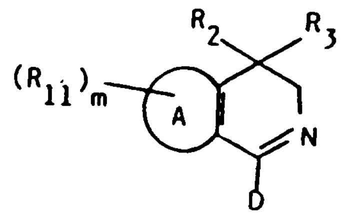

I

dans laquelle

A représente un reste benzo ou thiéno;

$R_2$ et $R_3$ représentent indépendamment l'un de l'autre un hydrogène ou un alkyle en $C_1$-$C_5$, ou forment ensemble avec l'atome de carbone auquel ils sont liés un noyau carbocyclique de 5 ou 6 chaînons;

$R_{11}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux substituants $R_{11}$ adjacents forment ensemble -O-$CH_2$-O- ou -O-$CH_2$-$CH_2$-O-;

m signifie 0, 1, 2 ou 3 lorsque A est un reste benzo, et 0, 1 ou 2 lorsque A est un reste thiéno;

et D représente un coupe de formule Ia ou Ib

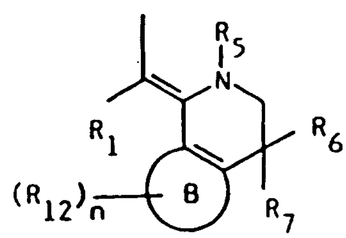

Ia

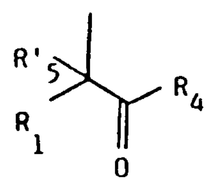

Ib

où, dans le groupe de formule Ia

B représente un reste benzo ou thiéno;

$R_1$ représente un hydrogène, un alkyle en $C_1$-$C_{10}$, un phényle, un phényl(alkyle en $C_1$-$C_5$), un alcoxy en $C_1$-$C_4$ ou -NHCOX (où X est un alkyle en $C_1$-$C_5$);

$R_5$ représente un hydrogène, un alkyle en $C_1$-$C_4$ ou un hydroxyméthyle;

$R_6$ et $R_7$ représentent indépendamment l'un de l'autre un hydrogène ou un alkyle en $C_1$-$C_5$, ou forment ensemble avec l'atome de carbone auquel ils sont liés un noyau carboocyclique de 5 ou 6 chaînons;

$R_{12}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux substituants $R_{12}$ adjacents forment ensemble -O-$CH_2$-O- ou -O-$CH_2$-$CH_2$-O-;

n signifie 0, 1, 2 ou 3 lorsque B est un reste benzo, et 0, 1 ou 2 lorsque B est un reste thiéno;

et dans le groupe de formule Ib

$R_1$ représente un hydrogène, un alkyle en $C_1$-$C_{10}$, un phényl(alkyle en $C_1$-$C_5$), un alcoxy en $C_1$-$C_4$ ou -NHCOX (où X est un alkyle en $C_1$-$C_5$);

$R_5$' représente un hydrogène ou un alkyle en $C_1$-$C_4$;

$R_4$ représente un groupe -$NR_9R_{10}$ dans lequel

$R_9$ et $R_{10}$ représentent indépendamment l'un de l'autre (a) un hydrogène, (b) un alkyle, alcényle ou alcynyle linéaire ou ramifié de 1 à 12 atomes de carbone (l'alkyle pouvant être substitué par des restes hydroxy, alcoxy en $C_1$-$C_4$, di(alkyle en $C_1$-$C_4$)amino, furyle, pyrrolidinyle, morpholinyle, pyridinyle,

indolyle ou le groupe

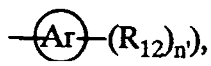

(c) un cycloalkyle de 3 à 7 chaînons cycliques, (d) un diméthylamino, (e) un amino(alkyle en $C_2$-$C_4$) (le groupe amino pouvant être non substitué ou être un mono- ou di(alkyl en $C_1$-$C_4$)amino), (f) un phényle, (g) un morpholinyle ou (h) un pyridinyle, $R_9$ et $R_{10}$ ne pouvant être en même temps un hydrogène, un diméthylamino ou un di(alkyl en $C_1$-$C_4$)aminométhyle;

ou bien $R_9$ et $R_{10}$ forment ensemble, avec l'atome d'azote auquel ils sont liés, un reste pyrrolidinyle, pipéridinyle, morpholinyle ou pipérazinyle, le cycle pipérazinyle pouvant éventuellement être N-substitué par un phényle non substitué, un mono- ou di(alcoxy en $C_1$-$C_4$)phényle, un pyrimidinyle ou un phényl(alkyle en $C_1$-$C_4$);

$R_{12}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux restes $R_{12}$ adjacents forment ensemble -O-$CH_2$-O- ou -O-$CH_2$-$CH_2$-O-;

Ar est un phényle ou un thiényle;

n' signifie 0, 1, 2 ou 3 lorsque Ar est un phényle, et 0, 1 ou 2 lorsque Ar est un thiényle;

et ses sels pharmaceutiquement acceptables avec des acides inorganiques ou organiques;

à l'exception du composé de formule I dans laquelle D est le groupe Ia et $R_1$, $R_2$, $R_3$, $R_5$, $R_6$ et $R_7$ représentent l'hydrogène et A et B représentent le reste

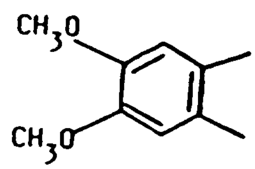

et à l'exception du composé de formule I dans laquelle D est le groupe de formule Ib et $R_1$, $R_2$, $R_3$ et $R_5$' sont l'hydrogène, A représente le reste

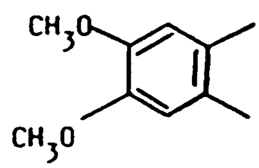

et $R_4$ représente le groupe

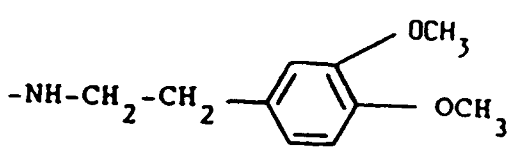

2. Composé selon la revendieation 1, dans lequel D représente le groupe de formule Ia; $R_1$ représente un hydrogène, un alkyle en $C_1$-$C_{10}$, un phényl(alkyle en $C_1$-$C_5$) ou -NHCOX (où X est un alkyle en $C_1$-$C_5$);

$R_{11}$ et $R_{12}$ représentent indépendamment l'un de l'autre un alkyle en $C_1$-$C_4$, un hydroxy, un alcoxy en $C_1$-$C_4$, un méthanesulfonyloxy ou un méthanesulfonamido, ou bien deux substituants $R_{11}$ ou $R_{12}$ adjacents forment respectivement ensemble -O-$CH_2$-O- ou -O-$CH_2$-$CH_2$-O-;

$R_2$, $R_3$, $R_6$ et $R_7$ représentent indépendamment les uns des autres l'hydrogène ou $R_2$ forme avec $R_3$ et/ou $R_6$ forme avec $R_7$, et l'atome de carbone respectif auquel ils sont liés, un noyau carbocyclique de 5 ou 6 chaînons;

$R_{11}$ et $R_{12}$ représentent indépendamment l'un de l'autre un hydroxy, un alcoxy en $C_1$-$C_4$, un

méthanesulfonyloxy ou un méthanesulfonamido, ou bien deux substituants $R_{11}$ ou $R_{12}$ adjacents forment respectivement ensemble $-O-CH_2-O-$ ou $-O-CH_2-CH_2-O-$.

**3.** Composé selon la revendication 2, dans lequel $R_1$ représente un hydrogène, un alkyle en $C_1$-$C_{10}$ ou -NHCOX (où X est un alkyle en $C_1$-$C_5$).

**4.** Composé selon la revendication 2, dans lequel $R_1$ représente un hydrogène, un alkyle en $C_1$-$C_6$ ou $-NHCOCH_3$ et/ou

$R_5$ représente un hydrogène, un méthyle ou un hydroxyméthyle et/ou

$R_2$, $R_3$, $R_6$ et $R_7$ représentent indépendamment les uns des autres un hydrogène ou $R_2$ forme avec $R_3$ et/ou $R_6$ avec $R_7$, et l'atome de carbone respectif auquel ils sont liés, un noyau carbocyclique de 5 chaînons et/ou

$R_{11}$ et $R_{12}$ représentent indépendamment l'un de l'autre un hydroxy, un méthoxy, un méthanesulfonyloxy ou un méthanesulfonamido, ou bien deux substituants $R_{11}$ ou $R_{12}$ adjacents forment respectivement ensemble $-O-CH_2-O-$.

**5.** Composé selon l'une des revendications 1 à 4, dans lequel D représente le groupe de formule Ia et m et/ou n signifient 2.

**6.** Composé selon la revendication 5, dans lequel D représente le groupe de formule Ia et, lorsque A et/ou B représentent un reste benzo, les deux substituants $R_{11}$ et/ou $R_{12}$ sont situés sur le reste benzo respectivement en position méta et para par rapport aux points de condensation du reste A ou B, et $R_{11}$ et $R_{12}$ sont de préférence un méthoxy.

**7.** Composé selon la revendication 1, qui est

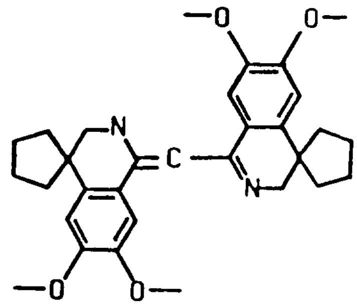

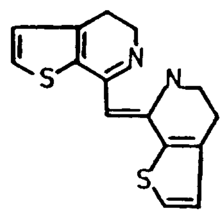

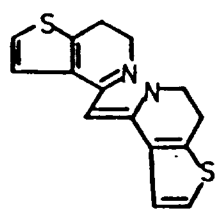

ou

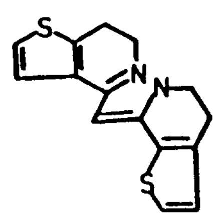

ou un de ses sels physiologiquement acceptables.

**8.** Composé selon la revendication 1, dans lequel D représente le groupe de formule Ib, et $R_4$ représente un reste $-NR_9R_{10}$, où

$R_9$ et $R_{10}$ représentent indépendamment l'un de l'autre (a) un hydrogène, (b) un alkyle de 1 à 8 atomes de carbone, un alcényle ou alcynyle de 2 ou 3 atomes de carbone (l'alkyle pouvant être substitué par des restes hydroxy, alcoxy en $C_1$-$C_4$, di(alkyl en $C_1$-$C_4$)amino, furyle, pyrrolidinyle, morpholinyle, pyridinyle ou le groupe

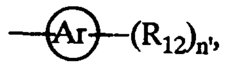

où Ar, $R_{12}$ et n'sont tels que définis dans la revendication 1), (d) un diméthylamino, (f) un pnényle, (g) un morpholinyle ou (h) un pyridinyle, $R_9$ et $R_{10}$ ne pouvant être en même temps un hydrogène, un diméthylamino ou un di(alkyl en $C_1$-$C_4$)aminométhyle;

ou bien $R_9$ et $R_{10}$ forment ensemble, avec l'atome d'azote auquel ils sont liés, un reste pyrrolidinyle, morpholinyle ou pipérazinyle, le cycle pipérazinyle pouvant éventuellement être N-substitué par un phényle non substitué, un mono-ou di(alcoxy en $C_1$-$C_4$)phényle, un pyrimidinyle ou un phényl(alkyle en $C_1$-$C_4$).

**9.** Composé selon la revendication 8, dans lequel $R_4$ est un groupe $-NR_9R_{10}$, où $R_9$ est l'hydrogène et $R_{10}$ est un alkyle substitué de formule VII

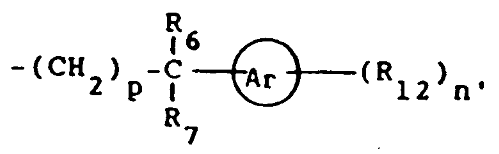

VII

dans laquelle p est 0, 1 ou 2;

$R_6$ et $R_7$ représentent indépendamment l'un de l'autre un hydrogène ou un alkyle en $C_1$-$C_5$, ou forment ensemble avec l'atome de carbone auquel ils sont liés un noyau carbocyclique de 5 ou 6 chaînons;

Ar représente un phényle ou un thiényle;

$R_{12}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux restes $R_{12}$ adjacents forment ensemble $-O-CH_2-O-$ ou $-O-CH_2-CH_2-O-$ et

n' signifie 0, 1, 2 ou 3 lorsque Ar est un phényle, et 0, 1 ou 2 lorsque Ar est un thiényle.

**10.** Composé selon l'une des revendications 1, 8 et 9, dans lequel D représente un groupe de formule Ib, $R_1$ représente un hydrogène, un alkyle en $C_1$-$C_6$ ou $-NHCOCH_3$;

$R_{11}$ représente un hydroxy, un alcoxy en $C_1$-$C_4$, un méthanesulfonyloxy ou un méthanesulfonamido, ou bien deux restes $R_{11}$ adjacents forment ensemble $-O-CH_2-O-$ ou $-O-CH_2-CH_2-O-$ et

$R_2$ et $R_3$ représentent un hydrogène ou forment ensemble avec l'atome de carbone auquel ils sont liés un noyau carbocyclique de 5 ou 6 chaînons;

et $R_5$' représente un hydrogène, un méthyle ou un éthyle.

**11.** Composé selon l'une des revendications 1 et 8 à 10, dans lequel A représente un reste benzo, m signifie 2 et les deux substituants $R_{11}$ sont situés respectivement en position méta et en position para par rapport aux points de condensation du reste A, $R_{11}$ étant de préférence un méthoxy.

**12.** Composé selon la revendication 1, dans lequel D est un groupe de formule Ib, A est un reste benzo, $R_{11}$ est un méthoxy, m est 2, $R_1$ représente un hydrogène ou un alkyle en $C_1$-$C_5$, $R_2$, $R_3$ et $R_5$' sont l'hydrogène, et $R_4$ représente un morpholino, un méthylamino, un diéthylamino ou un phénéthylamino.

**13.** Composé selon la revendication 1, qui est la morpholinocarbonylméthyl-6,7-diméthoxy-3,4-dihydroisoquinoléine, le méthylamide de l'acide 6,7-diméthoxy-3,4-dihydroisoquinoléine-acétique, le diéthylamide de l'acide 6,7-diméthoxy-3,4-dihydroisoquinoléine-acetique ou le phényléthylamide de l'acide 6,7-diméthoxy-3,4-dihydroisoquinoléine-acétique.

**14.** Procédé de préparation d'un composé selon l'une des revendications 1 à 13, caractérisé en ce que

A. (pour la préparation d'un composé de formule I dans laquelle D est le groupe de formule Ia) on condense un composé (II),

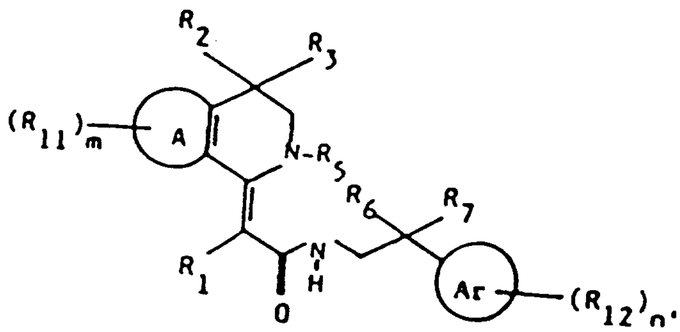

II

dans lequel A, $R_1$, $R_2$, $R_3$, $R_6$, $R_7$, $R_{11}$, $R_{12}$ et m sont tels que définis dans l'une des revendications 1 à 7, Ar représente un phényle ou un thiényle, et n' signifie 0, 1, 2 ou 3 lorsque Ar est un phényle et 0, 1 ou 2 lorsque Ar est un thiényle, et $R_5$ représente un hydrogène ou un alkyle en $C_1$-$C_4$,

en présence d'un agent de condensation;

B. (pour la préparation d'un composé de formule I dans laquelle D est le groupe de formule Ia) on condense un composé (IIIa)

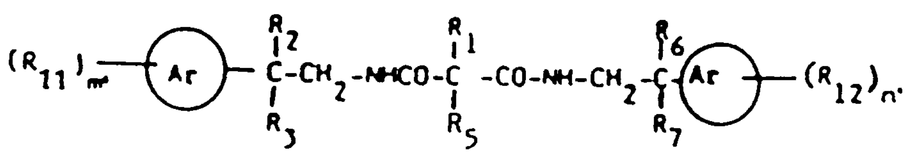

IIIa

dans lequel $R_5$ est l'hydrogène, $R_1$, $R_2$, $R_3$, $R_6$, $R_7$, $R_{11}$ et $R_{12}$ sont tels que définis dans l'une des revendications 1 à 7, Ar représente un phényle ou un thiényle et m' et n' signifient indépendamment l'un de l'autre 0, 1, 2 ou 3 lorsque le reste Ar correspondant est un phényle ou 0, 1 ou 2 lorsque le reste Ar correspondant est un thiényle;

en présence d'un agent de condensation, sans isoler le produit intermédiaire de formule générale (II);

C. (pour la préparation d'un composé de formule I dans laquelle D est le groupe de formule Ib) on condense un composé III

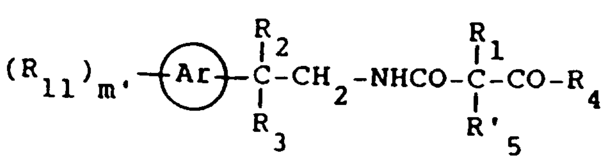

III

dans lequel $R_5$' est l'hydrogène, $R_1$, $R_2$, $R_3$ et $R_{11}$ sont tels que définis dans l'une des revendications 1 et 8 à 13, Ar représente un phényle ou un thiényle, et m' signifie 0, 1, 2 ou 3 lorsque Ar est un phényle et 0, 1 ou 2 lorsque Ar est un thiényle;

en présence d'un agent de condensation;

et en ce qu'éventuellement on effectue ensuite un ou plusieurs des traitements ultérieurs suivants:

l'alkylation d'un composé (I) dans lequel $R_5$ ou $R_5$' est l'hydrogène, pour donner un composé (I) dans lequel $R_5$ ou $R_5$' est un alkyle en $C_1$-$C_4$;

l'hydroxyméthylation d'un composé (I) dans lequel D est le groupe de formule Ia et $R_5$ est l'hydrogène, pour donner un composé dans lequel $R_5$ est un hydroxyméthyle;

l'isolement des différents tautomères compris dans la formule I;

l'isolement du composé (I) libre à partir de son sel;

la transformation d'un composé (I) en son sel pharmaceutiquement acceptable.

**15.** Composition pharmaceutique contenant comme substance active un ou plusieurs composés de formule générale I

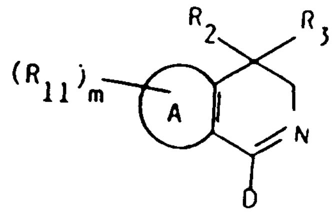

I

dans laquelle

A représente un reste benzo ou thiéno;

$R_2$ et $R_3$ représentent indépendamment l'un de l'autre un hydrogène ou un alkyle en $C_1$-$C_5$, ou forment ensemble avec l'atome de carbone auquel ils sont liés un noyau carbocyclique de 5 ou 6 chainons;

$R_{11}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux substituants $R_{11}$ adjacents forment ensemble $-O-CH_2-O-$ ou $-O-CH_2-CH_2-O-$;

m signifie 0, 1, 2 ou 3 lorsque A est un reste benzo, et 0, 1 ou 2 lorsque A est un reste thiéno;

et D représente un groupe de formule Ia ou Ib

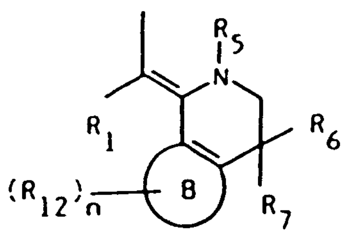

Ia

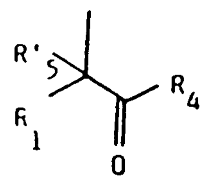

Ib

où, dans le groupe de formule Ia

B représente un reste benzo ou thiéno;

$R_1$ représente un hydrogène, un alkyle en $C_1$-$C_{10}$, un phényle, un phényl(alkyle en $C_1$-$C_5$), un alcoxy en $C_1$-$C_4$ ou -NHCOX (où X est un alkyle en $C_1$-$C_5$);

$R_5$ représente un hydrogène, un alkyle en $C_1$-$C_4$ ou un hydroxyméthyle;

$R_6$ et $R_7$ représentent indépendamment l'un de l'autre un hydrogène ou un alkyle en $C_1$-$C_5$, ou forment ensemble avec l'atome de carbone auquel ils sont liés un noyau carbocyclique de 5 ou 6 chaînons;

$R_{12}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux substituants $R_{12}$ adjacents forment ensemble $-O-CH_2-O-$ ou $-O-CH_2-CH_2-O-$;

n signifie 0, 1, 2 ou 3 lorsque B est un reste benzo, et 0, 1 ou 2 lorsque B est un reste thiéno;

et dans le groupe de formule Ib

$R_1$ représente un hydrogène, un alkyle en $C_1$-$C_{10}$, un phényl(alkyle en $C_1$-$C_5$), un alcoxy en $C_1$-$C_4$ ou -NHCOX (où X est un alkyle en $C_1$-$C_5$);

$R_5'$ représente un hydrogène ou un alkyle en $C_1$-$C_4$;

$R_4$ représente un groupe $-NR_9R_{10}$ dans lequel

$R_9$ et $R_{10}$ représentent indépendamment l'un de l'autre (a) un hydrogène, (b) un alkyle, alcényle ou alcynyle linéaire ou ramifié de 1 à 12 atomes de carbone (l'alkyle pouvant être substitué par des restes hydroxy, alcoxy en $C_1$-$C_4$, di(alkyle en $C_1$-$C_4$)amino, furyle, pyrrolidinyle, morpholinyle, pyridinyle, indolyle ou le groupe

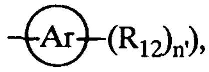

(c) un cycloalkyle de 3 à 7 chaînons cycliques, (d) un diméthylamino, (e) un amino(alkyle en $C_2$-$C_4$) (le groupe amino pouvant être non substitué ou être un mono- ou di(alkyl en $C_1$-$C_4$)amino), (f) un phényle,

(g) un morpholinyle ou (h) un pyridinyle, $R_9$ et $R_{10}$ ne pouvant être en même temps un hydrogène, un diméthylamino ou un di(alkyl en $C_1$-$C_4$)aminométhyle;

ou bien $R_9$ et $R_{10}$ forment ensemble, avec l'atome d'azote auquel ils sont liés, un reste pyrrolidinyle, pipéridinyle, morpholinyle ou pipérazinyle, le cycle pipérazinyle pouvant éventuellement être N-substitué par un phényle non substitué, un mono- ou di(alcoxy en $C_1$-$C_4$)phényle, un pyrimidinyle ou un phényl(alkyle en $C_1$-$C_4$);

$R_{12}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux restes $R_{12}$ adjacents forment ensemble -O-$CH_2$-O- ou -O-$CH_2$-$CH_2$-O-;

Ar est un phényle ou un thiényle;

n' signifie 0, 1, 2 ou 3 lorsque Ar est un phényle, et 0, 1 ou 2 lorsque Ar est un thiényle;

ou un ou plusieurs de leurs sels pharmaceutiquement acceptables.

**16.** Composition pharmaceutique selon la revendication 15, contenant un composé selon l'une des revendications 2 à 13.

**17.** Utilisation d'un composé de formule générale I, définie selon l'une des revendications 1 à 13 et 15 ou d'un ou plusieurs de ses sels pharmaceutiquement acceptables pour la préparation d'un médicament pour le traitement de l'insuffisance coronarienne, de l'infarctus aigu du myocarde, et pour la protection cardiaque et/ou cérébrale.

**Revendications pour les Etats contractants suivants : ES, GR**

**1.** Procédé de préparation d'un composé de formule générale (I)

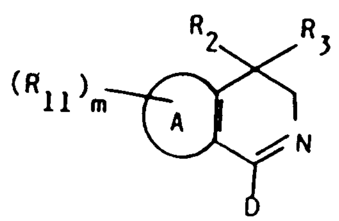

I

dans laquelle

A représente un reste benzo ou thiéno;

$R_2$ et $R_3$ représentent indépendamment l'un de l'autre un hydrogène ou un alkyle en $C_1$-$C_5$, ou forment ensemble avec l'atome de carbone auquel ils sont liés un noyau carbocyclique de 5 ou 6 chaînons;

$R_{11}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux substituants $R_{11}$ adjacents forment ensemble -O-$CH_2$-O- ou -O-$CH_2$-$CH_2$-O-;

m signifie 0, 1, 2 ou 3 lorsque A est un reste benzo, et 0, 1 ou 2 lorsque A est un reste thiéno;

et D représente un groupe de formule Ia ou Ib

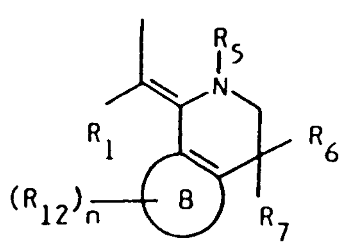

Ia

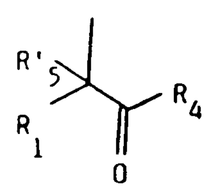

Ib

où, dans le groupe de formule Ia

B représente un reste benzo ou thiéno;

$R_1$ représente un hydrogène, un alkyle en $C_1$-$C_{10}$, un phényle, un phényl(alkyle en $C_1$-$C_5$), un

alcoxy en $C_1$-$C_4$ ou -NHCOX (où X est un alkyle en $C_1$-$C_5$);

$R_5$ représente un hydrogène, un alkyle en $C_1$-$C_4$ ou un hydroxyméthyle;

$R_6$ et $R_7$ représentent indépendamment l'un de l'autre un hydrogène ou un alkyle en $C_1$-$C_5$, ou forment ensemble avec l'atome de carbone auquel ils sont liés un noyau carbocyclique de 5 ou 6 chaînons;

$R_{12}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux substituants $R_{12}$ adjacents forment ensemble -O-$CH_2$-O ou -O-$CH_2$-$CH_2$-O-;

n signifie 0, 1, 2 ou 3 lorsque B est un reste benzo, et 0, 1 ou 2 lorsque B est un reste thiéno;

et dans le groupe de formule Ib

$R_1$ représente un hydrogène, un alkyle en $C_1$-$C_{10}$, un phényl(alkyle en $C_1$-$C_5$), un alcoxy en $C_1$-$C_4$ ou -NHCOX (où X est un alkyle en $C_1$-$C_5$);

$R_5'$ représente un hydrogène ou un alkyle en $C_1$-$C_4$;

$R_4$ représente un groupe -$NR_9R_{10}$ dans lequel

$R_9$ et $R_{10}$ représentent indépendamment l'un de l'autre (a) un hydrogène, (b) un alkyle, alcényle ou alcynyle linéaire ou ramifié de 1 à 12 atomes de carbone (l'alkyle pouvant être substitué par des restes hydroxy, alcoxy en $C_1$-$C_4$, di(alkyle en $C_1$-$C_4$)amino, furyle, pyrrolidinyle, morpholinyle, pyridinyle, indolyle ou le groupe

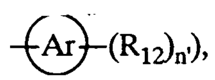

(c) un cycloalkyle de 3 à 7 chaînons cycliques, (d) un diméthylamino, (e) un amino(alkyle en $C_2$-$C_4$) (le groupe amino pouvant être non substitué ou être un mono- ou di(alkyl en $C_1$-$C_4$)amino), (f) un phényle, (g) un morpholinyle ou (h) un pyridinyle, $R_9$ et $R_{10}$ ne pouvant être en même temps un hydrogène, un diméthylamino ou un di(alkyl en $C_1$-$C_4$)aminométhyle;

ou bien $R_9$ et $R_{10}$ forment ensemble, avec l'atome d'azote auquel ils sont liés, un reste pyrrolidinyle, pipéridinyle, morpholinyle ou pipérazinyle, le cycle pipérazinyle pouvant éventuellement être N-substitué par un phényle non substitué, un mono- ou di(alcoxy en $C_1$-$C_4$)phényle, un pyrimidinyle ou un phényl(alkyle en $C_1$-$C_4$);

$R_{12}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux restes $R_{12}$ adjacents forment ensemble -O-$CH_2$-O- ou -O-$CH_2$-$CH_2$-O-;

Ar est un phényle ou un thiényle;

n' signifie 0, 1, 2 ou 3 lorsque Ar est un phényle, et 0, 1 ou 2 lorsque Ar est un thiényle;

et ses sels pharmaceutiquement acceptables avec des acides inorganiques ou organiques;

à l'exception du composé de formule I dans laquelle D est le groupe Ia et $R_1$, $R_2$, $R_3$, $R_5$, $R_6$ et $R_7$ représentent l'hydrogène et A et B représentent le reste

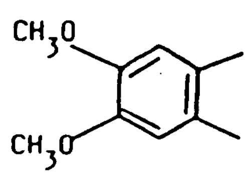

et à l'exception du composé de formule I dans laquelle D est le groupe de formule Ib et $R_1$, $R_2$, $R_3$ et $R_5'$ sont l'hydrogène, A représente le reste

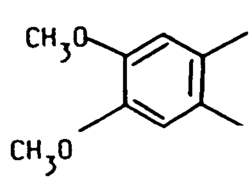

et $R_4$ représente le groupe

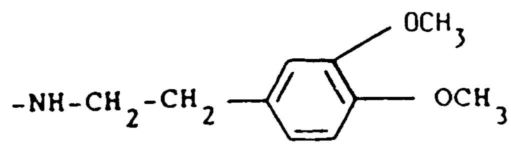

caractérisé en ce que

A. (pour la préparation d'un composé de formule I dans laquelle D est le groupe de formule Ia) on condense un composé (II),

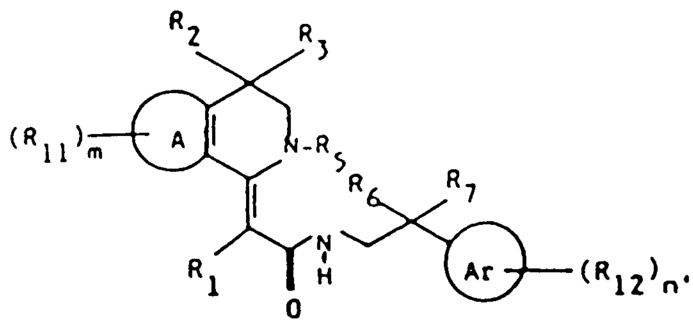

II

dans lequel A, $R_1$, $R_2$, $R_3$, $R_6$, $R_7$, $R_{11}$, $R_{12}$ et m sont tels que définis ci-dessus, Ar représente un phényle ou un thiényle, et n' signifie 0, 1, 2 ou 3 lorsque Ar est un phényle et 0, 1 ou 2 lorsque Ar est un thiényle, et $R_5$ représente l'hydrogène ou un alkyle en $C_1$-$C_4$,

en présence d'un agent de condensation;

B. (pour la préparation d'un composé de formule I dans laquelle D est le groupe de formule Ia) on condense un composé (IIIa)

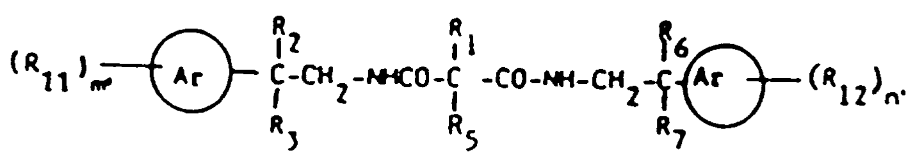

IIIa

dans lequel $R_5$ est l'hydrogène, $R_1$, $R_2$, $R_3$, $R_6$, $R_7$, $R_{11}$ et $R_{12}$ sont tels que définis ci-dessus, Ar représente un phényle ou un thiényle et m' et n' signifient indépendamment l'un de l'autre 0, 1, 2 ou 3 lorsque le reste Ar correspondant est un phényle ou 0, 1 ou 2 lorsque le reste Ar correspondant est un thiényle;

en présence d'un agent de condensation, sans isoler le produit intermédiaire de formule générale (II);

C. (pour la préparation d'un composé de formule I dans laquelle D est le groupe de formule Ib) on condense un composé III

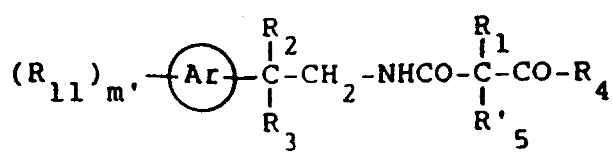

III

dans lequel $R_5$' est l'hydrogène, $R_1$, $R_2$, $R_3$ et $R_{11}$ sont tels que définis ci-dessus, Ar représente un phényle ou un thiényle, et m' signifie 0, 1, 2 ou 3 lorsque Ar est un phényle et 0, 1 ou 2 lorsque Ar est un thiényle;

en présence d'un agent de condensation;

et en ce qu'éventuellement on effectue ensuite un ou plusieurs des traitements ultérieurs

suivants:

l'alkylation d'un composé (I) dans lequel $R_5$ ou $R_5'$ est l'hydrogène, pour donner un composé (I) dans lequel $R_5$ ou $R_5'$ est un alkyle en $C_1$-$C_4$;

l'hydroxyméthylation d'un composé (I) dans lequel D est le groupe de formule Ia et $R_5$ est l'hydrogène, pour donner un composé dans lequel $R_5$ est un hydroxyméthyle;

l'isolement des différents tautomères compris dans la formule I;

l'isolement du composé (I) libre à partir de son sel;

la transformation d'un composé (I) en son sel pharmaceutiquement acceptable.

**2.** Procédé selon la revendication 1, caractérisé en ce que l'on prépare un composé dans lequel D représente le groupe de formule Ia; $R_1$ représente un hydrogène, un alkyle en $C_1$-$C_{10}$, un phényl(alkyle en $C_1$-$C_5$) ou -NHCOX (où X est un alkyle en $C_1$-$C_5$);

$R_{11}$ et $R_{12}$ représentent indépendamment l'un de l'autre un alkyle en $C_1$-$C_4$, un hydroxy, un alcoxy en $C_1$-$C_4$, un méthanesulfonyloxy ou un méthanesulfonamido, ou bien deux substituants $R_{11}$ ou $R_{12}$ adjacents forment respectivement ensemble $-O-CH_2-O-$ ou $-O-CH_2-CH_2-O-$;

$R_2$, $R_3$, $R_6$ et $R_7$ représentènt indépendamment les uns des autres l'hydrogène ou $R_2$ forme avec $R_3$ et/ou $R_6$ forme avec $R_7$, et l'atome de carbone respectif auquel ils sont liés, un noyau carbocyclique de 5 ou 6 chaînons;

$R_{11}$ et $R_{12}$ représentent indépendamment l'un de l'autre un hydroxy, un alcoxy en $C_1$-$C_4$, un méthanesulfonyloxy ou un méthanesulfonamido, ou bien deux substituants $R_{11}$ ou $R_{12}$ adjacents forment respectivement ensemble $- O-CH_2-O-$ ou $-O-CH_2-CH_2-O-$.

**3.** Procédé selon la revendication 2, caractérisé en ce que l'on prépare un composé dans lequel $R_1$ représente un hydrogène, un alkyle en $C_1$-$C_{10}$ ou -NHCOX (où X est un alkyle en $C_1$-$C_5$).

**4.** Procédé selon la revendication 2, caractérisé en ce que l'on prépare un composé dans lequel $R_1$ représente un hydrogène, un alkyle en $C_1$-$C_6$ ou $-NHCOCH_3$ et/ou

$R_5$ représente un hydrogène, un méthyle ou un hydroxyméthyle et/ou

$R_2$, $R_3$, $R_6$ et $R_7$ représentent indépendamment les uns des autres un hydrogène ou $R_2$ forme avec $R_3$ et/ou $R_6$ avec $R_7$, et l'atome de carbone respectif auquel ils sont liés, un noyau carbocyclique de 5 chaînons et/ou

$R_{11}$ et $R_{12}$ représentent indépendamment l'un de l'autre un hydroxy, un méthoxy, un méthanesulfonyloxy ou un méthanesulfonamido, ou bien deux substituants $R_{11}$ ou $R_{12}$ adjacents forment respectivement ensemble $- O-CH_2-O-$.

**5.** Procédé selon l'une des revendications 1 à 4, caractérisé en ce que l'on prépare un composé dans lequel D représente le groupe de formule Ia et m et/ou n signifient 2.

**6.** Procédé selon la revendication 5, dans lequel D représente le groupe de formule Ia et, lorsque A et/ou B représentent un reste benzo, les deux substituants $R_{11}$ et/ou $R_{12}$ sont situés sur le reste benzo respectivement en position méta et para par rapport aux points de condensation du reste A ou B, et $R_{11}$ et $R_{12}$ sont de préférence un méthoxy.

7. Procédé selon la revendication 1, caractérisé en ce que l'on prépare un composé de formule

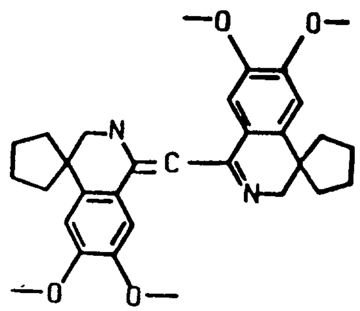

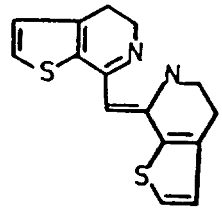

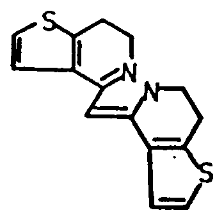

ou

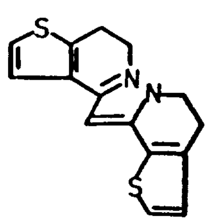

ou un de leurs sels physiologiquement acceptables.

8. Procédé selon la revendication 1, caractérisé en ce que l'on prépare un composé dans lequel D représente le groupe de formule Ib, et $R_4$ représente un reste $_3NR_9R_{10}$, où

$R_9$ et $R_{10}$ représentent indépendamment l'un de l'autre (a) un hydrogène, (b) un alkyle de 1 à 8 atomes de carbone, un alcényle ou alcynyle de 2 ou 3 atomes de carbone (l'alkyle pouvant être substitué par des restes hydroxy, alcoxy en $C_1$-$C_4$, di(alkyl en $C_1$-$C_4$)amino, furyle, pyrrolidinyle,

morpholinyle, pyridinyle, indolyle ou le groupe

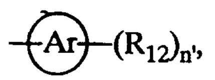

où Ar, $R_{12}$ et n' sont tels que définis dans la revendication 1), (d) un diméthylamino, (f) un phényle, (g) un morpholinyle ou (h) un pyridinyle, $R_9$ et $R_{10}$ ne pouvant être en même temps un hydrogène, un diméthylamino ou un di(alkyl en $C_1$-$C_4$)aminométhyle;

ou bien $R_9$ et $R_{10}$ forment ensemble, avec l'atome d'azote auquel ils sont liés, un reste pyrrolidinyle, morpholinyle ou pipérazinyle, le cycle pipérazinyle pouvant éventuellement être N-substitué par un phényle non substitué, un mono-ou di(alcoxy en $C_1$-$C_4$)phényle, un pyrimidinyle ou un phényl(alkyle en $C_1$-$C_4$).

**9.** Procédé selon la revendication 8, caractérisé en ce que l'on prépare un composé dans lequel $R_4$ est un groupe $-NR_9R_{10}$, où $R_9$ est l'hydrogène et $R_{10}$ est un alkyle substitué de formule VII

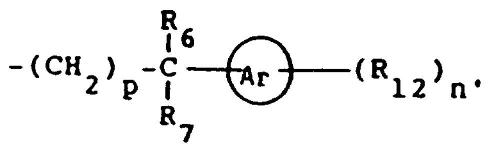

VII

dans laquelle p est 0, 1 ou 2;

$R_6$ et $R_7$ représentent indépendamment l'un de l'autre un hydrogène ou un alkyle en $C_1$-$C_5$, ou forment ensemble avec l'atome de carbone auquel ils sont liés un noyau carbocyclique de 5 ou 6 chaînons;

Ar représente un phényle ou un thiényle;

$R_{12}$ représente un alkyle en $C_1$-$C_4$, un halogène (F, Cl, Br, I), un hydroxy, un alcoxy en $C_1$-$C_4$, un amino, un thiométhyle, un méthanesulfonyloxy ou un méthanesulfonamido, ou bien deux restes $R_{12}$ adjacents forment ensemble $-O-CH_2-O-$ ou $-O-CH_2-CH_2-O-$ et

n' signifie 0, 1, 2 ou 3 lorsque Ar est un phényle, et 0, 1 ou 2 lorsque Ar est un thiényle.

**10.** Procédé selon l'une des revendications 1, 8 et 9, caractérisé en ce que l'on prépare un composé dans lequel D représente un groupe de formule Ib, $R_1$ représente un hydrogène, un alkyle en $C_1$-$C_6$ ou $-NHCOCH_3$;

$R_{11}$ représente un hydroxy, un alcoxy en $C_1$-$C_4$, un méthanesulfonyloxy ou un méthanesulfonamido, ou bien deux restes $R_{11}$ adjacents forment ensemble $-O-CH_2-O-$ ou $-O-CH_2-CH_2-O-$ et

$R_2$ et $R_3$ représentent un hydrogène ou forment ensemble avec l'atome de carbone auquel ils sont liés un noyau carbocyclique de 5 ou 6 chaînons;

et $R_5$' représente un hydrogène, un méthyle ou un éthyle.

**11.** Procédé selon l'une des revendications 1 et 8 à 10, caractérisé en ce que l'on prépare un composé dans lequel A représente un reste benzo, m signifie 2 et les deux substituants $R_{11}$ sont situés respectivement en position méta et en position para par rapport aux points de condensation du reste A, $R_{11}$ étant de préférence un méthoxy.

**12.** Procédé selon la revendieation 1, caractérisé en ce que l'on prépare un composé dans lequel D est le groupe de formule Ib, A est un reste benzo, $R_{11}$ est un méthoxy, m est 2, $R_1$ représente un hydrogène ou un alkyle en $C_1$-$C_5$, $R_2$ $R_3$ et $R_5$' sont l'hydrogène, et $R_4$ représente un morpholino, un méthylamino, un diéthylamino ou un phénéthylamino.

**13.** Procédé selon la revendication 1, caractérisé en ce que l'on prépare la morpholinocarbonylméthyl-6,7-diméthoxy-3,4-dihydroisoquinoléine, le méthylamide de l'acide 6,7-diméthoxy-3,4-dihydroisoquinoléine-acétique, le diéthylamide de l'aeide 6,7-diméthoxy-3,4-dihydroisoquinoléine-acétique ou le phényléthylamide de l'acide 6,7-diméthoxy-3,4-dihydroisoquinoléine-acétique.

14. Procédé de préparation d'une composition pharmaceutique, caractérisé en ce que l'on mélange un composé tel que défini dans l'une des revendications 1 à 13 avec un ou plusieurs supports pharmaceutiquement acceptables.